(19) Europäisches Patentamt
European Patent Office
Office européen des brevets

(11) Veröffentlichungsnummer: **0 014 885 B1**

# (12) EUROPÄISCHE PATENTSCHRIFT

(45) Veröffentlichungstag der Patentschrift: **15.06.83**

(51) Int. Cl.³: **C 07 D 239/26,** C 09 K 3/34

(21) Anmeldenummer: **80100549.7**

(22) Anmeldetag: **04.02.80**

(54) **Cyclohexylpyrimidine, deren Herstellung und Verwendung, sowie flüssigkristalline Gemische und elektro-optische Vorrichtungen, die solche Verbindungen enthalten, und deren Herstellung.**

(30) Priorität: **05.02.79 CH 1102/79**
**27.06.79 CH 5996/79**
**13.11.79 CH 10126/79**

(43) Veröffentlichungstag der Anmeldung:
**03.09.80 Patentblatt 80/18**

(45) Bekanntmachung des Hinweises auf die Patenterteilung:
**15.06.83 Patentblatt 83/24**

(84) Benannte Vertragsstaaten:
**AT BE CH DE FR IT NL**

(56) Entgegenhaltungen:
**DE-A-2 429 093**
**DE-A-2 636 684**
**DE-A-2 641 724**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Boller, Arthur, Dr., Benkenstrasse 65, CH-4102 Binningen (CH)**
Erfinder: **Schadt, Martin, Dr., Liestalerstrasse 77, CH-4411 Seltisberg (CH)**
Erfinder: **Villiger, Alois, Dr., Sonnenweg 18, CH-4052 Basel (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

**EP 0 014 885 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Cyclohexylpyrimidine, deren Herstellung und Verwendung, sowie flüssigkristalline Gemische und elektro-optische Vorrichtungen, die solche Verbindungen enthalten, und deren Herstellung

Die vorliegende Erfindung betrifft neue Pyrimidinderivate der allgemeinen Formel

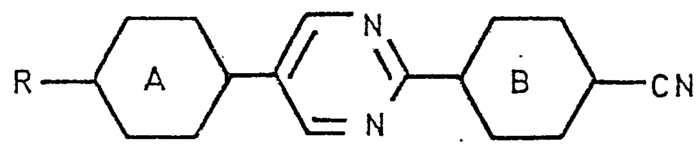

I

worin wenigstens einer der Ringe A und B einen trans-1,4-disubstituierten Cyclohexanring darstellt und der andere gegebenenfalls aromatisch ist und R eine geradkettige Alkyl- oder Alkoxygruppe mit 1 bis 10 Kohlenstoffatomen oder eine verzweigte Alkylgruppe

$C_2H_5-CH(CH_3)-(CH_2)_n-$,

worin n eine ganze Zahl von 1 bis 3 ist, bedeutet.

Die Erfindung betrifft ebenfalls die Herstellung der Verbindungen der obigen Formel I, nematische und cholesterische Mischungen, welche diese Verbindungen enthalten, sowie deren Verwendung in elektrooptischen Vorrichtungen.

Die erfindungsgemäßen Verbindungen sind besonders wertvoll als Komponenten von flüssigkristallinen Mischungen und besitzen zum größten Teil selbst flüssigkristalline Eigenschaften, wobei die Verbindungen mit gerader Seitenkette eine nematische, die optisch aktiven Verbindungen mit verzweigter Seitenkette hingegen eine cholesterische Mesophase bilden. Die erfindungsgemäßen Verbindungen besitzen unter anderem eine sehr hohe positive Anisotropie der Dielektrizitätskonstanten ($\varepsilon_{\parallel} > \varepsilon_{\perp}$, wobei $\varepsilon_{\parallel}$ die Dielektrizitätskonstante entlang der Moleküllängsachse und $\varepsilon_{\perp}$ die Dielektrizitätskonstante senkrecht dazu bedeuten).

In einem elektrischen Feld orientieren sich die erfindungsgemäßen Verbindungen (wegen $\varepsilon_{\parallel} > \varepsilon_{\perp}$) mit der Richtung ihrer größten Dielektrizitätskonstante, d. h. mit ihren Längsachsen parallel zur Feldrichtung. Dieser Effekt wird u. a. in der von J. H. Heilmeier und L. A. Zanoni (Applied Physics Letters 13, 91 [1968]) beschriebenen Wechselwirkung zwischen eingelagerten Molekülen und den flüssigkristallinen Molekülen (Guest-Host interaction) ausgenützt. Eine weitere interessante Anwendung der dielektrischen Feldorientierung liegt in der von M. Schadt und W. Helfrich (Applied Physics Letters 18, [1971)] gefundenen Drehzelle sowie bei der in Molecular Crystals and Liquid Crystals 17, 355 (1972) beschriebenen Kerrzelle vor.

Bei dieser elektrooptischen Drehzelle handelt es sich im wesentlichen um einen Kondensator mit lichtdurchlässigen Elektroden, dessen Dielektrikum von einem nematischen Kristall mit $\varepsilon_{\parallel} > \varepsilon_{\perp}$ gebildet wird. Die Moleküllängsachsen des flüssigen Kristalls sind im feldfreien Zustand schraubenförmig zwischen den Kondensatorplatten angeordnet, wobei die Schraubenstruktur durch die vorgegebene Wandorientierung der Moleküle bestimmt ist. Nach dem Anlegen einer elektrischen Spannung an die Kondensatorplatten stellen sich die Moleküle mit ihren Längsachsen in Feldrichtung (d. h. senkrecht zur Plattenoberfläche) ein, wodurch linear polarisiertes Licht im Dielektrikum nicht mehr gedreht wird (der flüssige Kristall wird senkrecht zur Oberfläche der Platten einachsig). Dieser Effekt ist reversibel und kann dazu verwendet werden, die optische Transparenz des Kondensators elektrisch zu steuern.

In einer derartigen »Licht-Drehzelle« ist es — unter anderem — wünschenswert, Verbindungen oder Mischungen zu benützen, welche eine niedrige Schwellenspannung besitzen, was z. B. bei Verwendung einer Drehzelle in Taschenrechnern, usw. wichtig ist.

Ferner ist bekannt, daß die Zugabe von cholesterischen Substanzen zu einer Matrix nematischer Flüssigkristalle mit positiver Anisotropie der Dielektrizitätskonstanten zu einem cholesterischen Gemisch führt, welches durch Anlegen eines elektrischen Feldes einen cholesterisch-nematischen Phasenübergang erfährt. Dieser Phasenübergang ist reversibel und ermöglicht hohe Schaltgeschwindigkeiten der mit derartigen Mischungen betriebenen elektro-optischen Vorrichtungen.

Aus DE-A-2 429 093 und DE-A-2 636 684 sind bereits Verbindungen, welche einen Cyclohexanring enthalten, bekannt, nämlich Cyclohexancarbonsäure-phenylester und Phenylcyclohexane. Erstere weisen jedoch zum Teil smektische Mesophasen auf, während letztere häufig nicht oder nur monotrop flüssigkristallin sind.

Aus DE-A-2 641 724 sind ferner bereits cyano-substituierte Diphenylpyrimidine bekannt.

Es wurde nun gefunden, daß die erfindungsgemäßen Verbindungen der Formel I einen besonders großen Mesophasenbereich mit hohen Klärpunkten aufweisen, weshalb sie sich ganz besonders dazu eignen, die Klärpunkte von nematischen Mischungen zu erhöhen. Außerdem besitzen sie nicht nur die erforderliche große positive Anisotropie der Dielektrizitätskonstanten und dementsprechend niedere Schwellenspannungen in Anzeigevorrichtungen basierend auf einem Feldeffekt, wie z. B. der oben

näher beschriebenen Drehzelle, sondern zeigen eine kurze Ansprechzeit und eine große chemische Stabilität. Gegenüber den aus DE-A-2 641 724 bekannten Diphenylpyrimidinen besitzen die Verbindungen der Formel I, worin Ring A Cyclohexan und Ring B Benzol bedeutet, eine verbesserte Viskosität und die Verbindungen der Formel I, worin Ring A Benzol und Ring B Cyclohexan bedeutet, eine höhere dielektrische Anisotropie. Ferner verringern die erfindungsgemäßen Verbindungen der Formel I in Anzeigevorrichtungen basierend auf einem Feldeffekt die Temperaturabhängigkeit der optischen Transmissionskurven beträchtlich, wenn sie anderen flüssigkristallinen Substanzen wie beispielsweise para-Cyano-substituierten Schiffschen Basen, Estern oder Biphenylen und dergleichen beigemischt werden. Diese Eigenschaft macht die Verbindungen der Formel I besonders wertvoll für den Multiplexbetrieb in Anzeigevorrichtungen mit niedriger Schwellenspannung. Ein weiterer Vorteil der erfindungsgemäßen Verbindungen liegt darin, daß sie farblos sind. Mischungen, welche die erfindungsgemäßen Verbindungen der Formel I enthalten, zeichnen sich durch leichte Orientierbarkeit und geringe smektische Tendenzen aus und ergeben in Anzeigevorrichtungen einen hohen Kontrast.

Die unter die allgemeine Formel I fallenden, erfindungsgemäßen Pyrimidinderivate sind entweder trans-p-[5-(4-Alkyl- oder 4-n-Alkoxycyclohexyl)-2-pyrimidinyl]benzonitrile der Formel

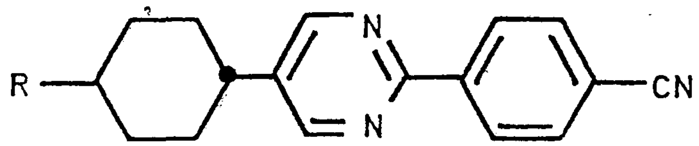

I A

trans-4-[5-(p-Alkyl- oder p-n-Alkoxyphenyl)-2-pyrimidinyl]-cyclohexancarbonitrile der Formel

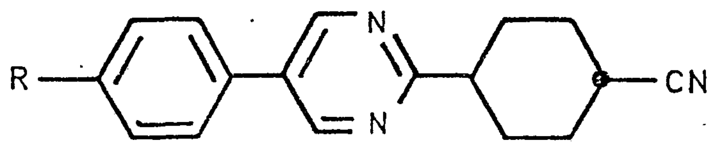

I B

oder trans-4-[5-(trans-4-Alkyl- oder trans-4-n-Alkoxycyclohexyl)-2-pyrimidinyl]-cyclohexancarbonitrile der Formel

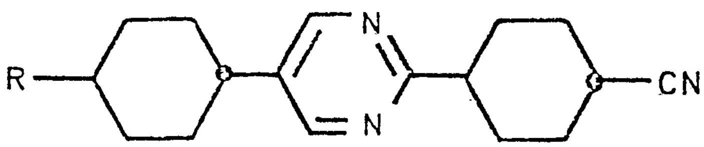

I C

in denen der Rest R die obige Bedeutung hat.

Von den Verbindungen der obigen Formeln IA, IB und IC sind diejenigen der Formel IA bevorzugt und insbesondere diejenigen der Formel IA, worin R eine Alkylgruppe darstellt. Falls R eine Alkylgruppe darstellt, sind bei allen Verbindungen der Formeln IA, IB und IC diejenigen bevorzugt, worin die Alkylgruppe 2 bis 8 Kohlenstoffatome und insbesondere 2—7 Kohlenstoffatome aufweist. Falls R eine Alkoxygruppe darstellt, sind diejenigen mit bis zu 6 Kohlenstoffatomen bevorzugt.

Als Beispiele von Verbindungen, welche unter die allgemeine Formel I fallen, können folgende genannt werden:

trans-p-[5-(4-Methylcyclohexyl)-2-pyrimidinyl]benzonitril;
trans-p-[5-(4-Äthylcyclohexyl)-2-pyrimidinyl]benzonitril;
trans-p-[5-(4-Propylcyclohexyl)-2-pyrimidinyl]benzonitril;
trans-p-[5-(4-Butylcyclohexyl)-2-pyrimidinyl]benzonitril;
trans-p-[5-(4-Pentylcyclohexyl)-2-pyrimidinyl]benzonitril;
trans-p-[5-(4-Hexylcyclohexyl)-2-pyrimidinyl]benzonitril;
trans-p-[5-(4-Heptylcyclohexyl)-2-pyrimidinyl]benzonitril;
trans-p-[5-(4-Octylcyclohexyl)-2-pyrimidinyl]benzonitril;
trans-p-[5-(4-Nonylcyclohexyl)-2-pyrimidinyl]benzonitril;
trans-p-[5-(4-Decylcyclohexyl)-2-pyrimidinyl]benzonitril;
trans-p-[5-(4-Methoxycyclohexyl)-2-pyrimidinyl]benzonitril;
trans-p-[5-(4-Äthoxycyclohexyl)-2-pyrimidinyl]benzonitril;
trans-p-[5-(4-Propyloxycyclohexyl)-2-pyrimidinyl]benzonitril;
trans-p-[5-(4-Butyloxycyclohexyl)-2-pyrimidinyl]benzonitril;
trans-p-[5-(4-Pentyloxycyclohexyl)-2-pyrimidinyl]benzonitril;
trans-p-[5-(4-Hexyloxycyclohexyl)-2-pyrimidinyl]benzonitril;
trans-p-[5-(4-Heptyloxycyclohexyl)-2-pyrimidinyl]benzonitril;

trans-p-[5-(4-Octyloxycyclohexyl)-2-pyrimidinyl]benzonitril;
trans-p-[5-(4-Nonyloxycyclohexyl)-2-pyrimidinyl]benzonitril;
trans-p-[5-(4-Decyloxycyclohexyl)-2-pyrimidinyl]benzonitril;
trans-4-[5-(p-Methylphenyl)-2-pyrimidinyl]cyclohexancarbonitril;
trans-4-[5-(p-Äthylphenyl)-2-pyrimidinyl]cyclohexancarbonitril;
trans-4-[5-(p-Propylphenyl)-2-pyrimidinyl]cyclohexancarbonitril;
trans-4-[5-(p-Butylphenyl)-2-pyrimidinyl]cyclohexancarbonitril;
trans-4-[5-(p-Pentylphenyl)-2-pyrimidinyl]cyclohexancarbonitril;
trans-4-[5-(p-Hexylphenyl)-2-pyrimidinyl]cyclohexancarbonitril;
trans-4-[5-(p-Heptylphenyl)-2-pyrimidinyl]cyclohexancarbonitril;
trans-4-[5-(p-Octylphenyl)-2-pyrimidinyl]cyclohexancarbonitril;
trans-4-[5-(p-Nonylphenyl)-2-pyrimidinyl]cyclohexancarbonitril;
trans-4-[5-(p-Decylphenyl)-2-pyrimidinyl]cyclohexancarbonitril;
trans-4-[5-(p-Methoxyphenyl)-2-pyrimidinyl]cyclohexancarbonitril;
trans-4-[5-(p-Äthoxyphenyl)-2-pyrimidinyl]cyclohexancarbonitril;
trans-4-[5-(p-Propyloxyphenyl)-2-pyrimidinyl]cyclohexancarbonitril;
trans-4-[5-(p-Butyloxyphenyl)-2-pyrimidinyl]cyclohexancarbonitril;
trans-4-[5-(p-Pentyloxyphenyl)-2-pyrimidinyl]cyclohexancarbonitril;
trans-4-[5-(p-Hexyloxyphenyl)-2-pyrimidinyl]cyclohexancarbonitril;
trans-4-[5-(p-Heptyloxyphenyl)-2-pyrimidinyl]cyclohexancarbonitril;
trans-4-[5-(p-Octyloxyphenyl)-2-pyrimidinyl]cyclohexancarbonitril;
trans-4-[5-(p-Nonyloxyphenyl)-2-pyrimidinyl]cyclohexancarbonitril;
trans-4-[5-(p-Decyloxyphenyl)-2-pyrimidinyl]cyclohexancarbonitril;
trans-4-[5-(trans-4-Methylcyclohexyl)-2-pyrimidinyl]cyclohexancarbonitril;
trans-4-[5-(trans-4-Äthylcyclohexyl)-2-pyrimidinyl]cyclohexancarbonitril;
trans-4-[5-(trans-4-Propylcyclohexyl)-2-pyrimidinyl]cyclohexancarbonitril;
trans-4-[5-(trans-4-Butylcyclohexyl)-2-pyrimidinyl]cyclohexancarbonitril:
trans-4-[5-(trans-4-Pentylcyclohexyl)-2-pyrimidinyl]cyclohexancarbonitril;
trans-4-[5-(trans-4-Hexylcyclohexyl)-2-pyrimidinyl]cyclohexancarbonitril;
trans-4-[5-(trans-4-Heptylcyclohexyl)-2-pyrimidinyl]cyclohexancarbonitril;
trans-4-[5-(trans-4-Octylcyclohexyl)-2-pyrimidinyl]cyclohexancarbonitril;
trans-4-[5-(trans-4-Nonylcyclohexyl)-2-pyrimidinyl]cyclohexancarbonitril;
trans-4-[5-(trans-4-Decylcyclohexyl)-2-pyrimidinyl]cyclohexancarbonitril;
trans-4-[5-(trans-4-Methoxycyclohexyl)-2-pyrimidinyl]cyclohexancarbonitril;
trans-4-[5-(trans-4-Äthoxycyclohexyl)-2-pyrimidinyl]cyclohexancarbonitril;
trans-4-[5-(trans-4-Propyloxycyclohexyl)-2-pyrimidinyl]cyclohexancarbonitril;
trans-4-[5-(trans-4-Butyloxycyclohexyl)-2-pyrimidinyl]cyclohexancarbonitril;
trans-4-[5-(trans-4-Pentyloxycyclohexyl)-2-pyrimidinyl]cyclocyclohexancarbonitril;
trans-4-[5-(trans-4-Hexyloxycyclohexyl)-2-pyrimidinyl]cyclohexancarbonitril;
trans-4-[5-(trans-4-Heptyloxycyclohexyl)-2-pyrimidinyl]cyclohexancarbonitril;
trans-4-[5-(trans-4-Octyloxycyclohexyl)-2-pyrimidinyl]cyclohexancarbonitril;
trans-4-[5-(trans-4-Nonyloxycyclohexyl)-2-pyrimidinyl]cyclohexancarbonitril;
trans-4-[5-(trans-4-Decyloxycyclohexyl)-2-pyrimidinyl]cyclohexancarbonitril;
(+)-trans-p-[5-[4-(2-Methylbutyl)cyclohexyl]-2-pyrimidinyl]benzonitril;
(+)-trans-p-[5-[4-(3-Methylpentyl)cyclohexyl]-2-pyrimidinyl]benzonitril;
(+)-trans-p-[5-[4-(4-Methylhexyl)cyclohexyl]-2-pyrimidinyl]benzonitril;
(+)-trans-4-[5-[p-(2-Methylbutyl)phenyl]-2-pyrimidinyl]cyclohexancarbonitril;
(+)-trans-4-[5-[p-(3-Methylpentyl)phenyl]-2-pyrimidinyl]cyclohexancarbonitril;
(+)-trans-4-[5-[p-(4-Methylhexyl)phenyl]-2-pyrimidinyl]cyclohexancarbonitril;
(+)-trans-4-[5-[trans-4-(2-Methylbutyl)cyclohexyl]-2-pyrimidinyl]cyclohexancarbonitril;
(+)-trans-4-[5-[trans-4-(3-Methylpentyl)cyclohexyl]-2-pyrimidinyl]cyclohexancarbonitril;
(+)-trans-4-[5-[trans-4-(4-Methylhexyl)cyclohexyl]-2-pyrimidinyl]cyclohexancarbonitril;

sowie die Antipoden der optisch aktiven Verbindungen.

Besonders bevorzugte Verbindungen sind:

trans-p-[5-(4-Äthylcyclohexyl)-2-pyrimidinyl]benzonitril;
trans-p-[5-(4-Pentylcyclohexyl)-2-pyrimidinyl]benzonitril;
trans-p-[5-(4-Heptylcyclohexyl)-2-pyrimidinyl]benzonitril.

Die Verbindungen der Formel I können erfindungsgemäß dadurch hergestellt werden, daß man

a) eine Verbindung der allgemeinen Formel

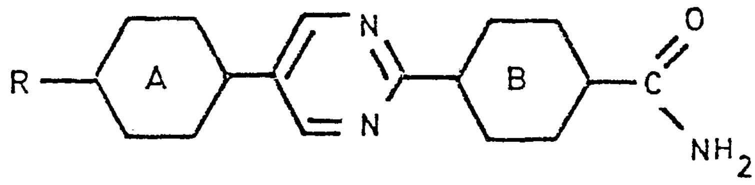

II

worin A, B und R die obige Bedeutung haben, dehydratisiert, oder

b) zur Herstellung einer Verbindung der Formel IA eine Verbindung der allgemeinen Formel

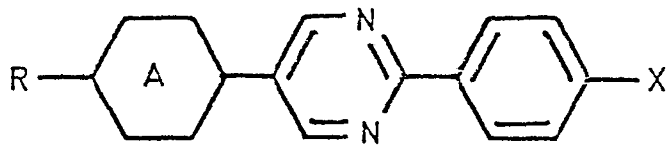

III

worin R und A die obige Bedeutung haben und X Fluor, Chlor oder Brom darstellt,

mit Kupfer-(I)-cyanid oder mit Natrium- oder Kaliumcyanid umsetzt.

Die Dehydratisierung einer Verbindung der Formel II kann mit irgendeinem geeigneten Dehydratisierungsmittel wie z. B. mit Phosphoroxychlorid, Phosphorpentoxyd, Thionylchlorid, Acetanhydrid oder insbesondere Benzolsulfochlorid und dergleichen durchgeführt werden. Die Dehydratisierung kann in einem inerten organischen Lösungsmittel, wie beispielsweise einem Kohlenwasserstoff oder Halogenkohlenwasserstoff, gegebenenfalls in Gegenwart einer Base, wie Natriumacetat, Pyridin oder Triäthylamin, erfolgen. Sie kann jedoch auch ohne organische Lösungsmittel durchgeführt werden. Die Reaktionstemperatur liegt vorzugsweise zwischen 50° C und der Rückflußtemperatur des Reaktionsgemisches. Der Druck ist nicht kritisch und die Reaktion wird mit Vorteil bei Atmosphärendruck durchgeführt.

Die Umsetzung einer Verbindung der Formel III mit Kupfer-(I)-cyanid, Natrium- oder Kaliumcyanid wird zweckmäßig in einem inerten organischen Lösungsmittel wie beispielsweise in Äthylenglykol, Tetrahydrofuran, Dimethylformamid, Dimethylsulfoxid, Pyridin oder Acetonitril durchgeführt. Temperatur und Druck sind keine kritischen Aspekte in dieser Reaktion. Zweckmäßigerweise werden Atmosphärendruck und eine Temperatur zwischen Raumtemperatur und der Siedetemperatur des Reaktionsgemisches angewendet. Vorzugsweise steht in der Verbindung der Formel III X für Brom.

Die Herstellung der Ausgangsstoffe der Formeln II und III wird anhand der nachstehenden Reaktionsschemata A – D veranschaulicht.

Im Rahmen der vorliegenden Erfindung bedeutet der Ausdruck »geradkettiges Alkyl mit 1 bis 10 Kohlenstoffatomen« insbesondere Methyl, Äthyl, Propyl, Butyl, Pentyl, Hexyl, Heptyl, Octyl, Nonyl und Decyl.

Der Ausdruck »geradkettiges Alkoxy mit 1 bis 10 Kohlenstoffatomen« bedeutet Methoxy, Äthoxy, Propyloxy, Butyloxy, Pentyloxy, Hexyloxy, Heptyloxy, Octyloxy, Nonyloxy und Decyloxy.

Der Ausdruck »verzweigte Alkylgruppe

$C_2H_5-CH(CH_3)-(CH_2)_n-$,

worin n eine ganze Zahl von 1 bis 3 ist«, bedeutet 2-Methylbutyl, 3-Methylpentyl und 4-Methylhexyl.

Schema A

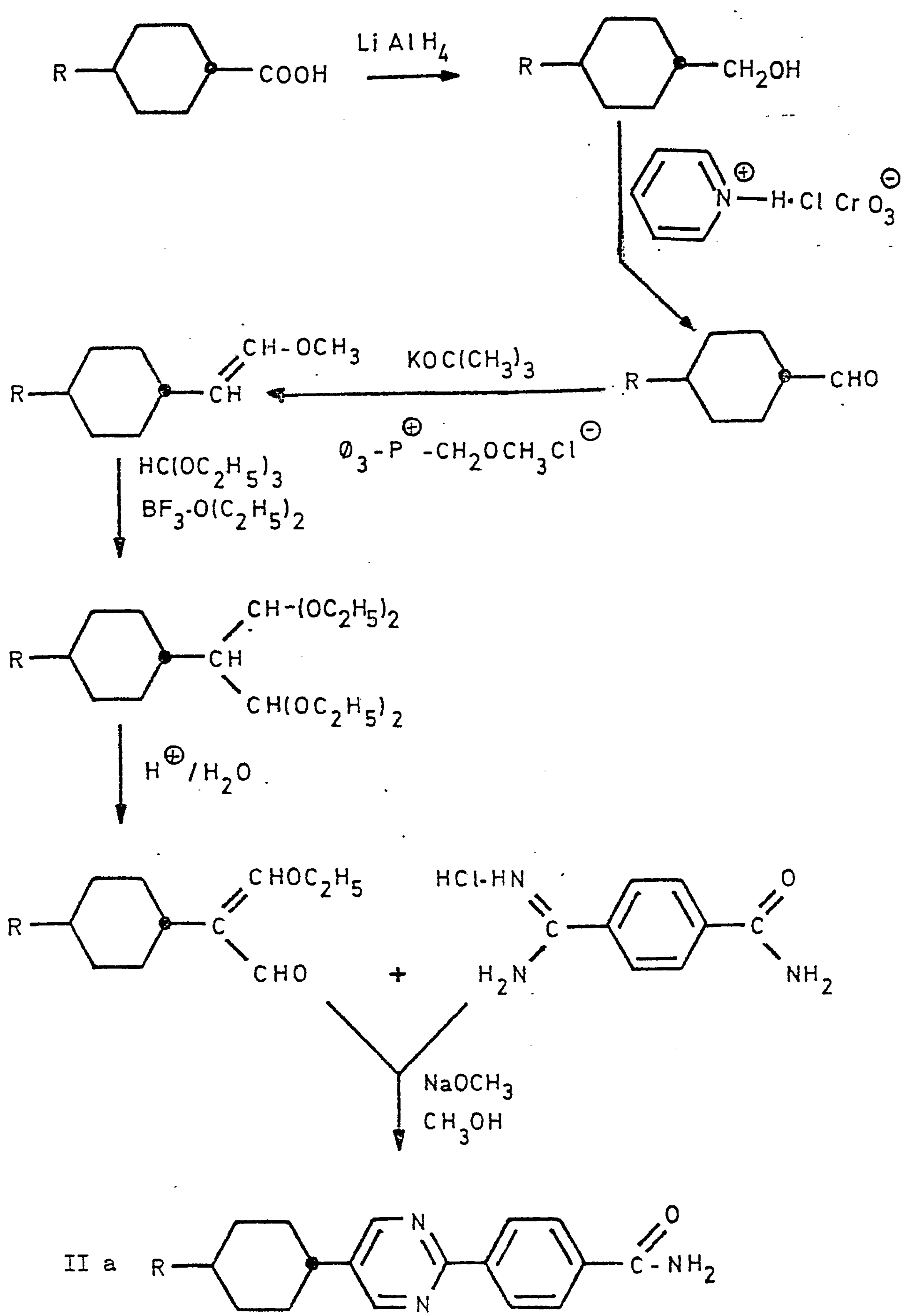
LiAlH4
KOC(CH3)3
Ø3-P⊕-CH2OCH3Cl⊖
HC(OC2H5)3
BF3·O(C2H5)2
H⊕/H2O
+
NaOCH3
CH3OH

II a

Schema B

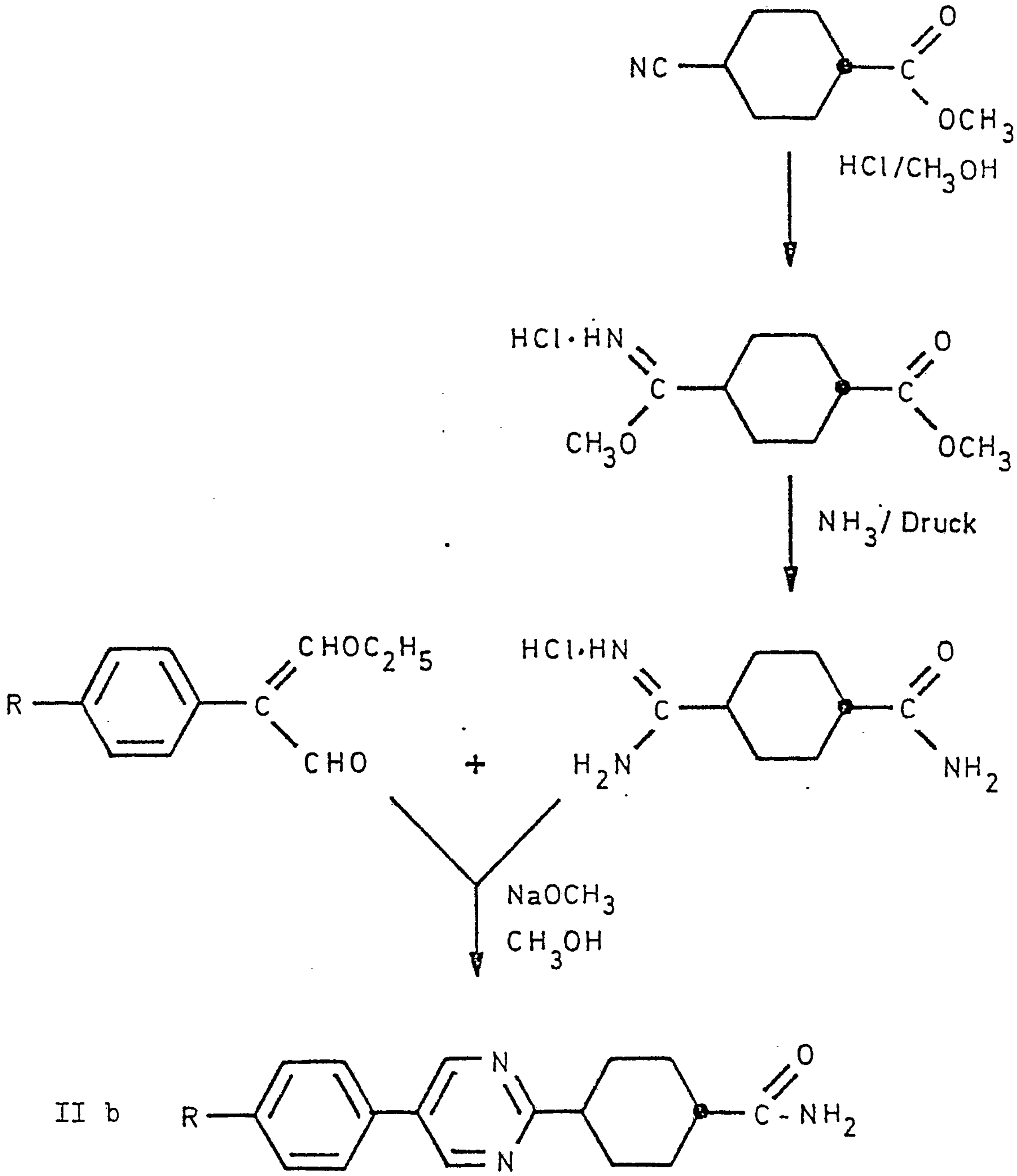


II b

Schema C

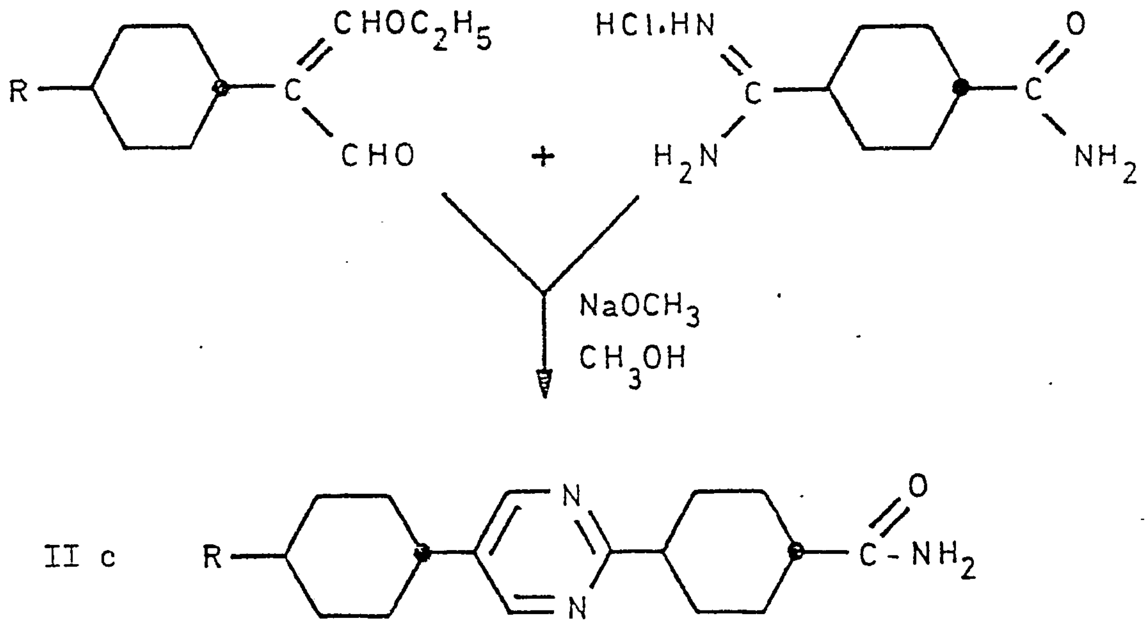


II c

Schema D

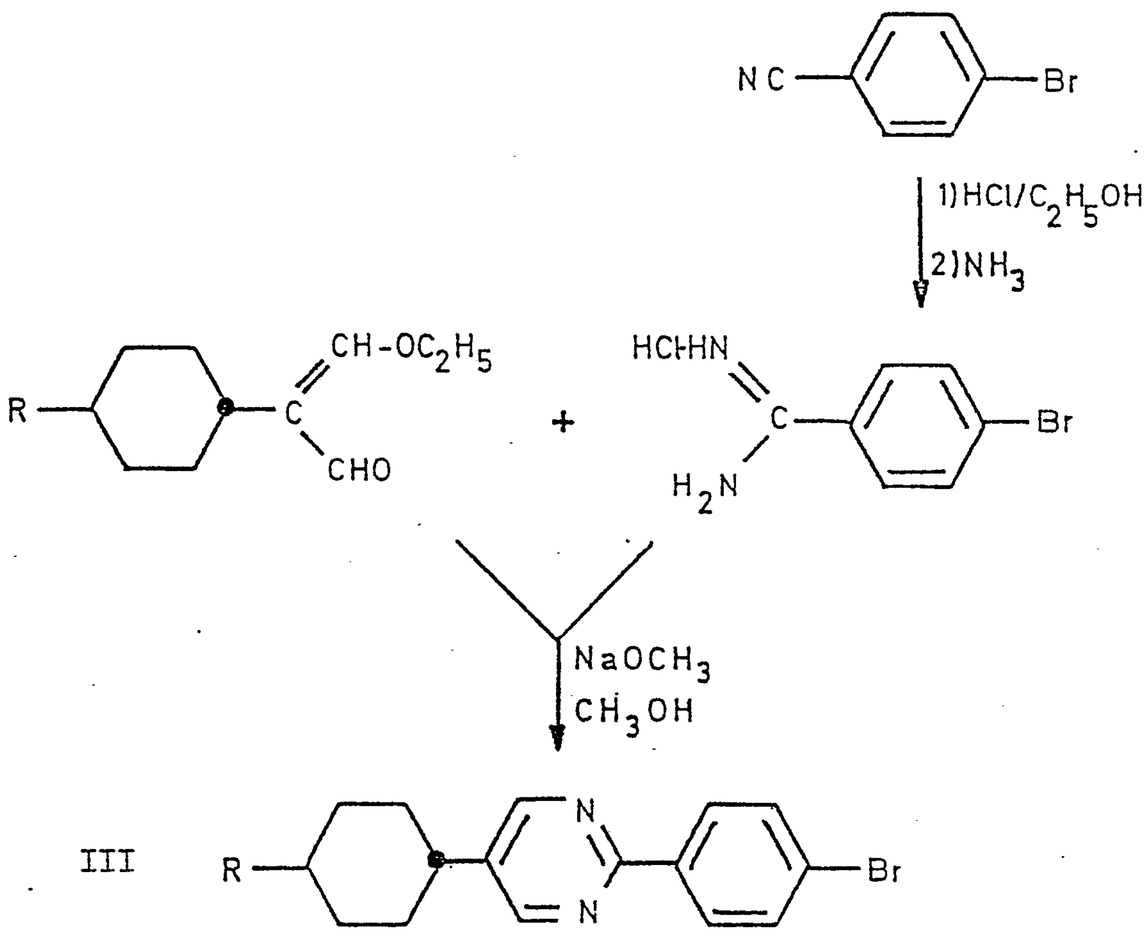


III

Die Ausgangssäuren mit verzweigter Seitenkette R können nach dem von Gray und McDonnell in der deutschen Offenlegungsschrift 2 736 772 und in Mol. Cryst. Liq. Cryst. 37 (1976) 189 beschriebenen Verfahren hergestellt werden.

Die Verbindungen der Formel I können in Form ihrer Gemische mit anderen nematischen oder nicht nematischen Substanzen verwendet werden, wie z. B. mit Substanzen aus den Klassen der Schiffschen Basen, Azo- oder Azoxybenzole, Phenylbenzoate, Cyclohexancarbonsäure-phenylester, Bi- und Terphenyle, Phenylcyclohexane, Zimtsäurederivate, Phenyl- und Diphenylpyrimidine und dergleichen. Derartige Verbindungen sind dem Fachmann geläufig und bekannt, z. B. aus den deutschen Offenlegungsschriften 2 306 738, 2 306 739, 2 429 093, 2 356 085, 2 636 684, 2 459 374, 2 547 737, 2 641 724, 2 708 276, 2 811 001. Viele derartige nematische oder nicht nematische Substanzen sind zudem im Handel erhältlich.

Die Verbindungen der Formel I, worin R eine geradkettige Alkyl- oder Alkoxygruppe bedeutet, liegen in flüssigkristallinen Mischungen für elektro-optische Zwecke in einem Gewichtsverhältnis vor, das vorzugsweise der eutektischen Zusammensetzung entspricht. Der Anteil dieser Verbindungen in einer flüssigkristallinen Mischung liegt jedoch in der Regel zwischen etwa 1 und etwa 50 Molprozenten und vorzugsweise zwischen etwa 5 und etwa 30 Molprozenten.

Die Verbindungen der Formel I, worin R eine verzweigte Alkylgruppe bedeutet, liegen in cholesterischen Mischungen für elektro-optische Zwecke in einem Gewichtsverhältnis vor, das vorzugsweise durch die gewünschte Ganghöhe der cholesterischen Mischung gegeben ist. Der Anteil dieser Verbindungen in einer farbstoffhaltigen cholesterischen Mischung liegt in der Regel unter etwa 15 Molprozenten und vorzugsweise unter etwa 5 Molprozenten. Für Mischungen, die keine Farbstoffe enthalten, werden hingegen häufig kleinere Mengen, vorzugsweise weniger als etwa 2 Molprozente dieser Verbindungen verwendet.

Durch Verwendung von Verbindungen der Formel I können insbesondere Mischungen hergestellt werden, welche gegenüber vorbekannten Mischungen z. T. erhebliche Vorteile aufweisen. So kann z. B. der Anteil der Verbindungen der Formel I in einer Mischung wesentlich höher sein als diejenige von vorbekannten Verbindungen mit ähnlich hohen Klärpunkten. Dies führt dazu, daß z. B. mehrere erfindungsgemäße Verbindungen in einer Mischung kombiniert werden können, was zu sehr hohen Klärpunkten führt, ohne daß die Mischung dadurch smektische oder fest kristalline Tendenzen erhält. Weiterhin weisen Mischungen, welche erfindungsgemäße Verbindungen enthalten, eine sehr geringe Temperaturabhängigkeit der Schwellenspannung auf. Dies ist von Vorteil, da Mischungen mit geringer Temperaturabhängigkeit der elektro-optischen Transmissionskurven (in Drehzellen) insbesondere für den Multiplexbetrieb gut geeignet sind.

Ein weiterer Vorteil der erfindungsgemäßen Verbindungen liegt darin, daß sie die Herstellung von Mischungen mit hohen Klärpunkten und dennoch niedrigen Viskositäten ermöglichen.

Die Verbindungen der Formel I können außerdem in Form farbstoffhaltiger Flüssigkristallmischungen verwendet werden. Um bei Flüssigkristalldisplays, bei welchen irgendeine Form des »Guest-Host-Effektes« verwendet wird, hohe Kontraste zu erreichen, ist es wichtig, daß die gelösten dichroitischen Farbstoffe einen großen Ordnungsgrad aufweisen. Dieser hängt sowohl von der Flüssigkristallmatrix als auch von den verwendeten dichroitischen Farbstoffen ab. Da nun die Flüssigkristallmischungen, welche eine oder mehrere Verbindungen der Formel I und gegebenenfalls weitere nematische und/oder nicht nematische Substanzen enthalten, einen großen Ordnungsgrad besitzen, sind sie als Flüssigkristallmatrix für farbstoffhaltige Mischungen besonders geeignet.

Als Farbstoffe für diese erfindungsgemäßen Mischungen sind vor allem Verbindungen mit länglichen Molekülen und einem relativ starren Molekülteil, wie beispielsweise Azo- oder Azoxyfarbstoffe, Polyene und Schiffsche Basen, überraschenderweise aber auch Anthrachinonderivate geeignet. Bevorzugte Farbstoffe sind die Verbindungen der allgemeinen Formeln:

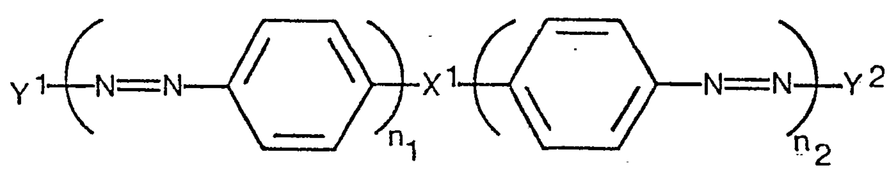

IV

worin $n_1$ und $n_2$ ganze Zahlen von 0 bis 2 und $X^1$ eine Azo- oder Azoxygruppe bedeuten, $Y^1$ und $Y^2$ gleich oder verschieden sein können und einen der Substituenten

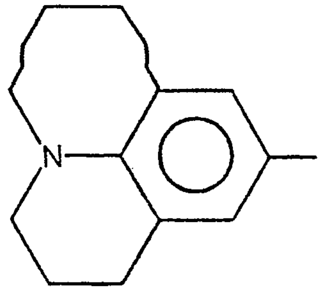 V oder 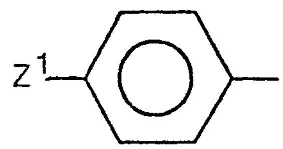 VI

bezeichnen und $Z^1$ Wasserstoff, Cyano, Nitro, Phenyl, p-substituiertes Phenyl, Hydroxy, Alkoxy, Amino, Dialkylamino oder Pyrrolidyl darstellt; oder Derivate der Formel IV, bei denen einer der Benzolringe zusätzlich eine oder mehrere der Gruppen Halogen, Methyl, halogensubstituiertes Methyl oder Methoxy besitzt;

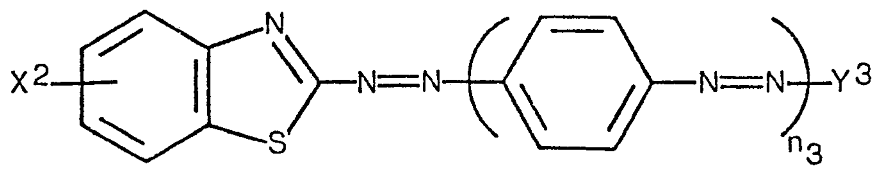 VII

worin $n_3$ 0 oder 1 ist, $Y^3$ einen Substituenten der Formel V oder VI bezeichnet, worin $Z^1$ Dialkylamino oder Pyrrolidyl bedeutet, und $X^2$ Wasserstoff, Cyano, Nitro, Halogen, Alkyl, Alkoxy, eine gegebenenfalls substituierte Phenyl- oder Benzothiazol-2-ylgruppe, Alkoxycarbonyl oder Alkylsulfonyl in 5- oder 6-Stellung darstellt;

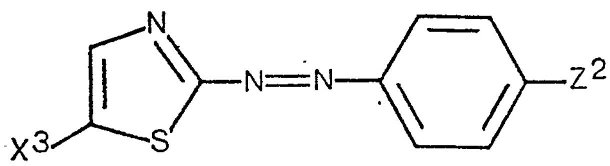 VIII

worin $X^3$ Cyano, Nitro oder Alkyl bedeutet, und $Z^2$ Hydroxy, Alkoxy, Aryl oder eine gegebenenfalls Alkyl- oder Aryl-substituierte Amino-, Pyrrolidyl- oder Piperidylgruppe darstellt; oder Derivate der Formel VIII, bei denen der Benz9lring zusätzlich eine oder mehrere der Gruppen Halogen, Hydroxy, Methyl oder Methoxy besitzt;

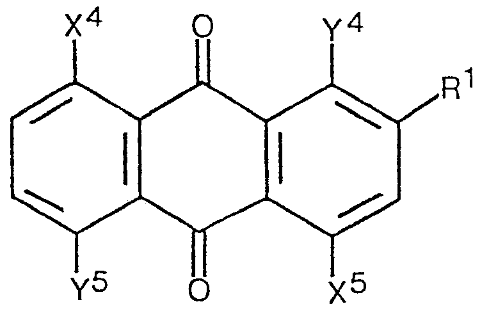 IX

worin $X^4$ und $X^5$ Hydroxy, $Y^4$ und $Y^5$ Amino und $R^1$ einen Substituenten der Formel VI oder $X^4$, $Y^5$ und $R^1$ Wasserstoff, $X^5$ Hydroxy und $Y^4$ die Gruppe $-NHR^2$ oder $X^4$, $X^5$ und $R^1$ Wasserstoff und $Y^4$ und $Y^5$ die Gruppe $-NHR^2$ bedeuten, $R^2$ einen Substituenten der Formel VI bezeichnet und $Z^1$ in den Substituenten der Formel VI eine Alkyl-, Alkoxy- oder Dialkylamino-Gruppe darstellt;

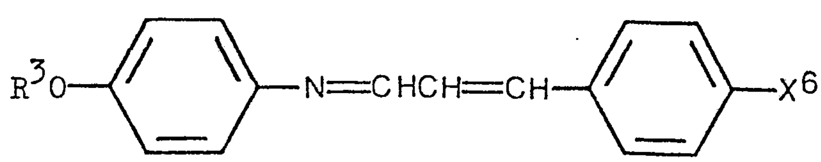 X

worin $R^3$ eine Alkyl- und $X^6$ eine Cyano- oder eine Nitrogruppe bedeutet;

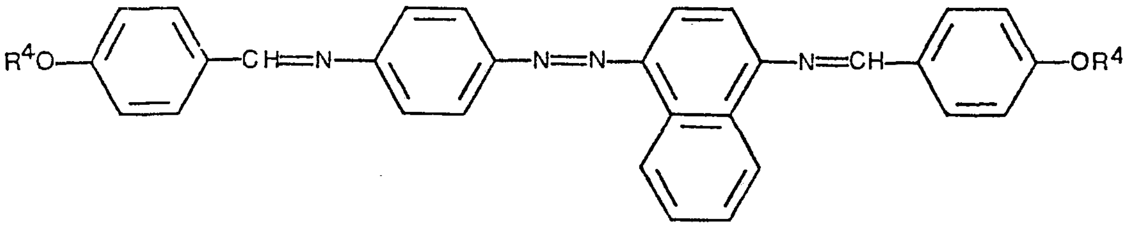

XI

worin $R^4$ eine Alkylgruppe darstellt; und

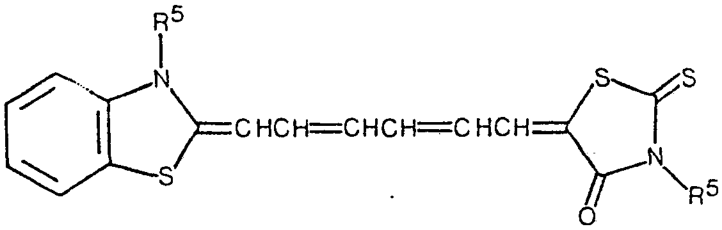

XII

worin $R^5$ eine Alkylgruppe darstellt.

Diese Verbindungen sind bekannte oder Analoge bekannter Verbindungen.
Die folgenden Verbindungen sind besonders bevorzugte Farbstoffe:

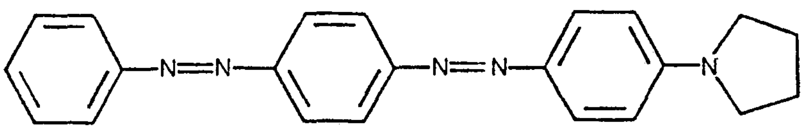

IVa

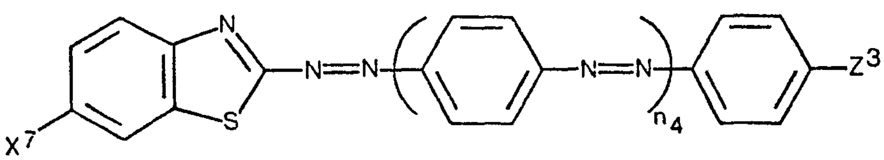

VIIa

worin $n_4$ 0 oder 1 ist, $Z^3$ die Dimethylamino- oder die Diäthylamino-Gruppe bedeutet und $X^7$ Wasserstoff, Methyl, Äthoxy, n-Butyloxy, Nitro oder n-Butylsulfonyl darstellt;

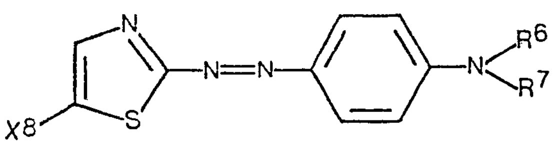

VIIIa

worin $R^6$, $R^7$ und $X^8$ Methyl oder $R^6$ Methyl, $X^8$ Nitro und $R^7$ Methyl, Phenyl oder p-n-Butylphenyl bedeuten;

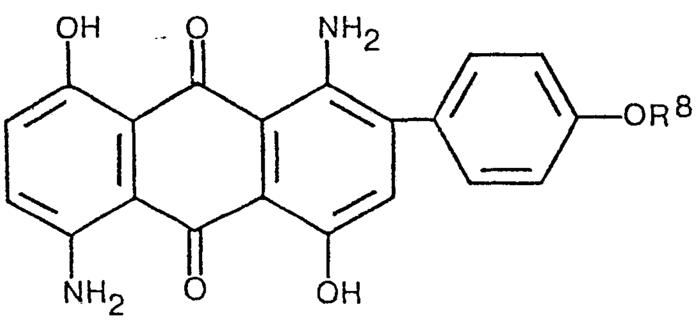

IXa

worin $R^8$ n-Alkyl mit 1 bis 10 Kohlenstoffatomen bedeutet;

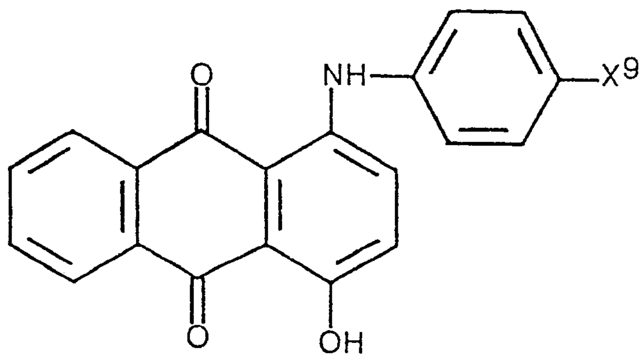

IXb

worin $X^9$ n-Butyl, n-Nonyloxy oder Dimethylamino bedeutet;

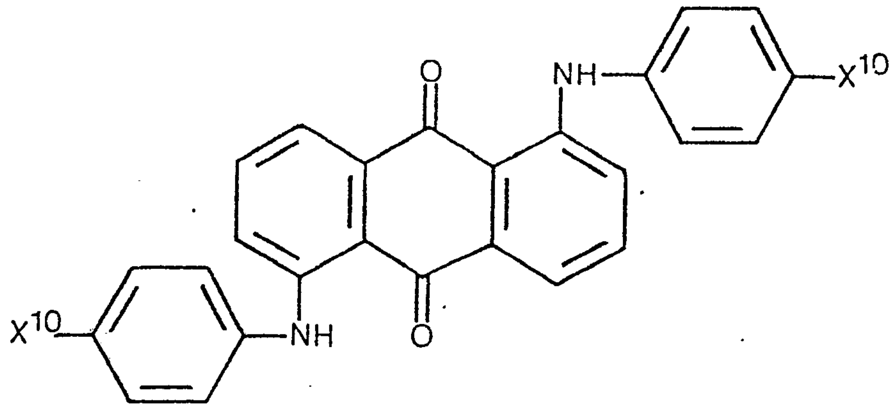

IXc

worin $X^{10}$ Äthyl, n-Butyl, Isopropyl, Pentyloxy oder Dimethylamino bedeutet;
die Verbindung der Formel X, worin $R^3$ n-Butyl und $X^6$ Nitro bedeutet;
die Verbindung der Formel XI, worin $R^4$ Äthyl bedeutet;
und die Verbindung der Formel XII, worin $R^5$ Äthyl bedeutet.

Die erfindungsgemäßen farbstoffhaltigen Mischungen können 1 bis etwa 4 Farbstoffe enthalten. Der Anteil der Farbstoffe in einer flüssigkristallinen Mischung liegt für Anthrachinone zwischen etwa 0,2 und etwa 3 Gewichtsprozenten, vorzugsweise zwischen etwa 1 und etwa 2 Gewichtsprozenten, und für die übrigen Farbstoffe zwischen etwa 0,1 und etwa 2 Gewichtsprozenten, vorzugsweise zwischen etwa 0,5 und etwa 1 Gewichtsprozent.

Die Herstellung von Gemischen, welche u. a. Verbindungen der Formel I sowie andere nematische und/oder nicht nematische Verbindungen und/oder einen oder mehrere Farbstoffe enthalten, kann in an sich bekannter Weise erfolgen, z. B. durch Erhitzen einer Mischung der Komponenten auf eine Temperatur knapp oberhalb des Klärpunktes und anschließendes Abkühlen.

Als Beispiele von bevorzugten Gemischen können folgende genannt werden, wobei die Prozente in Mol-Prozenten ausgedrückt sind und die Temperaturen in Grad Celsius, sofern nicht ausdrücklich etwas anderes angegeben wird, und S den Ordnungsgrad bedeutet.

Mischung 1

| | |
|---|---|
| 19% | trans-p-(4-Propylcyclohexyl)benzonitril, |
| 34% | trans-p-(4-Pentylcyclohexyl)benzonitril, |
| 22% | trans-p-(4-Heptylcyclohexyl)benzonitril, |
| 3% | trans-4-[5-(p-Butylphenyl)-2-pyrimidinyl]cyclohexancarbonitril, |
| 10% | trans-4-[5-(p-Hexylphenyl)-2-pyrimidinyl]cyclohexancarbonitril, |
| 12% | trans-4-[5-(p-Octylphenyl)-2-pyrimidinyl]cyclohexancarbonitril, |
| | Smp. $<0°$; Klp. 77° |

Mischung 2

| | |
|---|---|
| 16% | trans-4-Propylcyclohexancarbonsäure-p-cyanophenylester, |
| 9,5% | trans-4-Butylcyclohexancarbonsäure-p-cyanophenylester, |

14,5% trans-4-Pentylcyclohexancarbonsäure-p-cyanophenylester,
8% p-Butylbenzoesäure-p'-cyanophenylester,
8% p-(5-Pentyl-2-pyrimidinyl)benzonitril,
15% p-(5-Heptyl-2-pyrimidinyl)benzonitril,
12% trans-p-[5-(4-Äthylcyclohexyl)-2-pyrimidinyl]benzonitril,
17% trans-p-[5-(4-Heptylcyclohexyl)-2-pyrimidinyl]benzonitril,
Smp. <0°; Klp. 109°

Mischung 3

8% p-(5-Pentyl-2-pyrimidinyl)benzonitril,
14% p-(5-Heptyl-2-pyrimidinyl)benzonitril,
9% 4'-Octyloxy-4-cyanobiphenyl,
40% trans-p-(4-Pentylcyclohexyl)benzonitril,
12% trans-p-[5-(4-Äthylcyclohexyl)-2-pyrimidinyl]benzonitril,
17% trans-p-[5-(4-Heptylcyclohexyl)-2-pyrimidinyl]benzonitril,
Smp. <0°; Klp. 102°

Mischung 4

8% p-(4-Pentyl-2-pyrimidinyl)benzonitril,
12% p-(5-Heptyl-2-pyrimidinyl)benzonitril,
46% 4'-Pentyl-4-cyanobiphenyl,
8% 4'-Octyloxy-4-cyanobiphenyl,
11% trans-p-[5-(4-Äthylcyclohexyl)-2-pyrimidinyl]benzonitril,
15% trans-p-[5-(4-Heptylcyclohexyl)-2-pyrimidinyl]benzonitril,
Smp. <0°; Klp. 94°

Mischung 5

5% p-Butylbenzoesäure-p'-cyanophenylester,
6% p-(5-Pentyl-2-pyrimidinyl)benzonitril,
43% 4'-Pentyl-4-cyanobiphenyl,
12% trans-4-Propylcyclohexancarbonsäure-p-cyanophenylester,
7% trans-4-Butylcyclohexancarbonsäure-p-cyanophenylester,
11% trans-4-Pentylcyclohexancarbonsäure-p-cyanophenylester,
11% trans-p-[5-(4-Äthylcyclohexyl)-2-pyrimidinyl]benzonitril,
5% trans-p-[5-(4-Propylcyclohexyl)-2-pyrimidinyl]benzonitril,
Smp. <0°; Klp. 75°

Mischung 6

7% p-(5-Pentyl-2-pyrimidinyl)benzonitril,
46% 4'-Pentyl-4-cyanobiphenyl,
8% trans-4-Butylcyclohexancarbonsäure-p-cyanophenylester,
12% trans-4-Pentylcyclohexancarbonsäure-p-cyanophenylester,
11% trans-p-[5-(4-Äthylcyclohexyl)-2-pyrimidinyl]benzonitril,
16% trans-p-[5-(4-Heptylcyclohexyl)-2-pyrimidinyl]benzonitril,
Smp. <0°; Klp. 97°

Mischung 7

8% p-(5-Pentyl-2-pyrimidinyl)benzonitril,
51% 4'-Pentyl-4-cyanobiphenyl,
9% trans-4-Butylcyclohexancarbonsäure-p-cyanophenylester,
14% trans-4-Pentylcyclohexancarbonsäure-p-cyanophenylester,
12% trans-p-[5-(4-Äthylcyclohexyl)-2-pyrimidinyl]benzonitril,
6% trans-p-[5-(4-Propylcyclohexyl)-2-pyrimidinyl]benzonitril,
Smp. <0°; Klp. 78°

Mischung 8

9% p-(5-Pentyl-2-pyrimidinyl)benzonitril,
55% 4'-Pentyl-4-cyanobiphenyl,
16% trans-5-Pentylcyclohexancarbonsäure-p-cyanophenylester,
13% trans-p-[5-(4-Äthylcyclohexyl)-2-pyrimidinyl]benzonitril,
7% trans-p-[5-(4-Propylcyclohexyl)-2-pyrimidinyl]benzonitril,
Smp. <0°; Klp. 80°

Mischung 9

8% p-(5-Pentyl-2-pyrimidinyl)benzonitril,
14% p-(5-Heptyl-2-pyrimidinyl)benzonitril,
50% 4'-Pentyl-4-cyanobiphenyl,
10% 4'-Octyloxy-4-cyanobiphenyl,
12% trans-p-[5-(4-Äthylcyclohexyl)-2-pyrimidinyl]benzonitril,
6% trans-p-[5-(4-Propylcyclohexyl)-2-pyrimidinyl]benzonitril,
Smp. <0°; Klp. 77°

Mischung 10

7% p-Butylbenzoesäure-p'-cyanophenylester,
7% p-(5-Pentyl-2-pyrimidinyl)benzonitril,
39% trans-p-(4-Pentylcyclohexyl)benzonitril,
15% trans-4-Propylcyclohexancarbonsäure-p-cyanophenylester,
14% trans-4-Pentylcyclohexancarbonsäure-p-cyanophenylester,
12% trans-p-[5-(4-Äthylcyclohexyl)-2-pyrimidinyl]benzonitril,
6% trans-p-[5-(4-Propylcyclohexyl)-2-pyrimidinyl]benzonitril,
Smp. <20°; Klp. 90°

Mischung 11

6% p-Butylbenzoesäure-p'-cyanophenylester,
7% p-(5-Pentyl-2-pyrimidinyl)benzonitril,
38% trans-p-(4-Pentylcyclohexyl)benzonitril,
14% trans-4-Propylcyclohexancarbonsäure-p-cyanophenylester,
12% trans-4-Pentylcyclohexancarbonsäure-p-cyanophenylester,
11,5% p-[5-(4-Butylphenyl)-2-pyrimidinyl]benzonitril,
11,5% trans-p-[5-(4-Äthylcyclohexyl)-2-pyrimidinyl]benzonitril,
Smp. <20°; Klp. 94°

Mischung 12

4% p-Butylbenzoesäure-p'-cyanophenylester,
4% p-(5-Pentyl-2-pyrimidinyl)benzonitril,
8% p-(5-Heptyl-2-pyrimidinyl)benzonitril,
35% 4'-Pentyl-4-cyanobiphenyl,
27% trans-p-(4-Pentylcyclohexyl)benzonitril,
5% trans-4-Butylcyclohexancarbonsäure-p-cyanophenylester,
8% p-[5-(4-Butylphenyl)-2-pyrimidinyl]benzonitril,
9% trans-p-[5-(4-Äthylcyclohexyl)-2-pyrimidinyl]benzonitril,
Smp. < −10°; Klp. 72°

Mischung 13

5% p-(5-Pentyl-2-pyrimidinyl)benzonitril,
9% p-(5-Heptyl-2-pyrimidinyl)benzonitril,
38% 4'-Pentyl-4-cyanobiphenyl,
30% trans-p-(4-Pentylcyclohexyl)benzonitril,
9% p-[5-(4-Butylphenyl)-2-pyrimidinyl]benzonitril,

9% trans-p-[5-(4-Äthylcyclohexyl)-2-pyrimidinyl]benzonitril,
Smp. < −10°; Klp. 75°

Mischung 14

5% p-(5-Pentyl-2-pyrimidinyl)benzonitril,
9% p-(5-Heptyl-2-pyrimidinyl)benzonitril,
38% 4′-Pentyl-4-cyanobiphenyl,
10,4% trans-4-Propylcyclohexancarbonsäure-p-cyanophenylester,
5,6% trans-4-Butylcyclohexancarbonsäure-p-cyanophenylester,
9% trans-4-Pentylcyclohexancarbonsäure-p-cyanophenylester,
10% trans-p-[5-(4-Äthylcyclohexyl)-2-pyrimidinyl]benzonitril,
13% trans-p-[5-(4-Heptylcyclohexyl)-2-pyrimidinyl]benzonitril,
Smp. < −10°; Klp. 91°

Mischung 15

5,5% p-(5-Pentyl-2-pyrimidinyl)benzonitril,
10% p-(5-Heptyl-2-pyrimidinyl)benzonitril,
33% trans-p-(4-Pentylcyclohexyl)benzonitril,
11,5% trans-4-Propylcyclohexancarbonsäure-p-cyanophenylester,
6% trans-4-Butylcyclohexancarbonsäure-p-cyanophenylester,
10% trans-4-Pentylcyclohexancarbonsäure-p-cyanophenylester,
10% trans-p-[5-(4-Äthylcyclohexyl)-2-pyrimidinyl]benzonitril,
14% trans-p-[5-(4-Heptylcyclohexyl)-2-pyrimidinyl]benzonitril,
Smp. < −10°; Klp. 98°

Mischung 16

7% p-(5-Pentyl-2-pyrimidinyl)benzonitril,
13% p-(5-Heptyl-2-pyrimidinyl)benzonitril,
47% 4′-Pentyl-4-cyanobiphenyl,
9% 4′-Octyloxy-4-cyanobiphenyl,
8% 4″-Pentyl-4-cyano-p-terphenyl,
16% trans-p-[5-(4-Heptylcyclohexyl)-2-pyrimidinyl]benzonitril,
Smp. <0°; Klp. 95°

Mischung 17

5% p-Butylbenzoesäure-p′-cyanophenylester,
5% p-(5-Pentyl-2-pyrimidinyl)benzonitril,
40% 4′-Pentyl-4-cyanobiphenyl,
11% trans-4-Propylcyclohexancarbonsäure-p-cyanophenylester,
6% trans-4-Butylcyclohexancarbonsäure-p-cyanophenylester,
9% trans-4-Pentylcyclohexancarbonsäure-p-cyanophenylester,
10% trans-p-[5-(4-Äthylcyclohexyl)-2-pyrimidinyl]benzonitril,
14% trans-p-[5-(4-Heptylcyclohexyl)-2-pyrimidinyl]benzonitril,
Smp. < −10°; Klp. 93°

Mischung 18

6,5% p-(5-Pentyl-2-pyrimidinyl)benzonitril,
12% p-(5-Heptyl-2-pyrimidinyl)benzonitril,
47% 4′-Pentyl-4-cyanobiphenyl,
8% 4′-Octyloxy-4-cyanobiphenyl,
11% p-[5-(4-Butylphenyl)-2-pyrimidinyl]benzonitril,
15,5% trans-p-[5-(4-Heptylcyclohexyl)-2-pyrimidinyl]benzonitril,
Smp. <0°; Klp. 98°

Mischung 19

6% p-(5-Pentyl-2-pyrimidinyl)benzonitril,
42% 4'-Pentyl-4-cyanobiphenyl,
7% trans-4-Butylcyclohexancarbonsäure-p-cyanophenylester,
10% trans-4-Pentylcyclohexancarbonsäure-p-cyanophenylester,
10% p-[5-(4-Butylphenyl)-2-pyrimidinyl]benzonitril,
10,5% trans-p-[5-(4-Äthylcyclohexyl)-2-pyrimidinyl]benzonitril,
14,5% trans-p-[5-(4-Heptylcyclohexyl)-2-pyrimidinyl]benzonitril,
Smp. <0°; Klp. 112°

Mischung 20

8,1% p-(5-Pentyl-2-pyrimidinyl)benzonitril,
15,1% p-(5-Heptyl-2-pyrimidinyl]benzonitril,
16,2% trans-4-Propylcyclohexancarbonsäure-p-cyanophenylester,
9,7% trans-4-Butylcyclohexancarbonsäure-p-cyanophenylester,
14,8% trans-4-Pentylcyclohexancarbonsäure-p-cyanophenylester,
23,6% trans-4-Pentylcyclohexancarbonsäure-p-methoxyphenylester,
12,5% trans-p-[5-(4-Äthylcyclohexyl)-2-pyrimidinyl]benzonitril,
Smp. <0°; Klp. 76°

Mischung 21

7% p-Butylbenzoesäure-p'-cyanophenylester,
8% p-(5-Pentyl-2-pyrimidinyl)benzonitril,
53% 4'-Pentyl-4-cyanobiphenyl,
14% trans-4-Pentylcyclohexancarbonsäure-p-cyanophenylester,
12% trans-p-[5-(4-Äthylcyclohexyl)-2-pyrimidinyl]benzonitril,
6% trans-p-[5-(4-Propylcyclohexyl)-2-pyrimidinyl]benzonitril,
Smp. <0°; Klp. 77°

Mischung 22

5% p-(5-Pentyl-2-pyrimidinyl)benzonitril,
10% p-(5-Heptyl-2-pyrimidinyl)benzonitril,
40% 4'-Pentyl-4-cyanobiphenyl,
30% trans-p-(4-Pentylcyclohexyl)benzonitril,
10% trans-p-[5-(4-Äthylcyclohexyl)-2-pyrimidinyl]benzonitril,
5% trans-p-[5-(4-Propylcyclohexyl)-2-pyrimidinyl]benzonitril,
Smp. < −10°; Klp. 70°

Mischung 23

42% 4'-Pentyl-4-cyanobiphenyl,
33% trans-p-(4-Pentylcyclohexyl)benzonitril,
10,5% p-[5-(4-Butylphenyl)-2-pyrimidinyl]benzonitril,
10% trans-p-[5-(4-Äthylcyclohexyl)-2-pyrimidinyl]benzonitril,
4,5% trans-p-[5-(4-Propylcyclohexyl)-2-pyrimidinyl]benzonitril,
Smp. < −10°; Klp. 87°

Mischung 24

5% p-Butylbenzoesäure-p'-cyanophenylester,
6% p-(5-Pentyl-2-pyrimidinyl)benzonitril,
11% p-(5-Heptyl-2-pyrimidinyl)benzonitril,
12% trans-4-Propylcyclohexancarbonsäure-p-cyanophenylester,
7% trans-4-Butylcyclohexancarbonsäure-p-cyanophenylester,
10% trans-4-Pentylcyclohexancarbonsäure-p-cyanophenylester,
24% trans-4-Pentylcyclohexancarbonsäure-p-(propyloxy)phenylester,

10,5% trans-p-[5-(4-Äthylcyclohexyl)-2-pyrimidinyl]benzonitril,
14,5% trans-p-[5-(4-Heptylcyclohexyl)-2-pyrimidinyl]benzonitril,
Smp. <5°; Klp. 99°

Mischung 25

5% p-Butylbenzoesäure-p'-cyanophenylester,
6% p-[5-Pentyl-2-pyrimidinyl]benzonitril,
11% p-[5-Heptyl-2-pyrimidinyl]benzonitril,
12% trans-4-Propylcyclohexancarbonsäure-p-cyanophenylester,
7% trans-4-Butylcyclohexancarbonsäure-p-cyanophenylester,
10% trans-4-Pentylcyclohexancarbonsäure-p-cyanophenylester,
24% trans-4-Pentylcyclohexancarbonsäure-p-(propyloxy)phenylester,
10,5% p-[5-(4-Butylphenyl)-2-pyrimidinyl]benzonitril,
14,5% trans-p-[5-(4-Heptylcyclohexyl)-2-pyrimidinyl]benzonitril,
Smp. <20°; Klp. 102°

Mischung 26

4,4% p-(5-Pentyl-2-pyrimidinyl)benzonitril,
7,8% p-(5-Heptyl-2-pyrimidinyl)benzonitril,
35,6% 4'-Pentyl-4-cyanobiphenyl,
9,3% trans-4-Propylcyclohexancarbonsäure-p-cyanophenylester,
7,7% trans-4-Pentylcyclohexancarbonsäure-p-cyanophenylester,
21,0% trans-4-Butylcyclohexancarbonsäure-p-(hexyloxy)phenylester,
8,8% trans-p-[5-(4-Äthylcyclohexyl)-2-pyrimidinyl]benzonitril,
5,4% trans-p-[5-(4-Heptylcyclohexyl)-2-pyrimidinyl]benzonitril,
Smp. < −10°; Klp. 72°

Mischung 27

4,4% p-(5-Pentyl-2-pyrimidinyl)benzonitril,
35,3% 4'-Pentyl-4-cyanobiphenyl,
9,7% trans-4-Propylcyclohexancarbonsäure-p-cyanophenylester,
7,7% trans-4-Pentylcyclohexancarbonsäure-p-cyanophenylester,
13,6% trans-4-Pentylcyclohexancarbonsäure-p-(methoxy)phenylester,
8,5% 2-(4-Cyanophenyl)-5-(4-butylphenyl)pyrimidin,
8,8% trans-p-[5-(4-Äthylcyclohexyl)-2-pyrimidinyl]benzonitril,
12,0% trans-p-[5-(4-Heptylcyclohexyl)-2-pyrimidinyl]benzonitril,
Smp. < −10°; Klp. 104°

Mischung 28

38,2% 4'-Pentyl-4-cyanobiphenyl,
4,9% p-(5-Pentyl-2-pyrimidinyl)benzonitril,
8,8% p-(5-Heptyl-2-pyrimidinyl)benzonitril,
10,3% trans-4-Propylcyclohexancarbonsäure-p-cyanophenylester,
8,7% trans-4-Pentylcyclohexancarbonsäure-p-cyanophenylester,
23,2% trans-4-Butylcyclohexancarbonsäure-p-(hexyloxy)phenylester,
2,9% trans-4-[5-(4-Propylphenyl)-2-pyrimidinyl]cyclohexancarbonitril,
3,1% trans-4-[5-(4-Pentylphenyl)-2-pyrimidinyl]cyclohexancarbonitril,
Smp. < −10°; Klp. 57°

Mischung 29

35,5% 4'-Pentyl-4-cyanobiphenyl,
4,4% p-(5-Pentyl-2-pyrimidinyl)benzonitril,
7,8% p-(5-Heptyl-2-pyrimidinyl)benzonitril,
9,3% trans-4-Propylcyclohexancarbonsäure-p-cyanophenylester,
7,7% trans-4-Pentylcyclohexancarbonsäure-p-cyanophenylester,

20,8% trans-4-Butylcyclohexancarbonsäure-p-(hexyloxy)phenylester,
4,0% trans-p-[5-(4-Propylcyclohexyl)-2-pyrimidinyl]benzonitril,
10,5% trans-4-[5-(4-Heptylphenyl)-2-pyrimidinyl]cyclohexancarbonitril,
Smp. < −10°; Klp. 68°

Mischung 30

37,0% 4'-Pentyl-4-cyanobiphenyl,
10,0% trans-4-Propylcyclohexancarbonsäure-p-cyanophenylester,
8,0% trans-4-Pentylcyclohexancarbonsäure-p-cyanophenylester,
14,0% trans-4-Pentylcyclohexancarbonsäure-p-(methoxy)phenylester,
9,0% 2-(4-Cyanophenyl)-5-(4-butylphenyl)pyrimidin,
9,0% trans-p-[5-(4-Äthylcyclohexyl)-2-pyrimidinyl]benzonitril,
13,0% trans-p-[5-(4-Heptylcyclohexyl)-2-pyrimidinyl]benzonitril,
Smp. < −10°; Klp. 106°

Mischung 31

38,0% 4'-Pentyl-4-cyanobiphenyl,
10,0% trans-4-Propylcyclohexancarbonsäure-p-cyanophenylester,
9,0% trans-4-Pentylcyclohexancarbonsäure-p-cyanophenylester,
15,0% trans-4-Pentylcyclohexancarbonsäure-p-(methoxy)phenylester,
9,0% trans-p-[5-(4-Äthylcyclohexyl)-2-pyrimidinyl]benzonitril,
6,0% trans-p-[5-(4-Pentylcyclohexyl)-2-pyrimidinyl]benzonitril,
13,0% trans-p-[5-(4-Heptylcyclohexyl)-2-pyrimidinyl]benzonitril,
Smp. < −10°; Klp. 102°

Mischung 32

6,4% p-(5-Pentyl-2-pyrimidinyl)benzonitril,
11,9% p-(5-Heptyl-2-pyrimidinyl)benzonitril,
7,5% trans-4-Butylcyclohexancarbonsäure-p-cyanophenylester,
11,6% trans-4-Pentylcyclohexancarbonsäure-p-cyanophenylester,
19,3% trans-4-Pentylcyclohexancarbonsäure-p-(methoxy)phenylester,
26,2% trans-4-Pentylcyclohexancarbonsäure-p-(propyloxy)phenylester,
11,1% trans-p-[5-(4-Äthylcyclohexyl)-2-pyrimidinyl]benzonitril,
6,0% trans-p-[5-(4-Butylcyclohexyl)-2-pyrimidinyl]benzonitril,
Smp. < 10°; Klp. 83°

Mischung 33

5,0% p-(5-Pentyl-2-pyrimidinyl)benzonitril,
9,2% p-(5-Heptyl-2-pyrimidinyl)benzonitril,
39,1% 4'-Pentyl-4-cyanobiphenyl,
5,7% trans-4-Butylcyclohexancarbonsäure-p-cyanophenylester,
9,1% trans-4-Pentylcyclohexancarbonsäure-p-cyanophenylester,
22,2% trans-4-Pentylcyclohexancarbonsäure-p-(propyloxy)phenylester,
9,7% trans-p-[5-(4-Äthylcyclohexyl)-2-pyrimidinyl]benzonitril,
Smp. < 0°; Klp. 64°

Mischung 34

4,7% p-(5-Pentyl-2-pyrimidinyl)benzonitril,
8,5% p-(5-Heptyl-2-pyrimidinyl)benzonitril,
37,7% 4'-Pentyl-4-cyanobiphenyl,
5,3% trans-4-Butylcyclohexancarbonsäure-p-cyanophenylester,
8,4% trans-4-Pentylcyclohexancarbonsäure-p-cyanophenylester,
21,3% trans-4-Pentylcyclohexancarbonsäure-p-(propyloxy)phenylester,
9,3% trans-p-[5-(4-Äthylcyclohexyl)-2-pyrimidinyl]benzonitril,

4,8% trans-p-[5-(4-Butylcyclohexyl)-2-pyrimidinyl]benzonitril, Smp. <0°; Klp. 71°

Mischung 35

34,0% 4'-Pentyl-4-cyanobiphenyl,
5,0% trans-4-Butylcyclohexancarbonsäure-p-cyanophenylester,
8,0% trans-4-Pentylcyclohexancarbonsäure-p-cyanophenylester,
13,0% trans-4-Pentylcyclohexancarbonsäure-p-(methoxy)phenylester,
19,0% trans-4-Pentylcyclohexancarbonsäure-p-(propyloxy)phenylester,
9,0% trans-p-[5-(4-Äthylcyclohexyl)-2-pyrimidinyl]benzonitril,
4,0% trans-p-[5-(4-Butylcyclohexyl)-2-pyrimidinyl]benzonitril,
8,0% trans-p-[5-(4-Pentylcyclohexyl)-2-pyrimidinyl]benzonitril, Smp. < −10°; Klp. 87°

Mischung 36

40,0% 4'-Pentyl-4-cyanobiphenyl,
7,0% 4'-Octyloxy-4-cyanobiphenyl,
5,0% p-(5-Pentyl-2-pyrimidinyl)benzonitril,
10,0% p-(5-Heptyl-2-pyrimidinyl)benzonitril,
23,0% trans-4-Pentylcyclohexancarbonsäure-p-(propyloxy)phenylester,
10,0% trans-p-[5-(4-Äthylcyclohexyl)-2-pyrimidinyl]benzonitril,
5,0% trans-p-[5-(4-Butylcyclohexyl)-2-pyrimidinyl]benzonitril, Smp. <0°; Klp. 72°

Mischung 37

35,0% 4'-Pentyl-4-cyanobiphenyl,
19,0% 4'-Pentyloxy-4-cyanobiphenyl,
4,0% p-(5-Pentyl-2-pyrimidinyl)benzonitril,
8,0% p-(5-Heptyl-2-pyrimidinyl)benzonitril,
20,0% trans-4-Pentylcyclohexancarbonsäure-p-(propyloxy)phenylester,
9,0% trans-p-[5-(4-Äthylcyclohexyl-2-pyrimidinyl]benzonitril,
5,0% trans-p-[5-(4-Butylcyclohexyl)-2-pyrimidinyl]benzonitril, Smp. <10°; Klp. 73°

Mischung 38

7,0% p-Butylbenzoesäure-p'-cyanophenylester,
8,0% p-(5-Pentyl-2-pyrimidinyl)benzonitril,
15,0% p-(5-Heptyl-2-pyrimidinyl)benzonitril,
10,0% trans-4-Butylcyclohexancarbonsäure-p-cyanophenylester,
14,0% trans-4-Pentylcyclohexancarbonsäure-p-cyanophenylester,
25,0% trans-4-Butylcyclohexancarbonsäure-p-(äthoxy)phenylester,
13,0% trans-p-[5-(4-Äthylcyclohexyl)-2-pyrimidinyl]benzonitril,
8,0% trans-p-[5-(4-Pentylcyclohexyl)-2-pyrimidinyl]benzonitril, Smp. <10°; Klp. 88°

Mischung 39

5,0% p-Butylbenzoesäure-p'-cyanophenylester,
6,0% p-(5-Pentyl-2-pyrimidinyl)benzonitril,
10,0% p-(5-Heptyl-2-pyrimidinyl)benzonitril,
7,0% trans-4-Butylcyclohexancarbonsäure-p-cyanophenylester,
11,0% trans-4-Pentylcyclohexancarbonsäure-p-cyanophenylester,
19,0% trans-4-Butylcyclohexancarbonsäure-p-(äthoxy)phenylester,
20,0% 4'-Heptyl-4-cyanobiphenyl,
10,0% trans-p-[5-(4-Äthylcyclohexyl)-2-pyrimidinyl]benzonitril,
5,0% trans-p-[5-(4-Butylcyclohexyl)-2-pyrimidinyl]benzonitril,

7,0% trans-p-[5-(4-Pentylcyclohexyl)-2-pyrimidinyl]benzonitril,
Smp. <0°; Klp. 89°

Mischung 40

19,0% 4'-Heptyl-4-cyanobiphenyl,
5,0% p-Butylbenzoesäure-p'-cyanophenylester,

6,0% p-(5-Pentyl-2-pyrimidinyl)benzonitril,
10,0% p-(5-Heptyl-2-pyrimidinyl)benzonitril,
7,0% trans-4-Butylcyclohexancarbonsäure-p-cyanophenylester,
10,0% trans-4-Pentylcyclohexancarbonsäure-p-cyanophenylester,
19,0% trans-4-Butylcyclohexancarbonsäure-p-(äthoxy)phenylester,
10,0% trans-p-[5-(4-Äthylcyclohexyl)-2-pyrimidinyl]benzonitril,
14,0% trans-p-[5-(4-Heptylcyclohexyl)-2-pyrimidinyl]benzonitril,
Smp. <0°; Klp. 93°

Mischung 41

4,0% p-Butylbenzoesäure-p'-cyanophenylester,
5,0% p-(5-Pentyl-2-pyrimidinyl)benzonitril,
9,0% p-(5-Heptyl-2-pyrimidinyl)benzonitril,
29,0% trans-p-(4-Pentylcyclohexyl)benzonitril,
6,0% trans-4-Butylcyclohexancarbonsäure-p-cyanophenylester,
9,0% trans-4-Pentylcyclohexancarbonsäure-p-cyanophenylester,
16,0% trans-4-Butylcyclohexancarbonsäure-p-(äthoxy)phenylester,
9,0% trans-p-[5-(4-Äthylcyclohexyl)-2-pyrimidinyl]benzonitril,
13,0% trans-p-[5-(4-Heptylcyclohexyl)-2-pyrimidinyl]benzonitril,
Smp. < −10°; Klp. 93°

Mischung 42

16,0% 4'-Heptyl-4-cyanobiphenyl,
4,0% p-Butylbenzoesäure-p'-cyanophenylester,
5,0% p-(5-Pentyl-2-pyrimidinyl)benzonitril,
9,0% p-(5-Heptyl-2-pyrimidinyl)benzonitril,
5,0% trans-4-Butylcyclohexancarbonsäure-p-cyanophenylester,
9,0% trans-4-Pentylcyclohexancarbonsäure-p-cyanophenylester,
16,0% trans-4-Butylcyclohexancarbonsäure-p-(äthoxy)phenylester,
15,0% trans-4-Pentylcyclohexancarbonsäure-p-(methoxy)phenylester,
9,0% trans-p-[5-(4-Äthylcyclohexyl)-2-pyrimidinyl]benzonitril,
12,0% trans-p-[5-(4-Heptylcyclohexyl)-2-pyrimidinyl]benzonitril,
Smp. < −10°; Klp. 89°

Mischung 43

8,0% p-Butylbenzoesäure-p'-cyanophenylester,
9,0% p-(5-Pentyl-2-pyrimidinyl)benzonitril,
16,0% p-(5-Heptyl-2-pyrimidinyl)benzonitril,
10,0% trans-4-Butylcyclohexancarbonsäure-p-cyanophenylester,
16,0% trans-4-Pentylcyclohexancarbonsäure-p-cyanophenylester,
27,0% trans-4-Butylcyclohexancarbonsäure-p-(äthoxy)phenylester,
14,0% trans-p-[5-(4-Äthylcyclohexyl)-2-pyrimidinyl]benzonitril,
Smp. <10°; Klp. 77°

Mischung 44

7,0% p-Butylbenzoesäure-p'-cyanophenylester,
8,0% p-(5-Pentyl-2-pyrimidinyl)benzonitril,
15,0% p-(5-Heptyl-2-pyrimidinyl)benzonitril,
10,0% trans-4-Butylcyclohexancarbonsäure-p-cyanophenylester,

14,0% trans-4-Pentylcyclohexancarbonsäure-p-cyanophenylester,
26,0% trans-4-Butylcyclohexancarbonsäure-p-(äthoxy)phenylester,
8,0% trans-p-[5-(4-Äthylcyclohexyl)-2-pyrimidinyl]benzonitril,
12,0% trans-p-[5-(4-Pentylcyclohexyl)-2-pyrimidinyl]benzonitril,
Smp. <0°; Klp. 91°

Mischung 45

6,0% p-Butylbenzoesäure-p'-cyanophenylester,
7,0% p-(5-Pentyl-2-pyrimidinyl)benzonitril,
13,0% p-(5-Heptyl-2-pyrimidinyl)benzonitril,
8,0% trans-4-Butylcyclohexancarbonsäure-p-cyanophenylester,
12,0% trans-4-Pentylcyclohexancarbonsäure-p-cyanophenylester,
22,0% trans-4-Butylcyclohexancarbonsäure-p-(äthoxy)phenylester,
20,0% trans-4-Pentylcyclohexancarbonsäure-p-(methoxy)phenylester,
12,0% trans-p-[5-(4-Äthylcyclohexyl)-2-pyrimidinyl]benzonitril,
Smp. <10°; Klp. 74°

Mischung 46

5,0% p-Butylbenzoesäure-p'-cyanophenylester,
6,0% p-(5-Pentyl-2-pyrimidinyl)benzonitril,
10,0% p-(5-Heptyl-2-pyrimidinyl)benzonitril,
12,0% trans-4-Propylcyclohexancarbonsäure-p-cyanophenylester,
10,0% trans-4-Pentylcyclohexancarbonsäure-p-cyanophenylester,
19,0% trans-4-Butylcyclohexancarbonsäure-p-(äthoxy)phenylester,
17,0% trans-4-Pentylcyclohexancarbonsäure-p-(methoxy)phenylester,
7,0% trans-p-[5-(4-Pentylcyclohexyl)-2-pyrimidinyl]benzonitril,
14,0% trans-p-[5-(4-Heptylcyclohexyl)-2-pyrimidinyl]benzonitril,
Smp. <0°; Klp. 97°

Mischung 47

6,0% p-Butylbenzoesäure-p'-cyanophenylester,
6,0% p-(5-Pentyl-2-pyrimidinyl)benzonitril,
12,0% p-(5-Heptyl-2-pyrimidinyl)benzonitril,
13,0% trans-4-Propylcyclohexancarbonsäure-p-cyanophenylester,
12,0% trans-4-Pentylcyclohexancarbonsäure-p-cyanophenylester,
21,0% trans-4-Butylcyclohexancarbonsäure-p-(äthoxy)phenylester,
19,0% trans-4-Pentylcyclohexancarbonsäure-p-(methoxy)phenylester,
11,0% trans-p-[5-(4-Äthylcyclohexyl)-2-pyrimidinyl]benzonitril,
Smp. <0°; Klp. 74°

Mischung 48

6,0% p-Butylbenzoesäure-p'-cyanophenylester,
6,0% p-(5-Pentyl-2-pyrimidinyl)benzonitril,
11,0% p-(5-Heptyl-2-pyrimidinyl)benzonitril,
13,0% trans-4-Propylcyclohexancarbonsäure-p-cyanophenylester,
11,0% trans-4-Pentylcyclohexancarbonsäure-p-cyanophenylester,
20,0% trans-4-Butylcyclohexancarbonsäure-p-(äthoxy)phenylester,
18,0% trans-4-Pentylcyclohexancarbonsäure-p-(methoxy)phenylester,
7,0% 4''-Pentyl-4-cyano-p-terphenyl,
8,0% trans-p-[5-(4-Pentylcyclohexyl)-2-pyrimidinyl]benzonitril,
Smp. <0°; Klp. 85°

Mischung 49

17,5% 4'-Heptyl-4-cyanobiphenyl,
5,0% p-(5-Pentyl-2-pyrimidinyl)benzonitril,

| | |
|---|---|
| 9,0% | p-(5-Heptyl-2-pyrimidinyl)benzonitril, |
| 11,0% | trans-4-Propylcyclohexancarbonsäure-p-cyanophenylester, |
| 6,0% | trans-4-Butylcyclohexancarbonsäure-p-cyanophenylester, |
| 9,0% | trans-4-Pentylcyclohexancarbonsäure-p-cyanophenylester, |
| 15,5% | trans-4-Pentylcyclohexancarbonsäure-p-(methoxy)phenylester, |
| 17,0% | trans-4-Butylcyclohexancarbonsäure-p-(äthoxy)phenylester, |
| 10,0% | trans-p-[5-(4-Äthylcyclohexyl)-2-pyrimidinyl]benzonitril, |
| | Smp. <0°; Klp. 69° |

Mischung 50

| | |
|---|---|
| 23,0% | 4'-Heptyl-4-cyanobiphenyl, |
| 7,0% | p-(5-Pentyl-2-pyrimidinyl)benzonitril, |
| 12,0% | p-(5-Heptyl-2-pyrimidinyl)benzonitril, |
| 14,0% | trans-4-Propylcyclohexancarbonsäure-p-cyanophenylester, |
| 12,0% | trans-4-Pentylcyclohexancarbonsäure-p-cyanophenylester, |
| 20,0% | trans-4-Pentylcyclohexancarbonsäure-p-(methoxy)phenylester, |
| 12,0% | trans-p-[5-(4-Äthylcyclohexyl)-2-pyrimidinyl]benzonitril, |
| | Smp. <0°; Klp. 70° |

Mischung 51

| | |
|---|---|
| 6,0% | p-Butylbenzoesäure-p'-cyanophenylester, |
| 7,0% | p-(5-Pentyl-2-pyrimidinyl)benzonitril, |
| 12,0% | p-(5-Heptyl-2-pyrimidinyl)benzonitril, |
| 14,0% | trans-4-Propylcyclohexancarbonsäure-p-cyanophenylester, |
| 8,0% | trans-4-Butylcyclohexancarbonsäure-p-cyanophenylester, |
| 12,0% | trans-4-Pentylcyclohexancarbonsäure-p-cyanophenylester, |
| 22,0% | trans-4-Butylcyclohexancarbonsäure-p-(äthoxy)phenylester, |
| 8,0% | 4''-Pentyl-4-cyano-p-terphenyl, |
| 11,0% | trans-p-[5-(4-Äthylcyclohexyl)-2-pyrimidinyl]benzonitril, |
| | Smp. <0°; Klp. 89° |

Mischung 52

| | |
|---|---|
| 18,0% | 4'-Heptyl-4-cyanobiphenyl, |
| 5,0% | p-Butylbenzoesäure-p'-cyanophenylester, |
| 5,0% | p-(5-Pentyl-2-pyrimidinyl)benzonitril, |
| 9,5% | p-(5-Heptyl-2-pyrimidinyl)benzonitril, |
| 11,0% | trans-4-Propylcyclohexancarbonsäure-p-cyanophenylester, |
| 9,5% | trans-4-Pentylcyclohexancarbonsäure-p-cyanophenylester, |
| 18,0% | trans-4-Butylcyclohexancarbonsäure-p-(äthoxy)phenylester, |
| 10,0% | trans-p-[5-(4-Äthylcyclohexyl)-2-pyrimidinyl]benzonitril, |
| 14,0% | trans-p-[5-(4-Heptylcyclohexyl)-2-pyrimidinyl]benzonitril, |
| | Smp. <0°; Klp. 93° |

Mischung 53

| | |
|---|---|
| 5,0% | p-Butylbenzoesäure-p'-cyanophenylester, |
| 5,0% | p-(5-Pentyl-2-pyrimidinyl)benzonitril, |
| 10,0% | p-(5-Heptyl-2-pyrimidinyl)benzonitril, |
| 11,0% | trans-4-Propylcyclohexancarbonsäure-p-cyanophenylester, |
| 10,0% | trans-4-Pentylcyclohexancarbonsäure-p-cyanophenylester, |
| 18,0% | trans-4-Butylcyclohexancarbonsäure-p-(äthoxy)phenylester, |
| 17,0% | trans-4-Pentylcyclohexancarbonsäure-p-(methoxy)phenylester, |
| 10,0% | trans-p-[5-(4-Äthylcyclohexyl)-2-pyrimidinyl]benzonitril, |
| 14,0% | trans-p-[5-(4-Heptylcyclohexyl)-2-pyrimidinyl]benzonitril, |
| | Smp. <0°; Klp. 98° |

Mischung 54

| | |
|---|---|
| 15,0% | 4'-Heptyl-4-cyanobiphenyl, |
| 4,0% | p-Butylbenzoesäure-p'-cyanophenylester, |
| 4,5% | p-(5-Pentyl-2-pyrimidinyl)benzonitril, |
| 8,0% | p-(5-Heptyl-2-pyrimidinyl)benzonitril, |
| 9,5% | trans-4-Propylcyclohexancarbonsäure-p-cyanophenylester, |
| 8,0% | trans-4-Pentylcyclohexancarbonsäure-p-cyanophenylester, |
| 15,0% | trans-4-Butylcyclohexancarbonsäure-p-(äthoxy)phenylester, |
| 14,0% | trans-4-Pentylcyclohexancarbonsäure-p-(methoxy)phenylester, |
| 9,0% | trans-p-[5-(4-Äthylcyclohexyl)-2-pyrimidinyl]benzonitril, |
| 13,0% | trans-p-[5-(4-Heptylcyclohexyl)-2-pyrimidinyl]benzonitril, |
| | Smp. <0°; Klp. 92° |

Mischung 55

| | |
|---|---|
| 33,0% | 4'-Pentyl-4-cyanobiphenyl, |
| 8,5% | trans-4-Propylcyclohexancarbonsäure-p-cyanophenylester, |
| 7,0% | trans-4-Pentylcyclohexancarbonsäure-p-cyanophenylester, |
| 14,0% | trans-4-Butylcyclohexancarbonsäure-p-(äthoxy)phenylester, |
| 12,5% | trans-4-Pentylcyclohexancarbonsäure-p-(methoxy)phenylester, |
| 8,0% | trans-p-[5-(4-Äthylcyclohexyl)-2-pyrimidinyl]benzonitril, |
| 5,0% | trans-p-[5-(4-Pentylcyclohexyl)-2-pyrimidinyl]benzonitril, |
| 12,0% | trans-p-[5-(4-Heptylcyclohexyl)-2-pyrimidinyl]benzonitril, |
| | Smp. <0°; Klp. 96° |

Mischung 56

| | |
|---|---|
| 45,0% | 4'-Pentyl-4-cyanobiphenyl, |
| 6,0% | p-Butylbenzoesäure-p'-cyanophenylester, |
| 6,0% | p-(5-Pentyl-2-pyrimidinyl)benzonitril, |
| 13,0% | trans-4-Propylcyclohexancarbonsäure-p-cyanophenylester, |
| 7,5% | trans-4-Butylcyclohexancarbonsäure-p-cyanophenylester, |
| 11,5% | trans-4-Pentylcyclohexancarbonsäure-p-cyanophenylester, |
| 11,0% | trans-p-[5-(4-Äthylcyclohexyl)-2-pyrimidinyl]benzonitril, |
| | Smp. <0°; Klp. 65° |

Mischung 57

| | |
|---|---|
| 41,0% | 4'-Pentyl-4-cyanobiphenyl, |
| 5,0% | p-Butylbenzoesäure-p'-cyanophenylester, |
| 5,0% | p-(5-Pentyl-2-pyrimidinyl)benzonitril, |
| 12,0% | trans-4-Propylcyclohexancarbonsäure-p-cyanophenylester, |
| 10,0% | trans-4-Pentylcyclohexancarbonsäure-p-cyanophenylester, |
| 16,0% | trans-4-Pentylcyclohexancarbonsäure-p-(methoxy)phenylester, |
| 11,0% | trans-p-[5-(4-Äthylcyclohexyl)-2-pyrimidinyl]benzonitril, |
| | Smp. <0°; Klp. 65° |

Mischung 58

| | |
|---|---|
| 29,0% | 4'-Heptyl-4-cyanobiphenyl, |
| 8,0% | p-Butylbenzoesäure-p'-cyanophenylester, |
| 8,0% | p-(5-Pentyl-2-pyrimidinyl)benzonitril, |
| 17,0% | trans-4-Propylcyclohexancarbonsäure-p-cyanophenylester, |
| 10,0% | trans-4-Butylcyclohexancarbonsäure-p-cyanophenylester, |
| 15,0% | trans-4-Pentylcyclohexancarbonsäure-p-cyanophenylester, |
| 13,0% | trans-p-[5-(4-Äthylcyclohexyl)-2-pyrimidinyl]benzonitril, |
| | Smp. <0°; Klp. 73° |

Mischung 59

21,0% 4'-Heptyl-4-cyanobiphenyl,
5,0% p-Butylbenzoesäure-p'-cyanophenylester,
6,0% p-(5-Pentyl-2-pyrimidinyl)benzonitril,
11,0% p-(5-Heptyl-2-pyrimidinyl)benzonitril,
13,0% trans-4-Propylcyclohexancarbonsäure-p-cyanophenylester,
11,0% trans-4-Pentylcyclohexancarbonsäure-p-cyanophenylester,
21,0% trans-4-Butylcyclohexancarbonsäure-p-(äthoxy)phenylester,
12,0% trans-p-[5-(4-Äthylcyclohexyl)-2-pyrimidinyl]benzonitril,
Smp. <0°; Klp. 71°

Mischung 60

37,0% 4'-Pentyl-4-cyanobiphenyl,
5,0% p-(5-Pentyl-2-pyrimidinyl)benzonitril,
8,0% p-(5-Heptyl-2-pyrimidinyl)benzonitril,
28,0% trans-p-(4-Pentylcyclohexyl)benzonitril,
9,0% trans-p-[5-(4-Äthylcyclohexyl)-2-pyrimidinyl]benzonitril,
13,0% trans-p-[5-(4-Heptylcyclohexyl)-2-pyrimidinyl]benzonitril,
Smp. <0°; Klp. 84°

Mischung 61

3,5% p-Butylbenzoesäure-p'-cyanophenylester,
4,0% p-(5-Pentyl-2-pyrimidinyl)benzonitril,
7,0% p-(5-Heptyl-2-pyrimidinyl)benzonitril,
25,0% trans-p-(4-Pentylcyclohexyl)benzonitril,
8,5% trans-4-Propylcyclohexancarbonsäure-p-cyanophenylester,
7,0% trans-4-Pentylcyclohexancarbonsäure-p-cyanophenylester,
13,0% trans-4-Butylcyclohexancarbonsäure-p-(äthoxy)phenylester,
12,0% trans-4-Pentylcyclohexancarbonsäure-p-(methoxy)phenylester,
8,0% trans-p-[5-(4-Äthylcyclohexyl)-2-pyrimidinyl]benzonitril,
12,0% trans-p-[5-(4-Heptylcyclohexyl)-2-pyrimidinyl]benzonitril,
Smp. <0°; Klp. 89°

Mischung 62

37,0% 4'-Pentyl-4-cyanobiphenyl,
21,0% 4'-Pentyloxy-4-cyanobiphenyl,
4,0% p-Butylbenzoesäure-p'-cyanophenylester,
5,0% p-(5-Pentyl-2-pyrimidinyl)benzonitril,
8,0% p-(5-Heptyl-2-pyrimidinyl)benzonitril,
15,0% trans-4-Butylcyclohexancarbonsäure-p-(äthoxy)phenylester,
10,0% trans-p-[5-(4-Äthylcyclohexyl)-2-pyrimidinyl]benzonitril,
Smp. <0°; Klp. 65°

Mischung 63

43,0% 4'-Pentyl-4-cyanobiphenyl,
24,0% 4'-Pentyloxy-4-cyanobiphenyl,
6,0% p-(5-Pentyl-2-pyrimidinyl)benzonitril,
11,0% p-(5-Heptyl-2-pyrimidinyl)benzonitril,
10,0% trans-p-[5-(4-Äthylcyclohexyl)-2-pyrimidinyl]benzonitril,
6,0% trans-p-[5-(4-Butylcyclohexyl)-2-pyrimidinyl]benzonitril,
Smp. <0°; Klp. 70°

Mischung 64

44,0% 4'-Pentyl-4-cyanobiphenyl,
21,0% 4'-Heptyl-4-cyanobiphenyl,
6,0% p-(5-Pentyl-2-pyrimidinyl)benzonitril,
12,0% p-(5-Heptyl-2-pyrimidinyl)benzonitril,
11,0% trans-p-[5-(4-Äthylcyclohexyl)-2-pyrimidinyl]benzonitril,
6,0% trans-p-[5-(4-Butylcyclohexyl)-2-pyrimidinyl]benzonitril,
Smp. <0°; Klp. 70°

Mischung 65

41,0% 4'-Pentyl-4-cyanobiphenyl,
19,0% 4'-Heptyl-4-cyanobiphenyl,
5,0% p-(5-Pentyl-2-pyrimidinyl)benzonitril,
10,0% p-(5-Heptyl-2-pyrimidinyl)benzonitril,
18,0% trans-4-Butylcyclohexancarbonsäure-p-(äthoxy)phenylester,
7,0% trans-p-[5-(4-Pentylcyclohexyl)-2-pyrimidinyl]benzonitril,
Smp. <0°; Klp. 58°

Mischung 66

47,0% 4'-Pentyl-4-cyanobiphenyl,
23,0% 4'-Heptyl-4-cyanobiphenyl,
7,0% p-(5-Pentyl-2-pyrimidinyl)benzonitril,
11,0% p-(5-Heptyl-2-pyrimidinyl)benzonitril,
12,0% trans-p-[5-(4-Äthylcyclohexyl)-2-pyrimidinyl]benzonitril,
Smp. <0°; Klp. 60°

Mischung 67

36,0% 4'-Pentyl-4-cyanobiphenyl,
15,0% 4'-Heptyl-4-cyanobiphenyl,
5,0% p-(5-Pentyl-2-pyrimidinyl)benzonitril,
8,0% p-(5-Heptyl-2-pyrimidinyl)benzonitril,
15,0% trans-4-Butylcyclohexancarbonsäure-p-(äthoxy)phenylester,
14,0% trans-4-Pentylcyclohexancarbonsäure-p-(methoxy)phenylester,
7,0% trans-p-[5-(4-Pentylcyclohexyl)-2-pyrimidinyl]benzonitril,
Smp. <0°; Klp. 59°

Mischung 68

55,0% 4'-Pentyl-4-cyanobiphenyl,
11,0% 4'-Octyloxy-4-cyanobiphenyl,
9,0% p-(5-Pentyl-2-pyrimidinyl)benzonitril,
16,0% p-(5-Heptyl-2-pyrimidinyl)benzonitril,
9,0% trans-p-[5-(4-Pentylcyclohexyl)-2-pyrimidinyl]benzonitril,
Smp. <0°; Klp. 65°

Mischung 69

40,0% 4'-Pentyl-4-cyanobiphenyl,
23,0% 4'-Pentyloxy-4-cyanobiphenyl,
5,0% p-(5-Pentyl-2-pyrimidinyl)benzonitril,
10,0% p-(5-Heptyl-2-pyrimidinyl)benzonitril,
16,0% trans-4-Pentylcyclohexancarbonsäure-p-(methoxy)phenylester,
6,0% trans-p-[5-(4-Pentylcyclohexyl)-2-pyrimidinyl]benzonitril,
Smp. <0°; Klp. 62°

Mischung 70

7,0% 4'-Pentyl-4-cyanobiphenyl,
5,6% p-Butylbenzoesäure-p'-cyanophenylester,
6,5% p-(5-Pentyl-2-pyrimidinyl)benzonitril,
12,1% p-(5-Heptyl-2-pyrimidinyl)benzonitril,
7,4% trans-4-Butylcyclohexancarbonsäure-p-cyanophenylester,
11,2% trans-4-Pentylcyclohexancarbonsäure-p-cyanophenylester,
20,4% trans-4-Butylcyclohexancarbonsäure-p-(äthoxy)phenylester,
18,6% trans-4-Pentylcyclohexancarbonsäure-p-(methoxy)phenylester,
11,2% trans-p-[5-(4-Äthylcyclohexyl)-2-pyrimidinyl]benzonitril,
Smp. $<0°$; Klp. 71°

Mischung 71

31,0% 4'-Pentyl-4-cyanobiphenyl,
30,0% p-[(p-Hexylbenzyliden)amino]benzonitril,
12,0% trans-4-Butylcyclohexancarbonsäure-p-(äthoxy)phenylester,
19,0% trans-4-Pentylcyclohexancarbonsäure-p-(propyloxy)phenylester,
8,0% trans-p-[5-(4-Äthylcyclohexyl)-2-pyrimidinyl]benzonitril,
Smp. $<0°$; Klp. 66°

Mischung 72

5,0% p-Butylbenzoesäure-p'-cyanophenylester,
4,0% p-Pentylbenzoesäure-p'-cyanophenylester,
26,0% p-[(p-Butylbenzyliden)amino]benzonitril,
5,0% p-(5-Pentyl-2-pyrimidinyl)benzonitril,
10,0% p-(5-Heptyl-2-pyrimidinyl)benzonitril,
11,0% trans-4-Propylcyclohexancarbonsäure-p-cyanophenylester,
10,0% trans-4-Pentylcyclohexancarbonsäure-p-cyanophenylester,
19,0% trans-4-Butylcyclohexancarbonsäure-p-(äthoxy)phenylester,
10,0% trans-p-[5-(4-Äthylcyclohexyl)-2-pyrimidinyl]benzonitril,
Smp. $<0°$; Klp. 73°

Mischung 73

6,0% 4'-Octyloxy-4-cyanobiphenyl,
7,0% p-Butylbenzoesäure-p'-cyanophenylester,
6,0% p-(5-Pentyl-2-pyrimidinyl)benzonitril,
12,0% p-(5-Heptyl-2-pyrimidinyl)benzonitril,
12,0% trans-4-Propylcyclohexancarbonsäure-p-cyanophenylester,
11,0% trans-4-Pentylcyclohexancarbonsäure-p-cyanophenylester,
19,0% trans-4-Butylcyclohexancarbonsäure-p-(äthoxy)phenylester,
18,0% trans-4-Pentylcyclohexancarbonsäure-p-(methoxy)phenylester,
9,0% trans-p-[5-(4-Äthylcyclohexyl)-2-pyrimidinyl]benzonitril,
Smp. $<0°$; Klp. 71°

Mischung 74

23,0% 4'-Heptyl-4-cyanobiphenyl,
21,0% trans-4-Butylcyclohexancarbonsäure-p-äthoxyphenylester,
19,0% trans-4-Pentylcyclohexancarbonsäure-p-(methoxy)phenylester,
26,0% trans-4-Pentylcyclohexancarbonsäure-p-(propyloxy)phenylester,
11,0% trans-p-[5-(4-Äthylcyclohexyl)-2-pyrimidinyl]benzonitril,
Smp. $<0°$; Klp. 73°

Mischung 75

75,0 Gew.-% Mischung 74
25,0 Gew.-% Mischung 47
Smp. <0°; Klp. 71,9°

Mischung 76

60,0 Gew.-% Mischung 74
40,0 Gew.-% Mischung 47
Smp. <0°; Klp. 71,7°

Mischung 77

45,0 Gew.-% Mischung 74
55,0 Gew.-% Mischung 47
Smp. <0°; Klp. 71,8°

Mischung 78

15,0 Gew.-% Mischung 74
85,0 Gew.-% Mischung 47
Smp. <0°; Klp. 72,4°

Mischung 79

75,0 Gew.-% Mischung 74
25,0 Gew.-% Mischung 70
Smp. <0°; Klp. 71,5°

Mischung 80

15,0 Gew.-% Mischung 74
85,0 Gew.-% Mischung 70
Smp. <0°; Klp. 70,6°

Mischung 81

22,0% 4'-Heptyl-4-cyanobiphenyl,
13,0% trans-4-Propylcyclohexancarbonsäure-p-cyanophenylester,
21,0% trans-4-Butylcyclohexancarbonsäure-p-(äthoxy)phenylester,
19,0% trans-4-Pentylcyclohexancarbonsäure-p-(methoxy)phenylester,
18,0% trans-4-Pentylcyclohexancarbonsäure-p-(propyloxy)phenylester,
7,0% trans-p-[5-(4-Pentylcyclohexyl)-2-pyrimidinyl]benzonitril,
Smp. <0°; Klp. 71,6°

Mischung 82

Mischung 55 + Farbstoff der Formel IXa, worin $R^8$ n-Heptyl bedeutet; Smp. <0°; Klp. 97°; blau, $\lambda_{max}$ = 594 nm; S = 0,745

Mischung 83

Mischung 46 + Farbstoff der Formel IXa, worin $R^8$ n-Heptyl bedeutet; Smp. <0°; Klp. 96°; blau, $\lambda_{max}$ = 594 nm; S = 0,707

Mischung 84

Mischung 47 + Farbstoff der Formel IXa, worin $R^8$ n-Heptyl bedeutet; Smp. <0°; Klp. 73°; blau, $\lambda_{max}$=594 nm; S=0,717

Mischung 85

Mischung 53 + Farbstoff der Formel IXa, worin $R^8$ n-Butyl bedeutet; Smp. <0°; Klp. 98°; blau, $\lambda_{max}$=594 nm; S=0,709

Mischung 86

Mischung 53 + Farbstoff der Formel IXa, worin $R^8$ Methyl bedeutet; Smp. <0°; Klp. 98°; blau, $\lambda_{max}$=594 nm; S=0,694

Mischung 87

Mischung 31 + Farbstoff der Formel IXa, worin $R^8$ n-Heptyl bedeutet; Smp. <0°; Klp. 102°; blau, $\lambda_{max}$=594 nm; S=0,768

Mischung 88

Mischung 46 + Farbstoff der Formel VIIa, worin $R^7$ n-Butyloxy und $Z^3$ Dimethylamino bedeutet und $n_4$=0 ist; Smp. <0°; Klp. 97°; rot, $\lambda_{max}$=509 nm; S=0,683

Mischung 89

Mischung 55 + Farbstoff der Formel VIIa, worin $X^7$ Äthoxy und $Z^3$ Dimethylamino bedeutet und $n_4$=0 ist; Smp. <0°; Klp. 97°; rot, $\lambda_{max}$=514 nm

Mischung 90

Mischung 55 + Farbstoff der Formel XI, worin $R^4$ Äthyl bedeutet; Smp. <0°; Klp. 97°; gelb, $\lambda_{max}$=446 nm; S=0,889

Mischung 91

Mischung 55 + Farbstoff der Formel IVa; Smp. <0°; Klp. 97°; gelb-rot, $\lambda_{max}$=500 nm; S=0,817

Mischung 92

Mischung 55 + Farbstoff der Formel VIIa, worin $X^7$ Nitro und $Z^3$ Diäthylamino bedeutet und $n_4$=1 ist; Smp. <0°; Klp. 97°; stahlblau, $\lambda_{max}$=605 nm; S=0,816

Mischung 93

Mischung 55 + Farbstoff der Formel XII, worin $R^5$ Äthyl bedeutet; Smp. <0°; Klp. 97°; blau, $\lambda_{max}$=636 nm; S= 0,716

Mischung 94

35,0% 4'-Pentyl-4-cyanobiphenyl,
4,0% p-Butylbenzoesäure-p'-cyanophenylester,
4,0% p-Pentylbenzoesäure-p'-cyanophenylester,
4,0% p-(5-Pentyl-2-pyrimidinyl)benzonitril,

8,0% p-(5-Heptyl-2-pyrimidinyl)benzonitril,
9,0% trans-4-Propylcyclohexancarbonsäure-p-cyanophenylester,
8,0% trans-4-Pentylcyclohexancarbonsäure-p-cyanophenylester,
19,0% trans-4-Pentylcyclohexancarbonsäure-p-(propyloxy)phenylester,
9,0% trans-p-[5-(4-Äthylcyclohexyl)-2-pyrimidinyl]benzonitril,
Smp. <0°; Klp. 63°

Mischung 95

33,0% 4'-Pentyl-4-cyanobiphenyl,
30,0% 4'-Hexyl-4-cyanobiphenyl,
5,0% 4'-Octyloxy-4-cyanobiphenyl,
4,0% p-(5-Pentyl-2-pyrimidinyl)benzonitril,
8,0% p-(5-Heptyl-2-pyrimidinyl)benzonitril,
3,0% p-Butylbenzoesäure-p'-cyanophenylester,
7,0% trans-4-Pentylcyclohexancarbonsäure-p-cyanophenylester,
10,0% trans-p-[5-(4-Äthylcyclohexyl)-2-pyrimidinyl]benzonitril,
Smp. <0°; Klp. 58°

Mischung 96

25,5% 4'-Heptyl-4-cyanobiphenyl,
13,0% p-[(p-Propylbenzyliden)amino]benzonitril,
7,0% p-Butylbenzoesäure-p'-cyanophenylester,
6,0% p-Pentylbenzoesäure-p'-cyanophenylester,
7,5% p-(5-Pentyl-2-pyrimidinyl)benzonitril,
14,0% p-(5-Heptyl-2-pyrimidinyl)benzonitril,
15,0% trans-4-Propylcyclohexancarbonsäure-p-cyanophenylester,
12,0% trans-p-[5-(4-Äthylcyclohexyl)-2-pyrimidinyl]benzonitril,
Smp. <0°; Klp. 70°.

Für farbstoffhaltige Mischungen kommen auch weitere Flüssigkristallmatrizen in Frage, sofern die Klärpunkte höher als etwa 80°C sind. Bevorzugte Flüssigkristallmatrizen für farbstoffhaltige Mischungen sind die Mischungen 2, 15, 24, 25, 27, 31, 39–42, 46–48, 52–55 und 61.

Die Herstellung der erfindungsgemäßen Verbindungen der Formel I wird anhand der folgenden Beispiele veranschaulicht. Alle Temperaturen sind in Grad Celsius angegeben.

Beispiel 1

Zu einer Suspension von 17,2 g rohem trans-p-[5-(4-n-Pentylcyclohexyl)-2-pyrimidinyl]benzamid in 150 ml Pyridin werden unter Rühren 9,5 ml Benzolsulfochlorid getropft. Im Ölbad wird dann 6 Stunden auf 55° erwärmt. Man gießt das Gemisch auf 500 ml eiskalte 0,5 N-Salzsäure und nimmt das Produkt in Methylenchlorid auf. Der Extrakt wird dreimal mit je 200 ml 3 N-Salzsäure, dann mit 100 ml gesättigter Natriumbicarbonatlösung und mit Wasser gewaschen, über Natriumsulfat getrocknet und eingedampft. Das Rohprodukt wird an einer Säule von 600 g Kieselgel mit Toluol/5% Aceton (v/v) chromatographiert. Die nach Dünnschichtchromatographie reinen Fraktionen werden in Hexan gelöst, heiß mit Aktivkohle filtriert und umkristallisiert. Man erhält trans-p-[5-(4-n-Pentyl-cyclohexyl)-2-pyrimidinyl]benzonitril; Smp. 100,5°, Klp. 231°.

Das als Ausgangsmaterial verwendete trans-p-[5-(4-n-Pentyl-cyclohexyl)-2-pyrimidinyl]benzamid kann wie folgt hergestellt werden:

a) Eine Lösung von 63,9 g trans-4-n-Pentylcyclohexancarbonsäure in 250 ml trockenem Äther wird zu einer Suspension von 12,2 g Lithiumaluminiumhydrid in 1000 ml trockenem Äther getropft, so, daß das Gemisch leicht siedet. Nach beendetem Zutropfen wird noch 1 Stunde gerührt. Dann werden vorsichtig 80 ml Essigsäureäthylester und danach 100 ml Eiswasser zugetropft. Das Reaktionsgemisch wird auf Eiswasser gegossen und mit verdünnter Salzsäure kongosauer gemacht. Die organische Phase wird abgetrennt und der Reihe nach mit Wasser, Natriumhydrogencarbonatlösung und Wasser gewaschen, über Natriumsulfat getrocknet und eingedampft. Das erhaltene trans-4-n-Pentylcyclohexancarbinol wird roh weiter umgesetzt.

b) Zu einer Suspension von 125 g Pyridiniumchlorchromat in 900 ml Methylenchlorid gibt man eine Lösung von 59,0 g trans-4-n-Pentylcyclohexancarbinol in 100 ml Äther und rührt das Gemisch 2 Stunden bei Raumtemperatur. Dann verdünnt man mit 400 ml Äther, rührt noch 15 Minuten und dekantiert die Lösung vom teerartigen Niederschlag. Der Niederschlag wird mit Äther nachgewaschen. Die vereinigten organischen Lösungen werden an einer Florisil-Säule filtriert. Der nach Eindampfen des Filtrats erhaltene trans-4-n-Pentylcyclohexancarboxaldehyd wird direkt weiter verarbeitet.

c) Eine Suspension von 223,1 g (Methoxymethyl)triphenylphosphoniumchlorid in 1600 ml trockenem Äther wird mit 76,2 g Kalium-t-butylat versetzt. Nach 45minütigem Rühren wird eine Lösung von 51,4 g trans-4-n-Pentylcyclohexancarboxaldehyd in 300 ml Äther zugetropft. Das Reaktionsgemisch wird noch 2 Stunden gerührt und dann in 2,5 l Eiswasser gegossen. Die organische Phase wird abgetrennt, mit Wasser neutralgewaschen, über Natriumsulfat getrocknet und eingedampft. Der erhaltene rohe trans-2-(4-n-Pentylcyclohexyl)vinylmethyläther wird zur Abtrennung des restlichen Triphenylphosphins bei 92—96°/0,7 mm Hg destilliert.

d) Zu einer auf 0° gekühlten Lösung von 13,1 g Bortrifluoriddiäthylätherat in 700 ml frisch destilliertem Orthoameisensäuretriäthylester wird unter Rühren 38,7 g trans-2-(4-n-Pentylcyclohexyl)vinylmethyläther getropft und das Reaktionsgemisch über Nacht bei Raumtemperatur gerührt. Danach verdünnt man mit 1000 ml Toluol, wäscht mit 200 ml Natriumbicarbonatlösung, dann mit Wasser, trocknet über Natriumsulfat, filtriert und dampft ein. Das rohe trans-(4-n-Pentylcyclohexyl)malon-bis-acetal wird ohne weitere Reinigung eingesetzt.

e) 57,4 g rohes trans-(4-n-Pentylcyclohexyl)malon-bis-acetal werden mit 3,5 ml Wasser und 150 mg p-Toluolsulfonsäure versetzt. Das Gemisch wird 3 Stunden bei 80—85° gerührt. Dann läßt man erkalten, gibt 1,6 g Natriumhydrogencarbonat zu und rührt bei Raumtemperatur 1,5 Stunden weiter. Das Reaktionsgemisch wird mit Äther verdünnt, gewaschen mit 3×50 ml eiskalter 3 N-Natronlauge, mit Wasser neutral gewaschen, über Natriumsulfat getrocknet, filtriert und eingedampft. Das rohe 3-Äthoxy-2-(trans-4-n-pentylcyclohexyl)acrolein wird sofort weiter verarbeitet.

f) Durch vorsichtige Zugabe von 4,8 g Natrium in kleinen Portionen zu 175 ml absolutem Methanol wird eine Natriummethylat-Lösung bereitet. Man tropft unter Rühren 17,8 g 3-Äthoxy-2-(trans-4-n-pentylcyclohexyl)acrolein zu, rührt noch 10 Minuten und gibt dann 16,2 g p-Carbamoylbenzamidinhydrochlorid zu. Das Reaktionsgemisch wird über Nacht bei 50° gerührt. Nach dem Abkühlen wird mit 55 ml 3 N-Salzsäure angesäuert. Die Suspension wird genutscht und der Rückstand auf der Nutsche mit Wasser neutral gewaschen, trocken gesaugt und im Wasserstrahlvakuum bei 70° getrocknet. Das bräunliche, rohe trans-p-[5-(4-n-Pentylcyclohexyl)-2-pyrimidinyl]benzamid wird ohne Reinigung in der nächsten Stufe eingesetzt.

In analoger Weise können folgende Verbindungen hergestellt werden:

trans-p-[5-(4-Methylcyclohexyl)-2-pyrimidinyl]benzonitril; Smp. 150,5°, Klp. 213,5°
trans-p-[5-(4-Äthylcyclohexyl)-2-pyrimidinyl]benzonitril; Smp. 118°, Klp. 225°
trans-p-[5-(4-n-Propylcyclohexyl)-2-pyrimidinyl]benzonitril; Smp. 122,5°, Klp. 243,5°
trans-p-[5-(4-n-Butylcyclohexyl)-2-pyrimidinyl]benzonitril; Smp. 118°, Klp. 234°
trans-p-[5-(4-n-Hexylcyclohexyl)-2-pyrimidinyl]benzonitril; Smp. 91,5°, Klp. 224,5°
trans-p-[5-(4-n-Heptylcyclohexyl)-2-pyrimidinyl]benzonitril; Smp. 80,5°, Klp. 219°
(+)-trans-p-[5-[4-(2-Methylbutyl)cyclohexyl]-2-pyrimidinyl]benzonitril; $\alpha_D = +9$, Smp. 76°; smektisch-cholesterischer Phasenübergang 125°; Klp. 178°
(+)-trans-p-[5-[4-(3-Methylpentyl)cyclohexyl]-2-pyrimidinyl]benzonitril; $\alpha_D = +7$, Smp. 101°, smektisch-cholesterischer Phasenübergang 159°, Klp. 189,5°

## Beispiel 2

Zu einer Suspension von 10,8 g trans-4-[5-(p-n-Heptylphenyl)-2-pyrimidinyl]cyclohexancarboxamid in 150 ml Pyridin tropft man unter Rühren 6,0 ml Benzolsulfochlorid. Im Ölbad wird dann 2 Stunden auf 55° erwärmt. Man gießt das Reaktionsgemisch auf Eiswasser und nimmt das Produkt in Methylenchlorid auf. Der Extrakt wird nacheinander mit verdünnter Salzsäure, gesättigter Natriumhydrogencarbonatlösung und Wasser gewaschen, über Natriumsulfat getrocknet und eingedampft. Das Rohprodukt wird an einer Säule von 300 g Kieselgel mit Toluol/5% Aceton (v/v) chromatographiert. Die nach Dünnschichtchromatographie reinen Fraktionen werden gesammelt, eingedampft, in Hexan gelöst, heiß mit Aktivkohle filtriert und umkristallisiert. Das Kristallisat wird über Nacht im Vakuum getrocknet. Man erhält trans-4-[5-(p-n-Heptylphenyl)-2-pyrimidinyl]cyclohe-

xancarbonitril; Smp. 83,5°, Klp. 160°.

Das als Ausgangsmaterial verwendete trans-4-[5-(p-n-Heptylphenyl)-2-pyrimidinyl]cyclohexancarboxamid kann wie folgt hergestellt werden:

a) 96,6 g trans-4-Cyanocyclohexancarbonsäure-methylester werden unter Inertgas in 130 ml absolutem Methanol und 150 ml trockenem Benzol gelöst. Bei 0° wird unter Rühren trockener Chlorwasserstoff bis zur Sättigung eingeleitet. Die farblose Lösung wird noch über Nacht gerührt, dabei beginnt das Produkt, trans-4-Carbomethoxycyclohexancarbimidinsäure-methylester-hydrochlorid, auszukristallisieren. Man läßt weitere 3 Tage stehen und filtriert dann vom ausgefallenen Iminoesterhydrochlorid ab. 100,7 g des ausgefallenen Rohproduktes, suspendiert in 150 ml Methanol, werden nach dem Abkühlen auf ca. −40° mit 70 g flüssigem Ammoniak versetzt und in einem Autoklaven 24 Stunden bei +70° geschüttelt. Nach dem Abkühlen des Reaktionsgemisches auf Raumtemperatur und Ablassen des überschüssigen Ammoniaks dampft man die Suspension ein, wäscht das feste Produkt auf der Nutsche mehrmals mit Äther und kristallisiert den verbleibenden Rückstand aus Methanol um, wobei man das trans-4-Carbamoylcyclohexan-carboxamidin-hydrochlorid erhält.

b) Zu einer durch Zugabe von 3,2 g Natrium zu 125 ml Methanol bereiteten Natriummethylatlösung werden 12,6 g 3-Äthoxy-2-(p-n-heptylphenyl)acrolein und dann 9,9 g trans-4-Carbamoylcyclohexan-carboxamidin-hydrochlorid zugegeben. Das Reaktionsgemisch wird über Nacht bei 50° gerührt. Dann wird mit verdünnter Salzsäure angesäuert und die Suspension filtriert. Der Rückstand wird nutralgewaschen und im Vakuum getrocknet. Zur Reinigung wird mit Äther ausgekocht. Man erhält schwerlösliches trans-4-[5-(p-n-Heptylphenyl)-2-pyrimidinyl]cyclohexancarboxamid.

In analoger Weise können folgende Verbindungen hergestellt werden:

trans-4-[5-(p-Methylphenyl)-2-pyrimidinyl]cyclohexancarbonitril;
trans-4-[5-(p-Äthylphenyl)-2-pyrimidinyl]cyclohexancarbonitril; Smp. 172°, Klp. 197,5°
trans-4-[5-(p-Propylphenyl)-2-pyrimidinyl]cyclohexancarbonitril; Smp. 146°, Klp. 194,5°
trans-4-[5-(p-Butylphenyl)-2-pyrimidinyl]cyclohexancarbonitril; Smp. 139,5°, Klp. 179,5°
trans-4-[5-(p-Pentylphenyl)-2-pyrimidinyl]cyclohexancarbonitril; Smp. 112°, Klp. 175,5°
trans-4-[5-(p-Hexylphenyl)-2-pyrimidinyl]cyclohexancarbonitril; Smp. 84°, Klp. 163°
trans-4-[5-(p-Octylphenyl)-2-pyrimidinyl]cyclohexancarbonitril; 82,5°, Klp. 152,5°

## Beispiel 3

Zu einer Suspension von 13,2 g rohem trans-4-[5-(trans-4-n-Pentylcyclohexyl)-2-pyrimidinyl]cyclohexancarboxamid in 150 ml Pyridin tropft man unter Rühren 7,2 ml Benzolsulfochlorid. Im Ölbad wird dann 3 Stunden auf 55° erwärmt. Man gießt das Gemisch auf 500 ml Eiswasser und nimmt das Produkt in Methylenchlorid auf. Der Extrakt wird nacheinander mit 3 N-Salzsäure, gesättigter Natriumbicarbonatlösung und Wasser gewaschen, über Natriumsulfat getrocknet und eingedampft. Das Rohprodukt wird an einer Säule von 500 g Kieselgel mit Toluol/5% Aceton (v/v) chromatographiert. Die ersten Fraktionen sind Gemische und werden verworfen. Die späteren, nach Dünnschichtchromatographie einheitlichen Fraktionen werden in Hexan gelöst, heiß mit Aktivkohle filtriert und aus dem Filtrat umkristallisiert. Man erhält reines trans-4-[5-(trans-4-n-Pentylcyclohexyl)-2-pyrimidinyl]cyclohexancarbonitril; Smp. 109,5°, Klp. 175°.

Das als Ausgangsmaterial verwendete trans-4-[5-(trans-4-n-Pentyl-cyclohexyl)-2-pyrimidinyl]cyclohexancarboxamid kann wie folgt hergestellt werden:

Zu einer durch Zugabe von 4,4 g Natrium zu 175 ml Methanol bereiteten Natriummethylatlösung tropft man unter Rühren 16,0 g 3-Äthoxy-2-(trans-4-n-pentylcyclohexyl)acrolein (hergestellt gemäß Beispiel 1) und gibt dann 15,0 g trans-4-Carbamoylcyclohexancarboxamidin-hydrochlorid (hergestellt gemäß Beispiel 2) hinzu. Das Reaktionsgemisch wird über Nacht bei 50° gerührt. Dann wird mit verdünnter Salzsäure angesäuert. Die Suspension wird filtriert. Der Rückstand wird mit Wasser neutralgewaschen und im Vakuum getrocknet. Das erhaltene, rohe trans-4-[5-(trans-4-Pentylcyclohexyl)-2-pyrimidinyl]cyclohexancarboxamid wird direkt in der nächsten Stufe eingesetzt.

In analoger Weise können folgende Verbindungen hergestellt werden:

trans-4-[5-(trans-4-Methylcyclohexyl)-2-pyrimidinyl]cyclohexancarbonitril;
trans-4-[5-(trans-4-Äthylcyclohexyl)-2-pyrimidinyl]cyclohexancarbonitril; Smp. 166,5°, Klp. 161°
trans-4-[5-(trans-4-Propylcyclohexyl)-2-pyrimidinyl]cyclohexancarbonitril; Smp. 132°, Klp. 184°

trans-4-[5-(trans-4-Butylcyclohexyl)-2-pyrimidinyl]cyclohexancarbonitril; Smp. 121°, Klp. 172°
trans-4-[5-(trans-4-Hexylcyclohexyl)-2-pyrimidinyl]cyclohexancarbonitril;
trans-4-[5-(trans-4-Heptylcyclohexyl)-2-pyrimidinyl]cyclohexancarbonitril; Smp. 102,5°, Klp. 163,5°
(+)-trans-4-[5-[trans-4-(2-Methylbutyl)cyclohexyl]-2-pyrimidinyl]cyclohexancarbonitril; $\alpha_D = +10$, Smp. 124°, Klp. 125°
(+)-trans-4-[5-[trans-4-(3-Methylpentyl)cyclohexyl]-2-pyrimidinyl]cyclohexancarbonitril; $\alpha_D = +7$; Smp. 138°, Klp. 134°.

## Beispiel 4

Zu einer Suspension von 16,0 g trans-p-[5-(4-n-Propyloxycyclohexyl)-2-pyrimidinyl]benzamid in 170 ml Pyridin werden unter Rühren 9,2 ml Benzolsulfochlorid getropft. Im Ölbad wird dann 15 Stunden auf 40° erwärmt. Das klare Reaktionsgemisch gießt man auf eine Mischung von 200 g Eis und 24,1 ml konz. Salzsäure und nimmt das Produkt in Methylenchlorid auf. Der Extrakt wird dreimal mit je 195 ml 3 N-Salzsäure, dann mit 100 ml gesättigter Natriumbicarbonatlösung und mit Wasser gewaschen, über Natriumsulfat getrocknet und eingedampft. Das Rohprodukt wird an einer Säule von 200 g Kieselgel mit Benzol, Benzol/1% und 2% Aceton (v/v) chromatographiert. Die nach Dünnschichtchromatographie reinen Fraktionen werden aus Aceton-Hexan umkristallisiert. Man erhält trans-p-[5-(4-n-Propyloxycyclohexyl)-2-pyrimidinyl]benzonitril; Smp. 114,5°, Klp. 223,5°.

Das als Ausgangsmaterial verwendete trans-p-[5-(4-n-Propyloxy-cyclohexyl)-2-pyrimidinyl]benzamid kann wie folgt hergestellt werden:

a) Eine Lösung von 41,0 g trans-4-n-Propyloxycyclohexancarbonsäure in 90 ml trockenem Äther wird zu einer Suspension von 8,4 g Lithiumaluminiumhydrid in 390 ml trockenem Äther so zugetropft, das das Gemisch leicht siedet. Nach beendetem Zutropfen wird noch 1 Stunde gerührt. Dann werden vorsichtig 22 ml Aceton und dann 35 ml Wasser zugetropft. Das Reaktionsgemisch wird auf Eiswasser gegossen und mit 280 ml 3 N-Salzsäure kongosauer gestellt. Die organische Phase wird abgetrennt und der Reihe nach mit Wasser, Natriumhydrogencarbonatlösung und Wasser gewaschen, über Natriumsulfat getrocknet und eingedampft. Das erhaltene trans-4-n-Propyloxycyclohexancarbinol wird roh weiter umgesetzt.

b) Zu einer Suspension von 85,9 g Pyridiniumchlorchromat in 620 ml Methylenchlorid gibt man eine Lösung von 38,6 g trans-4-n-Propyloxycyclohexancarbinol in 70 ml Methylenchlorid und rührt das Gemisch 2 1/2 Stunden bei Raumtemperatur. Dann verdünnt man mit 275 ml Äther, rührt noch 15 Minuten und dekantiert die Lösung vom teerartigen Niederschlag. Der Niederschlag wird mit Äther nachgewaschen. Die vereinigten organischen Lösungen werden durch eine Säule von 175 g Florisil filtriert. Der nach Eindampfen des Filtrats erhaltene trans-4-n-Propyloxycyclohexancarboxaldehyd wird direkt weiter verarbeitet.

c) Eine Suspension von 94,6 g (Methoxymethyl)triphenylphosphoniumchlorid in 1000 ml trockenem Äther wird mit 33,0 g Kalium-t-butylat versetzt. Nach 45minütigem Rühren wird eine Lösung von 31,4 g trans-4-n-Propyloxycyclohexancarboxaldehyd in 200 ml Äther zugetropft. Das Reaktionsgemisch wird noch 2 1/2 Stunden gerührt und dann auf 1,2 l Eiswasser gegossen. Die organische Phase wird abgetrennt, mit Wasser neutralgewaschen, über Natriumsulfat getrocknet und eingedampft. Der erhaltene rohe trans-2-(4-n-Propyloxycyclohexyl)vinylmethyläther wird zur Abtrennung des restlichen Triphenylphosphins bei 82–85°/0,6 mm Hg destilliert.

d) Zu einer auf 0° gekühlten Lösung von 8,9 ml Bortrifluoridäthylätherat in 535 ml frisch destilliertem Orthoameisensäuretriäthylester wird unter Rühren 28,0 g trans-2-(4-n-Propyloxycyclohexyl)vinylmethyläther getropft und das Reaktionsgemisch über Nacht bei Raumtemperatur gerührt. Danach verdünnt man mit 735 ml Toluol, wäscht mit 155 ml Natriumbicarbonatlösung, dann mit Wasser, trocknet über Natriumsulfat, filtriert und dampft ein. Das rohe trans-(4-n-Propyloxycyclohexyl)malon-bis-acetal wird ohne Reinigung weiter umgesetzt.

e) 49,0 g rohes trans-(4-n-Propyloxycyclohexyl)malon-bis-acetal werden mit 3,1 ml Wasser und 132 mg p-Toluolsofonsäure versetzt. Das Gemisch wird 2 Stunden bei 85° gerührt. Dann läßt man erkalten, gibt 1,4 g Natriumhydrogencarbonat zu und rührt bei Raumtemperatur 1 1/2 Stunden. Das Reaktionsgemisch wird mit Äther verdünnt, dreimal mit je 44 ml eiskalter 3 N-Natronlauge und dann mit Wasser gewaschen, über Natriumsulfat getrocknet, filtriert und eingedampft. Das rohe 3-Äthoxy-2-(trans-4-n-propyloxycyclohexyl)acrolein wird sofort weiter verarbeitet.

f) Aus 9,7 g Natrium und 392 ml absolutem Methanol wird eine Natriummethylat-Lösung bereitet. Bei Raumtemperatur gibt man unter Rühren 31,4 g p-Carbamoyl-benzamidinhydrochlorid und

dann 29,8 g 3-Äthoxy-2-(trans-4-n-propyloxycyclohexyl)acrolein, gelöst in 100 ml absolutem Methanol, zu, rührt über Nacht bei 50° unter Stickstoffbegasung, läßt die gelbe Suspension auf Raumtemperatur abkühlen und säuert mit 131 ml 3 N-Salzsäure an. Die Suspension wird genutscht, der Niederschlag mit Wasser neutral gewaschen und getrocknet. Das gelbliche, rohe trans-p-[5-(4-n-Propyloxycyclohexyl)-2-pyrimidinyl]benzamid wird zur Reinigung aus Dioxan umkristallisiert; Smp. 261 – 264° (Zers.).

In analoger Weise können folgende Verbindungen hergestellt werden:

trans-p-[5-(4-Äthoxycyclohexyl)-2-pyrimidinyl]benzonitril; Smp. 144,5°, Klp. 232°
trans-p-[5-(4-Pentyloxycyclohexyl)-2-pyrimidinyl]benzonitril; Smp. 93°, Klp. 205°.

**Patentansprüche für die Vertragsstaaten BE, CH, DE, FR, IT, NL**

1. Verbindungen der allgemeinen Formel

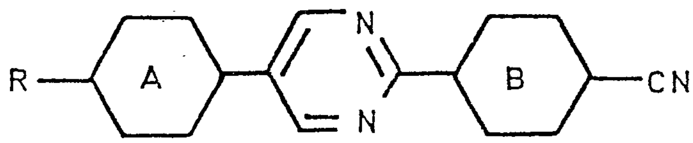

I

worin wenigstens einer der Ringe A und B einen trans-1,4-disubstituierten Cyclohexanring darstellt und der andere gegebenenfalls aromatisch ist und R eine geradkettige Alkyl- oder Alkoxygruppe mit 1 bis 10 Kohlenstoffatomen oder eine verzweigte Alkylgruppe

$C_2H_5-CH(CH_3)-(CH_2)_n-$,

worin n eine ganze Zahl von 1 bis 3 ist, bedeutet.

2. trans-p-[5-(4-Alkyl- oder 4-n-Alkoxycyclohexyl)-2-pyrimidinyl]benzonitrile der Formel

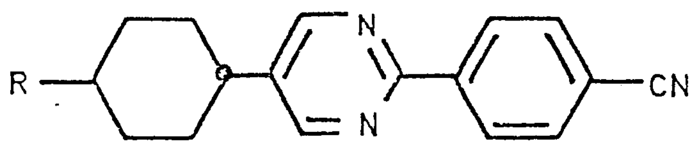

I A

worin R geradkettiges Alkyl mit 2 bis 8 Kohlenstoffatomen, geradkettiges Alkoxy mit bis zu 6 Kohlenstoffatomen oder eine verzweigte Alkylgruppe

$C_2H_5-CH(CH_3)-(CH_2)_n-$,

worin n eine ganze Zahl von 1 bis 3 ist, darstellt.

3. trans-4-[5-(p-Alkyl- oder p-n-Alkoxyphenyl)-2-pyrimidinyl]cyclohexancarbonitrile der Formel

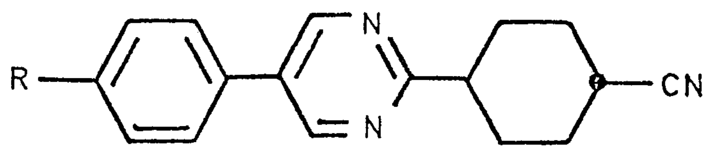

I B

worin R geradkettiges Alkyl mit 2 bis 8 Kohlenstoffatomen, geradkettiges Alkoxy mit bis zu 6 Kohlenstoffatomen oder eine verzweigte Alkylgruppe

$C_2H_5-CH(CH_3)-(CH_2)_n-$,

worin n eine ganze Zahl von 1 bis 3 ist, darstellt.

4. trans-4-[5-(trans-4-Alkyl- oder trans-4-n-Alkoxycyclohexyl)-2-pyrimidinyl]cyclohexancarbonitrile der Formel

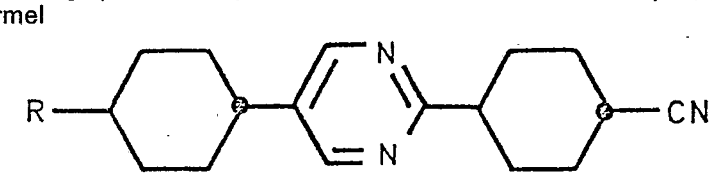

I C

worin R geradkettiges Alkyl mit 2 bis 8 Kohlenstoffatomen, geradkettiges Alkoxy mit bis zu 6 Kohlenstoffatomen oder eine verzweigte Alkylgruppe

$C_2H_5-CH(CH_3)-(CH_2)_n-$,

worin n eine ganze Zahl von 1 bis 3 ist, darstellt.

5. trans-p-[5-(4-Äthylcyclohexyl)-2-pyrimidinyl]benzonitril.

6. trans-p-[5-(4-Pentylcyclohexyl)-2-pyrimidinyl]benzonitril.

7. trans-p-[5-(4-Heptylcyclohexyl)-2-pyrimidinyl]benzonitril.

8. Flüssigkristalline Gemische, dadurch gekennzeichnet, daß sie eine oder mehrere Verbindungen der in Anspruch 1 definierten Formel I und, gegebenenfalls, andere nematische und/oder nicht nematische Verbindungen und/oder einen oder mehrere dichroitische Farbstoffe enthalten.

9. Flüssigkristalline Gemische nach Anspruch 8, dadurch gekennzeichnet, daß sie eine oder mehrere Verbindungen der in Anspruch 1 definierten Formel I und einen oder mehrere Farbstoffe der allgemeinen Formeln

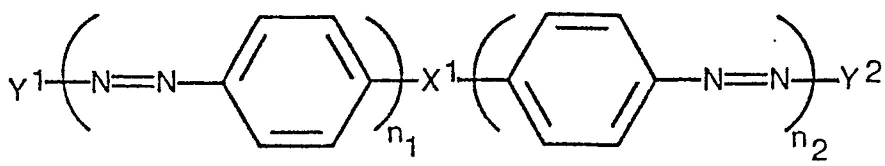

IV

worin $n_1$ und $n_2$ ganze Zahlen von 0 bis 2 und $X^1$ eine Azo- oder Azoxygruppe bedeuten, $Y^1$ und $Y^2$ gleich oder verschieden sein können und einen der Substituenten

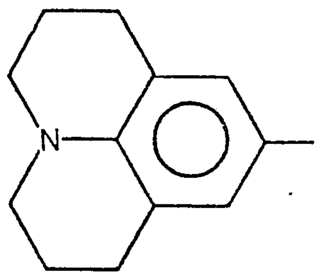

V oder

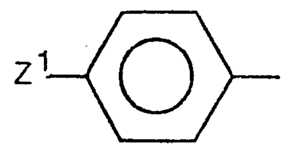

VI

bezeichnen und $Z^1$ Wasserstoff, Cyano, Nitro, Phenyl, p-substituiertes Phenyl, Hydroxy, Alkoxy, Amino, Dialkylamino oder Pyrrolidyl darstellt; oder Derivate der Formel IV, bei denen einer der Benzolringe zusätzlich eine oder mehrere der Gruppen Halogen, Methyl, halogensubstituiertes Methyl oder Methoxy besitzt;

oder

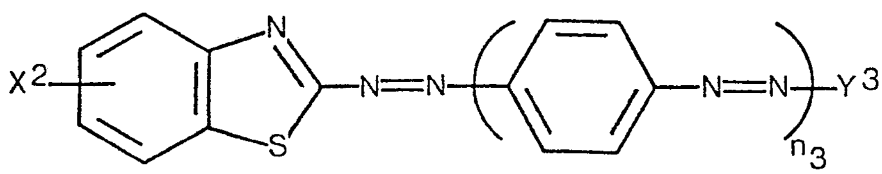

VII

worin $n_3$ 0 oder 1 ist, $Y^3$ einen Substituenten der Formel V oder VI bezeichnet, worin $Z^1$ Dialkylamino oder Pyrrolidyl bedeutet, und $X^2$ Wasserstoff, Cyano, Nitro, Halogen, Alkyl, Alkoxy, eine gegebenenfalls substituierte Phenyl- oder Benzothiazol-2-ylgruppe, Alkoxycarbonyl oder Alkylsulfonyl in 5- oder 6-Stellung darstellt;

oder

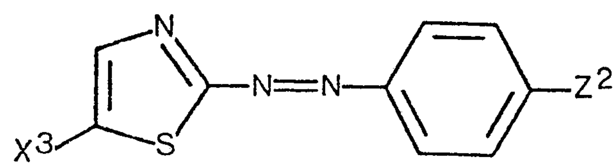

VIII

worin $X^3$ Cyano, Nitro oder Alkyl bedeutet, und $Z^2$ Hydroxy, Alkoxy, Aryl oder eine gegebenenfalls Alkyl- oder Aryl-substituierte Amino-, Pyrrolidyl- oder Piperidylgruppe darstellt; oder Derivate der Formel VIII, bei denen der Benzolring zusätzlich eine oder mehrere der Gruppen Halogen, Hydroxy, Methyl oder Methoxy besitzt;

oder

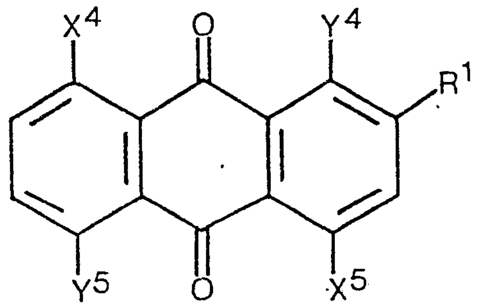

IX

worin $X^4$ und $X^5$ Hydroxy, $Y^4$ und $Y^5$ Amino und $R^1$ einen Substituenten der Formel VI oder $X^4$, $Y^5$ und $R^1$ Wasserstoff, $X^5$ Hydroxy und $Y^4$ die Gruppe $-NHR^2$ oder $X^4$, $X^5$ und $R^1$ Wasserstoff und $Y^4$ und $Y^5$ die Gruppe $-NHR^2$ bedeuten, $R^2$ einen Substituenten der Formel VI bezeichnet und $Z^1$ in den Substituenten der Formel VI eine Alkyl-, Alkoxy- oder Dialkylamino-Gruppe darstellt;

oder

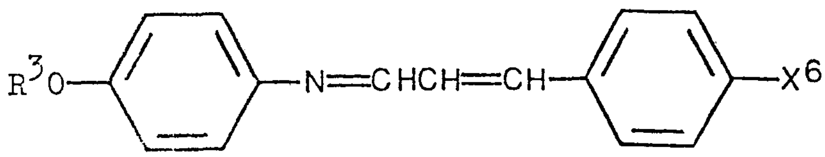

X

worin $R^3$ eine Alkyl- und $X^6$ eine Cyano- oder eine Nitrogruppe bedeutet;

oder

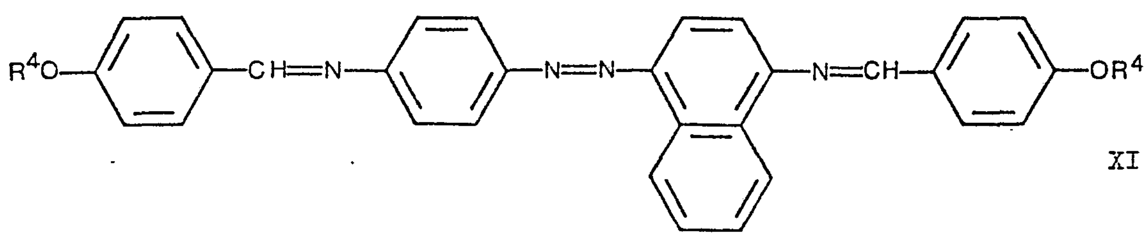

XI

worin $R^4$ eine Alkylgruppe darstellt;

oder

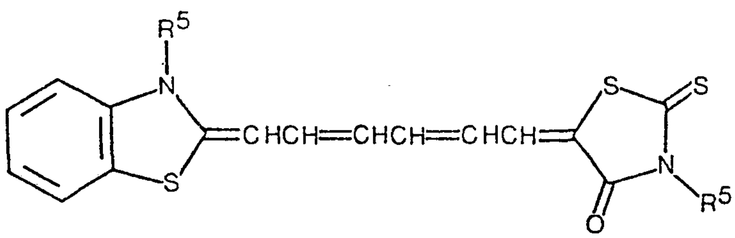

XII

worin $R^5$ eine Alkylgruppe darstellt;

und, gegebenenfalls, weitere nematische und/oder nicht nematische Verbindungen enthalten.

10. Flüssigkristalline Gemische nach Anspruch 9, dadurch gekennzeichnet, daß sie mindestens einen Farbstoff der Formel

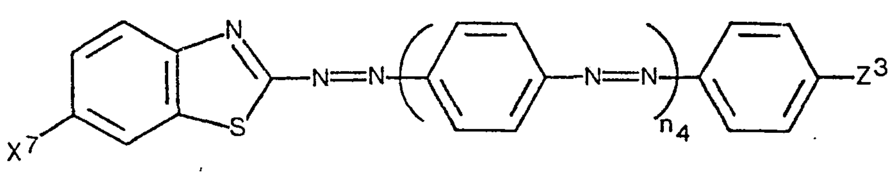

VIIa

worin $n_4$ 0 oder 1 ist, $Z^3$ die Dimethylamino- oder die Diäthylamino-Gruppe bedeutet und $X^7$

Wasserstoff, Methyl, Äthoxy, n-Butyloxy, Nitro oder n-Butylsulfonyl darstellt, enthalten.

11. Flüssigkristalline Gemische nach Anspruch 9, dadurch gekennzeichnet, daß sie mindestens einen Farbstoff der Formel

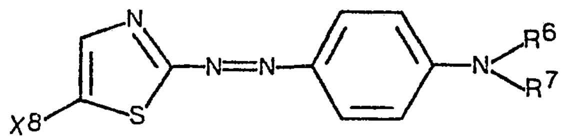

VIIIa

worin $R^6$, $R^7$ und $X^8$ Methyl oder $R^6$ Methyl, $X^8$ Nitro und $R^7$ Methyl, Phenyl oder p-n-Butylsulfonyl bedeuten, enthalten.

12. Flüssigkristalline Gemische nach Anspruch 9, dadurch gekennzeichnet, daß sie mindestens einen Farbstoff der Formel

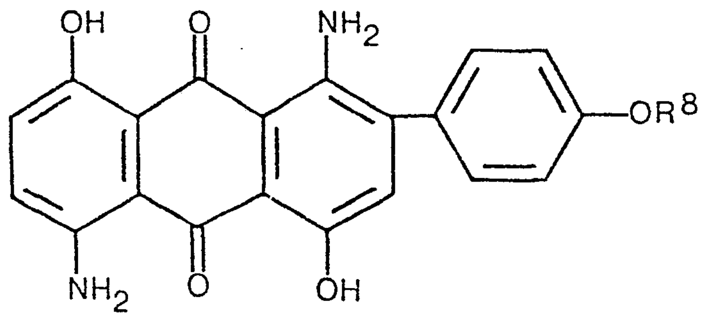

IXa

worin $R^8$ n-Alkyl mit 1 bis 10 Kohlenstoffatomen bedeutet, enthalten.

13. Flüssigkristalline Gemische nach Anspruch 9, dadurch gekennzeichnet, daß sie mindestens einen Farbstoff der Formel

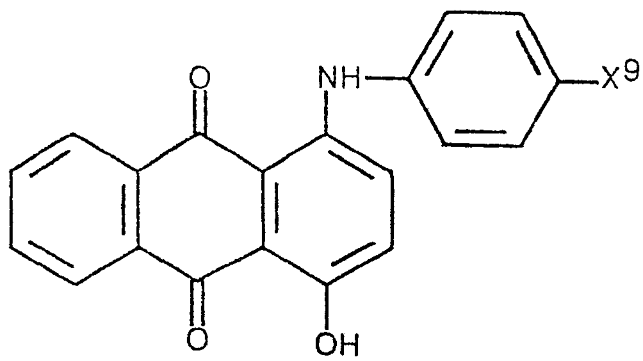

IXb

worin $X^9$ n-Butyl, n-Nonyloxy oder Dimethylamino bedeutet, enthalten.

14. Flüssigkristalline Gemische nach Anspruch 9, dadurch gekennzeichnet, daß sie mindestens einen Farbstoff der Formel

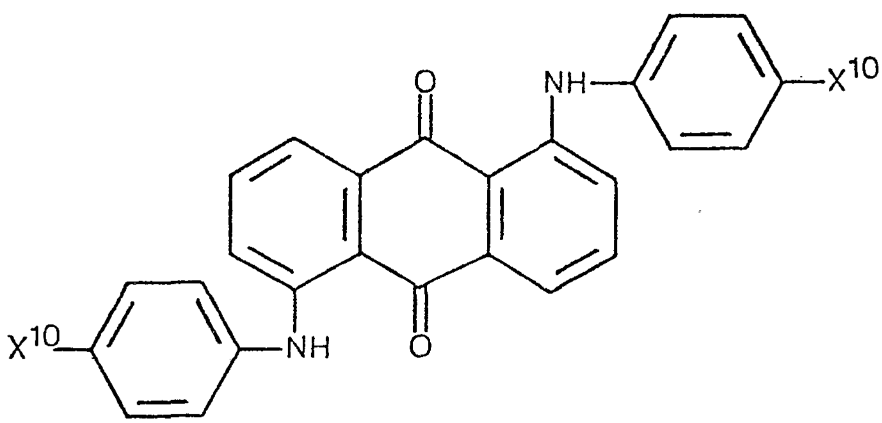

IXc

worin $X^{10}$ Äthyl, n-Butyl, Isopropyl, Pentyloxy oder Dimethylamino bedeutet, enthalten.

15. Flüssigkristalline Gemische nach Anspruch 9, dadurch gekennzeichnet, daß sie den Farbstoff der Formel

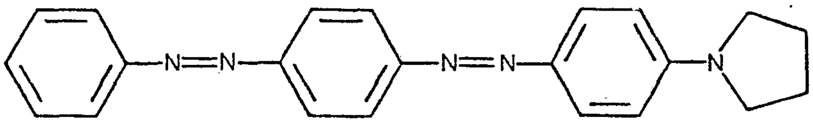

IVa

und/oder den Farbstoff der Formel X, worin $R^3$ n-Butyl und $X^6$ Nitro bedeutet, und/oder den Farbstoff der Formel XI, worin $R^4$ Äthyl bedeutet, und/oder den Farbstoff der Formel XII, worin $R^5$ Äthyl bedeutet, enthalten.

16. Flüssigkristallines Gemisch gemäß Anspruch 8, dadurch gekennzeichnet, daß es
4'-Pentyl-4-cyanobiphenyl,
trans-4-Propylcyclohexancarbonsäure-p-cyanophenylester,
trans-4-Pentylcyclohexancarbonsäure-p-cyanophenylester,
trans-4-Pentylcyclohexancarbonsäure-p-(methoxy)phenylester,
trans-p-[5-(4-Äthylcyclohexyl)-2-pyrimidinyl]benzonitril,
trans-p-[5-(4-Pentylcyclohexyl)-2-pyrimidinyl]benzonitril und
trans-p-[5-(4-Heptylcyclohexyl)-2-pyrimidinyl]benzonitril
enthält.

17. Flüssigkristallines Gemisch gemäß Anspruch 8, dadurch gekennzeichnet, daß es
p-Butylbenzoesäure-p'-cyanophenylester,
p-(5-Pentyl-2-pyrimidinyl)benzonitril,
p-(5-Heptyl-2-pyrimidinyl)benzonitril,
trans-4-Butylcyclohexancarbonsäure-p-cyanophenylester,
trans-4-Pentylcyclohexancarbonsäure-p-cyanophenylester,
trans-4-Butylcyclohexancarbonsäure-p-(äthoxy)phenylester,
trans-4-Pentylcyclohexancarbonsäure-p-(methoxy)phenylester und
trans-p-[5-(4-Äthylcyclohexyl)-2-pyrimidinyl]benzonitril
enthält.

18. Flüssigkristallines Gemisch gemäß Anspruch 8, dadurch gekennzeichnet, daß es
p-Butylbenzoesäure-p'-cyanophenylester,
p-(5-Pentyl-2-pyrimidinyl)benzonitril,
p-(5-Heptyl-2-pyrimidinyl)benzonitril,
trans-4-Propylcyclohexancarbonsäure-p-cyanophenylester,
trans-4-Pentylcyclohexancarbonsäure-p-cyanophenylester,
trans-4-Butylcyclohexancarbonsäure-p-(äthoxy)phenylester,
trans-4-Pentylcyclohexancarbonsäure-p-(methoxy)phenylester,
trans-p-[5-(4-Pentylcyclohexyl)-2-pyrimidinyl]benzonitril und
trans-p-[5-(4-Heptylcyclohexyl)-2-pyrimidinyl]benzonitril
enthält.

19. Flüssigkristallines Gemisch gemäß Anspruch 8, dadurch gekennzeichnet, daß es
p-Butylbenzoesäure-p'-cyanophenylester,
p-(5-Pentyl-2-pyrimidinyl)benzonitril,
p-(5-Heptyl-2-pyrimidinyl)benzonitril,
trans-4-Propylcyclohexancarbonsäure-p-cyanophenylester,
trans-4-Pentylcyclohexancarbonsäure-p-cyanophenylester,
trans-4-Butylcyclohexancarbonsäure-p-(äthoxy)phenylester,
trans-4-Pentylcyclohexancarbonsäure-p-(methoxy)phenylester und
trans-p-[5-(4-Äthylcyclohexyl)-2-pyrimidinyl]benzonitril
enthält.

20. Flüssigkristallines Gemisch gemäß Anspruch 8, dadurch gekennzeichnet, daß es
4'-Heptyl-4-cyanobiphenyl,
p-(5-Pentyl-2-pyrimidinyl)benzonitril,
p-(5-Heptyl-2-pyrimidinyl)benzonitril,
trans-4-Propylcyclohexancarbonsäure-p-cyanophenylester,
trans-4-Butylcyclohexancarbonsäure-p-cyanophenylester,
trans-4-Pentylcyclohexancarbonsäure-p-cyanophenylester,
trans-4-Pentylcyclohexancarbonsäure-p-(methoxy)phenylester,
trans-4-Butylcyclohexancarbonsäure-p-(äthoxy)phenylester und
trans-p-[5-(4-Äthylcyclohexyl)-2-pyrimidinyl]benzonitril
enthält.

21. Flüssigkristallines Gemisch gemäß Anspruch 8, dadurch gekennzeichnet, daß es
p-Butylbenzoesäure-p'-cyanophenylester,
p-(5-Pentyl-2-pyrimidinyl)benzonitril,
p-(5-Heptyl-2-pyrimidinyl)benzonitril,
trans-4-Propylcyclohexancarbonsäure-p-cyanophenylester,
trans-4-Butylcyclohexancarbonsäure-p-cyanophenylester,

trans-4-Pentylcyclohexancarbonsäure-p-cyanophenylester,
trans-4-Butylcyclohexancarbonsäure-p-(äthoxy)phenylester,
4''-Pentyl-4-cyano-p-terphenyl und
trans-p-[5-(4-Äthylcyclohexyl)-2-pyrimidinyl]benzonitril
enthält.

22. Flüssigkristallines Gemisch gemäß Anspruch 8, dadurch gekennzeichnet, daß es
4'-Pentyl-4-cyanobiphenyl,
trans-4-Propylcyclohexancarbonsäure-p-cyanophenylester,
trans-4-Pentylcyclohexancarbonsäure-p-cyanophenylester,
trans-4-Butylcyclohexancarbonsäure-p-(äthoxy)phenylester,
trans-4-Pentylcyclohexancarbonsäure-p-(methoxy)phenylester,
trans-p-[5-(4-Äthylcyclohexyl)-2-pyrimidinyl]benzonitril,
trans-p-[5-(4-Pentylcyclohexyl)-2-pyrimidinyl]benzonitril und
trans-p[5-(4-Heptylcyclohexyl)-2-pyrimidinyl]benzonitril
enthält.

23. Flüssigkristallines Gemisch gemäß Anspruch 8, dadurch gekennzeichnet, daß es
4'-Pentyl-4-cyanobiphenyl,
p-Butylbenzoesäure-p'-cyanophenylester,
p-(5-Pentyl-2-pyrimidinyl)benzonitril,
trans-4-Propylcyclohexancarbonsäure-p-cyanophenylester,
trans-4-Pentylcyclohexancarbonsäure-p-cyanophenylester,
trans-4-Pentylcyclohexancarbonsäure-p-(methoxy)phenylester und
trans-p-[5-(4-Äthylcyclohexyl)-2-pyrimidinyl]benzonitril
enthält.

24. Flüssigkristallines Gemisch gemäß Anspruch 8, dadurch gekennzeichnet, daß es
4'-Heptyl-4-cyanobiphenyl,
p-Butylbenzoesäure-p'-cyanophenylester,
p-(5-Pentyl-2-pyrimidinyl)benzonitril,
p-(5-Heptyl-2-pyrimidinyl)benzonitril,
trans-4-Propylcyclohexancarbonsäure-p-cyanophenylester,
trans-4-Pentylcyclohexancarbonsäure-p-cyanophenylester,
trans-4-Butylcyclohexancarbonsäure-p-(äthoxy)phenylester und
trans-p-[5-(4-Äthylcyclohexyl)-2-pyrimidinyl]benzonitril
enthält.

25. Flüssigkristallines Gemisch gemäß Anspruch 8, dadurch gekennzeichnet, daß es
4'-Pentyl-4-cyanobiphenyl,
4'-Heptyl-4-cyanobiphenyl,
p-(5-Pentyl-2-pyrimidinyl)benzonitril,
p-(5-Heptyl-2-pyrimidinyl)benzonitril,
trans-4-Butylcyclohexancarbonsäure-p-(äthoxy)phenylester und
trans-p-[5-(4-Pentylcyclohexyl)-2-pyrimidinyl]benzonitril
enthält.

26. Flüssigkristallines Gemisch gemäß Anspruch 8, dadurch gekennzeichnet, daß es
4'-Pentyl-4-cyanobiphenyl,
4'-Heptyl-4-cyanobiphenyl,
p-(5-Pentyl-2-pyrimidinyl)benzonitril,
p-(5-Heptyl-2-pyrimidinyl)benzonitril und
trans-p-[5-(4-Äthylcyclohexyl)-2-pyrimidinyl]benzonitril
enthält.

27. Flüssigkristallines Gemisch gemäß Anspruch 8, dadurch gekennzeichnet, daß es
4'-Pentyl-4-cyanobiphenyl,
4'-Heptyl-4-cyanobiphenyl,
p-(5-Pentyl-2-pyrimidinyl)benzonitril,
p-(5-Heptyl-2-pyrimidinyl)benzonitril,
trans-4-Butylcyclohexancarbonsäure-p-(äthoxy)phenylester,
trans-4-Pentylcyclohexancarbonsäure-p-(methoxy)phenylester und
trans-p-[5-(4-Pentylcyclohexyl)-2-pyrimidinyl]benzonitril
enthält.

28. Flüssigkristallines Gemisch gemäß Anspruch 8, dadurch gekennzeichnet, daß es
4'-Pentyl-4-cyanobiphenyl,
4'-Octyloxy-4-cyanobiphenyl,
p-(5-Pentyl-2-pyrimidinyl)benzonitril,
p-(5-Heptyl-2-pyrimidinyl)benzonitril und
trans-p-[5-(4-Pentylcyclohexyl)-2-pyrimidinyl]benzonitril
enthält.

29. Flüssigkristallines Gemisch gemäß Anspruch 8, dadurch gekennzeichnet, daß es
4′-Pentyl-4-cyanobiphenyl,
p-Butylbenzoesäure-p′-cyanophenylester,
p-(5-Pentyl-2-pyrimidinyl)benzonitril,
p-(5-Heptyl-2-pyrimidinyl)benzonitril,
trans-4-Butylcyclohexancarbonsäure-p-cyanophenylester,
trans-4-Pentylcyclohexancarbonsäure-p-cyanophenylester,
trans-4-Butylcyclohexancarbonsäure-p-(äthoxy)phenylester,
trans-4-Pentylcyclohexancarbonsäure-p-(methoxy)phenylester und
trans-p-[5-(4-Äthylcyclohexyl)-2-pyrimidinyl]benzonitril
enthält.

30. Flüssigkristallines Gemisch gemäß Anspruch 8, dadurch gekennzeichnet, daß es
4′-Heptyl-4-cyanobiphenyl,
trans-4-Propylcyclohexancarbonsäure-p-cyanophenylester,
trans-4-Butylcyclohexancarbonsäure-p-(äthoxy)phenylester,
trans-4-Pentylcyclohexancarbonsäure-p-(methoxy)phenylester,
trans-4-Pentylcyclohexancarbonsäure-p-(propyloxy)phenylester und
trans-p-[5-(4-Pentylcyclohexyl)-2-pyrimidinyl]benzonitril
enthält.

31. Verfahren zur Herstellung von Verbindungen der allgemeinen Formel

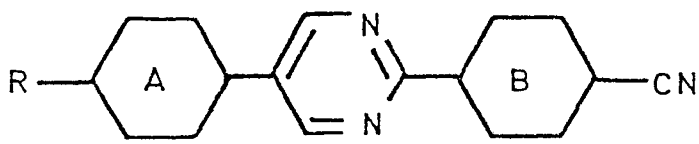

I

worin wenigstens einer der Ringe A und B einen trans-1,4-disubstituierten Cyclohexanring darstellt und der andere gegebenenfalls atomatisch ist und R eine geradkettige Alkyl- oder Alkoxygruppe mit 1 bis 10 Kohlenstoffatomen oder eine verzweigte Alkylgruppe

$C_2H_5-CH(CH_3)-(CH_2)_n-$,

worin n eine ganze Zahl von 1 bis 3 ist, bedeutet, dadurch gekennzeichnet, daß man

a) eine Verbindung der allgemeinen Formel

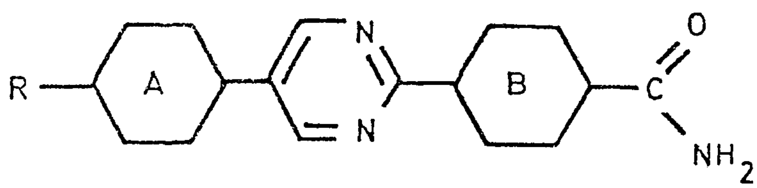

II

worin A, B und R die obige Bedeutung haben, dehydratisiert, oder

b) zur Herstellung einer Verbindung der Formel I, worin der Ring B aromatisch ist, eine Verbindung der allgemeinen Formel

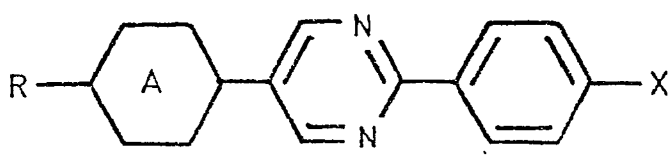

III

worin R und A die obige Bedeutung haben und X Fluor, Chlor oder Brom darstellt, mit Kupfer-(I)-cyanid oder mit Natrium- oder Kaliumcyanid umsetzt.

32. Verfahren zur Herstellung von flüssigkristallinen Gemischen, dadurch gekennzeichnet, daß man eine oder mehrere Verbindungen der in Anspruch 1 definierten Formel I mit anderen nematischen und/oder nicht nematischen Verbindungen und/oder einem oder mehreren dichroitischen Farbstoffen vermischt.

33. Verfahren zur Herstellung einer elektro-optischen Vorrichtung, dadurch gekennzeichnet, daß man eine oder mehrere Verbindungen der in Anspruch 1 definierten Formel I, gegebenenfalls im Gemisch mit anderen nematischen und/oder nicht nematischen Verbindungen und/oder einem mehreren dichroitischen Farbstoffen, in eine geeignete Zelle einbringt.

34. Verwendung der Verbindungen der in Anspruch 1 definierten Formel I für elektro-optische Zwecke.

35. Elektro-optische Vorrichtung, enthaltend eine oder mehrere Verbindungen der in Anspruch 1 definierten Formel I in flüssigkristallinem Zustand.

**Patentansprüche für den Vertragsstaat Österreich**

1. Flüssigkristalline Gemische, dadurch gekennzeichnet, daß sie eine oder mehrere Verbindungen der allgemeinen Formel

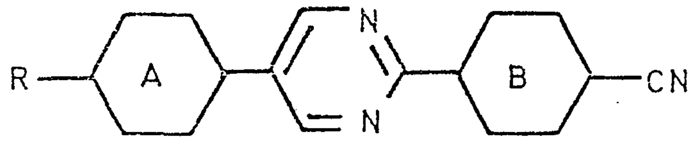

I

worin wenigstens einer der Ringe A und B einen trans-1,4-disubstituierten Cyclohexanring darstellt und der andere gegebenenfalls aromatisch ist und R eine geradkettige Alkyl- oder Alkoxygruppe mit 1 bis 10 Kohlenstoffatomen oder eine verzweigte Alkylgruppe

$C_2H_5-CH(CH_3)-(CH_2)_n-$,

worin n eine ganze Zahl von 1 bis 3 ist, bedeutet,

und, gegebenenfalls, andere nematische und/oder nicht nematische Verbindungen und/oder einen oder mehrere dichroitische Farbstoffe enthalten.

2. Flüssigkristalline Gemische gemäß Anspruch 1, dadurch gekennzeichnet, daß sie eine oder mehrere Verbindungen der allgemeinen Formel

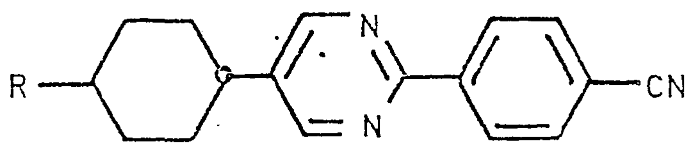

I A

worin R geradkettiges Alkyl mit 2 bis 8 Kohlenstoffatomen, geradkettiges Alkoxy mit bis zu 6 Kohlenstoffatomen oder eine verzweigte Alkylgruppe

$C_2H_5-CH(CH_3)-(CH_2)_n-$,

worin n eine ganze Zahl von 1 bis 3 ist, darstellt, enthalten.

3. Flüssigkristalline Gemische gemäß Anspruch 1, dadurch gekennzeichnet, daß sie eine oder mehrere Verbindungen der allgemeinen Formel

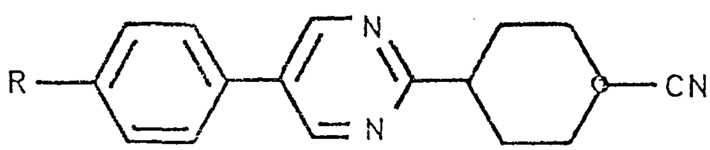

I B

worin R geradkettiges Alkyl mit 2 bis 8 Kohlenstoffatomen, geradkettiges Alkoxy mit bis zu 6 Kohlenstoffatomen oder eine verzweigte Alkylgruppe

$C_2H_5-CH(CH_3)-(CH_2)_n-$,

worin n eine ganze Zahl von 1 bis 3 ist, darstellt, enthalten.

4. Flüssigkristalline Gemische gemäß Anspruch 1, dadurch gekennzeichnet, daß sie eine oder mehrere Verbindungen der allgemeinen Formel

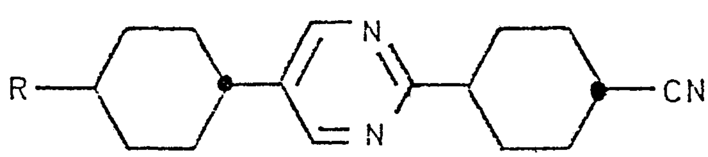

I C

worin R geradkettiges Alkyl mit 2 bis 8 Kohlenstoffatomen, geradkettiges Alkoxy mit bis zu 6

Kohlenstoffatomen oder eine verzweigte Alkylgruppe

$C_2H_5-CH(CH_3)-(CH_2)_n-$,

worin n eine ganze Zahl von 1 bis 3 ist, darstellt, enthalten.

5. Flüssigkristalline Gemische gemäß Anspruch 1 oder 2, dadurch gekennzeichnet, daß sie trans-p-[5-(4-Äthylcyclohexyl)-2-pyrimidinyl]benzonitril enthalten.

6. Flüssigkristalline Gemische gemäß Anspruch 1 oder 2, dadurch gekennzeichnet, daß sie trans-p-[5-(4-Pentylcyclohexyl)-2-pyrimidinyl]benzonitril enthalten.

7. Flüssigkristalline Gemische gemäß Anspruch 1 oder 2, dadurch gekennzeichnet, daß sie trans-p-[5-(4-Heptylcyclohexyl)-2-pyrimidinyl]benzonitril enthalten.

8. Flüssigkristalline Gemische gemäß einem der Ansprüche 1 bis 7, dadurch gekennzeichnet, daß sie eine oder mehrere Verbindungen der Formel I und einen oder mehrere dichroitische Farbstoffe der allgemeinen Formeln

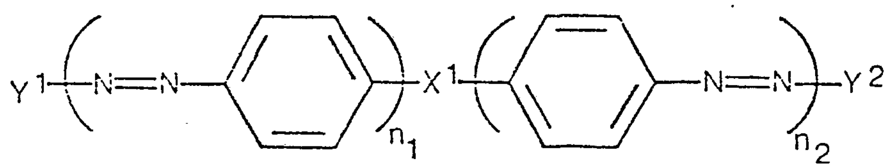

IV

worin $n_1$ und $n_2$ ganze Zahlen von 0 bis 2 und $X^1$ eine Azo- oder Azoxygruppe bedeuten, $Y^1$ und $Y^2$ gleich oder verschieden sein können und einen der Substituenten

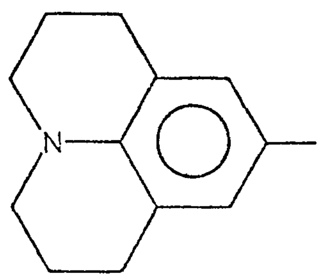

V oder

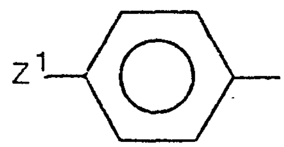

VI

bezeichnen und $Z^1$ Wasserstoff, Cyano, Nitro, Phenyl, p-substituiertes Phenyl, Hydroxy, Alkoxy, Amino, Dialkylamino oder Pyrrolidyl darstellt; oder Derivate der Formel IV, bei denen einer der Benzolringe zusätzlich eine oder mehrere der Gruppen Halogen, Methyl, halogensubstituiertes Methyl oder Methoxy besitzt;

oder

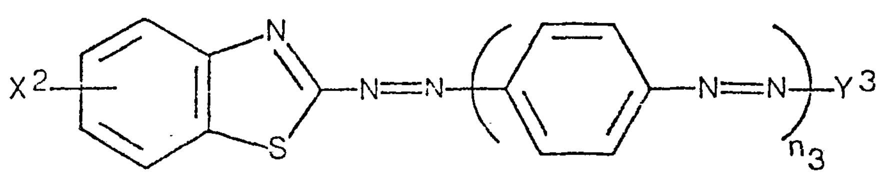

VII

worin $n_3$ 0 oder 1 ist, $Y^3$ einen Substituenten der Formel V oder VI bezeichnet, worin $Z^1$ Dialkylamino oder Pyrrolidyl bedeutet, und $X^2$ Wasserstoff, Cyano, Nitro, Halogen, Alkyl, Alkoxy, eine gegebenenfalls substituierte Phenyl- oder Benzothiazol-2-ylgruppe, Alkoxycarbonyl oder Alkylsulfonyl in 5- oder 6-Stellung darstellt;

oder

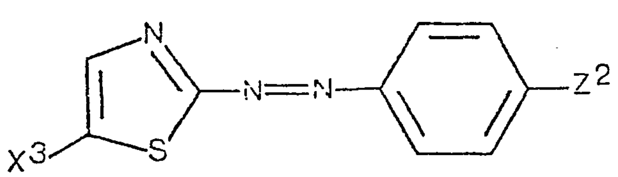

VIII

worin $X^3$ Cyano, Nitro oder Alkyl bedeutet, und $Z^2$ Hydroxy, Alkoxy, Aryl oder eine gegebenenfalls Alkyl- oder Aryl-substituierte Amino-, Pyrrolidyl- oder Piperidylgruppe darstellt; oder Derivate der Formel VIII, bei denen der Benzolring zusätzlich eine oder mehrere der Gruppen Halogen, Hydroxy, Methyl oder Methoxy besitzt;

oder

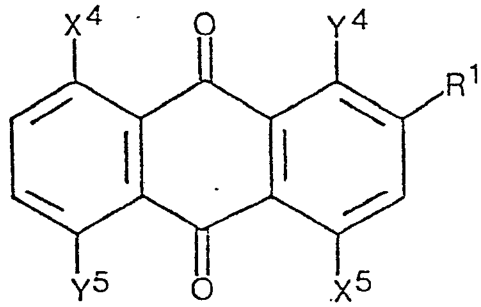

IX

worin $X^4$ und $X^5$ Hydroxy, $Y^4$ und $Y^5$ Amino und $R^1$ einen Substituenten der Formel VI oder $X^4$, $Y^5$ und $R^1$ Wasserstoff, $X^5$ Hydroxy und $Y^4$ die Gruppe $-NHR^2$ oder $X^4$, $X^5$ und $R^1$ Wasserstoff und $Y^4$ und $Y^5$ die Gruppe $-NHR^2$ bedeuten, $R^2$ einen Substituenten der Formel VI bezeichnet und $Z^1$ in den Substituenten der Formel VI eine Alkyl-, Alkoxy- oder Dialkylamino-Gruppe darstellt;

oder

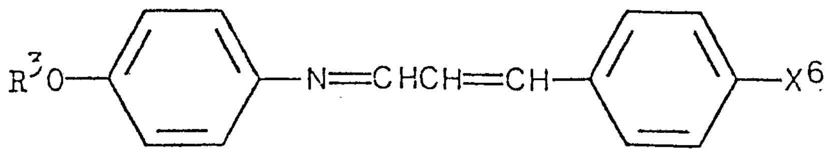

X

worin $R^3$ eine Alkyl- und $X^6$ eine Cyano- oder eine Nitrogruppe bedeutet;

oder

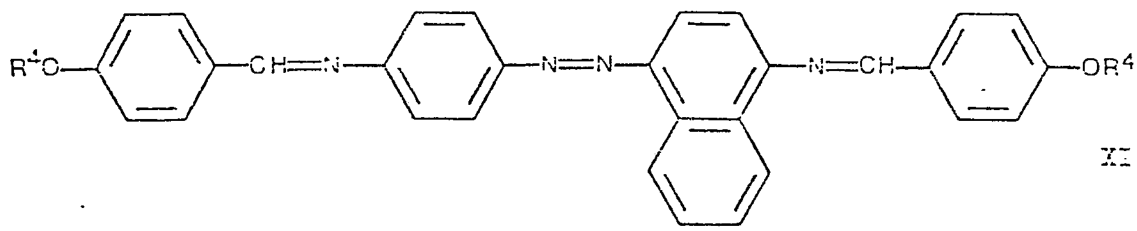

XI

worin $R^4$ eine Alkylgruppe darstellt;

oder

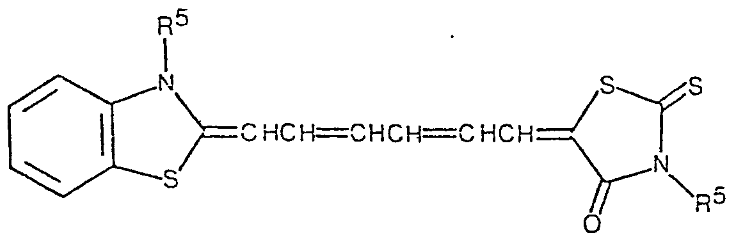

XII

worin $R^5$ eine Alkylgruppe darstellt;

und, gegebenenfalls, weitere nematische und/oder nicht nematische Verbindungen enthalten.

9. Flüssigkristalline Gemische nach Anspruch 8, dadurch gekennzeichnet, daß sie mindestens einen Farbstoff der Formel

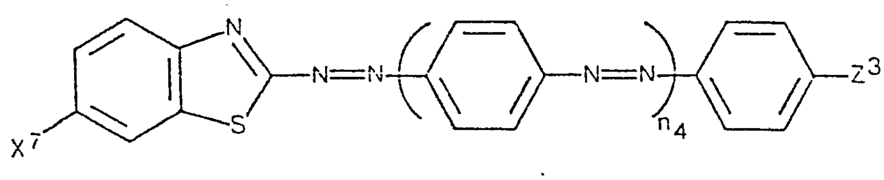

VIIa

worin $n_4$ 0 oder 1 ist, $Z^3$ die Dimethylamino- oder die Diäthylamino-Gruppe bedeutet und $X^7$ Wasserstoff, Methyl, Äthoxy, n-Butyloxy, Nitro oder n-Butylsulfonyl darstellt, enthalten.

10. Flüssigkristalline Gemische nach Anspruch 8, dadurch gekennzeichnet, daß sie mindestens einen Farbstoff der Formel

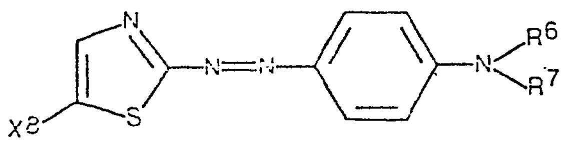

VIIIa

worin $R^6$, $R^7$ und $X^8$ Methyl oder $R^6$ Methyl, $X^8$ Nitro und $R^7$ Methyl, Phenyl oder p-n-Butylsulfonyl bedeuten, enthalten.

11. Flüssigkristalline Gemische nach Anspruch 8, dadurch gekennzeichnet, daß sie mindestens einen Farbstoff der Formel

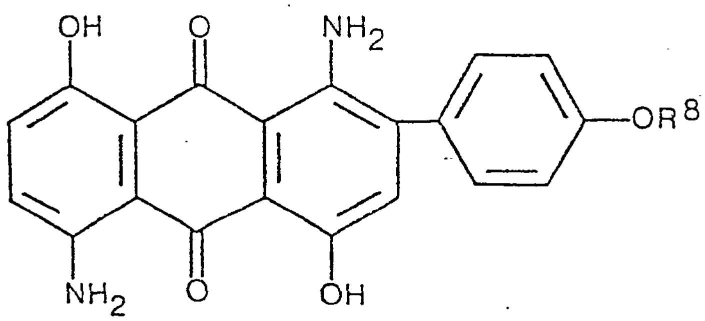

IXa

worin $R^8$ n-Alkyl mit 1 bis 10 Kohlenstoffatomen bedeutet, enthalten.

12. Flüssigkristalline Gemische nach Anspruch 8, dadurch gekennzeichnet, daß sie mindestens einen Farbstoff der Formel

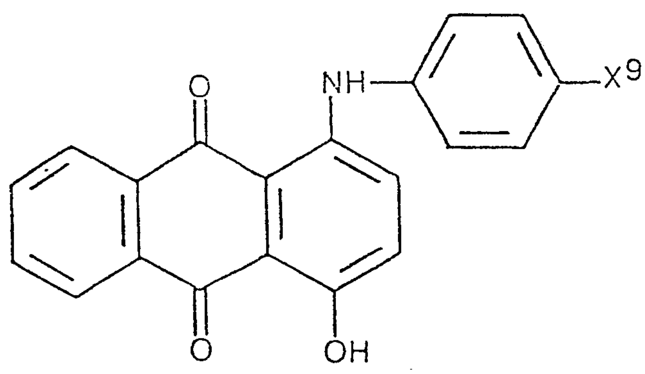

IXb

worin $X^9$ n-Butyl, n-Nonyloxy oder Dimethylamino bedeutet, enthalten.

13. Flüssigkristalline Gemische nach Anspruch 8, dadurch gekennzeichnet, daß sie mindestens einen Farbstoff der Formel

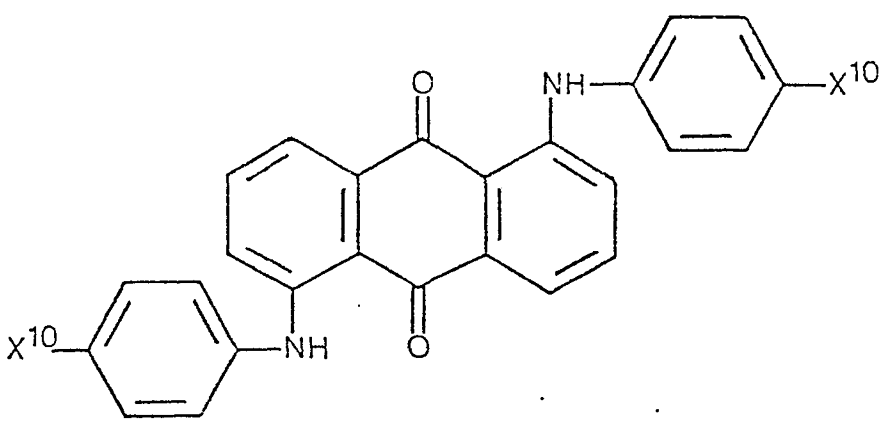

IXc

worin $X^{10}$ Äthyl, n-Butyl, Isopropyl, Pentyloxy oder Dimethylamino bedeutet, enthalten.

14. Flüssigkristalline Gemische nach Anspruch 8, dadurch gekennzeichnet, daß sie den Farbstoff der Formel

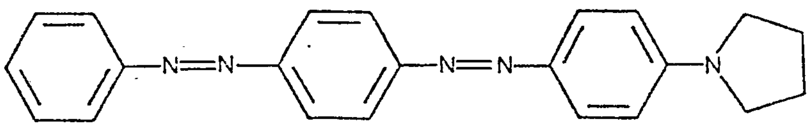

IVa

und/oder den Farbstoff der Formel X, worin $R^3$ n-Butyl und $X^6$ Nitro bedeutet,
und/oder den Farbstoff der Formel XI, worin $R^4$ Äthyl bedeutet,
und/oder den Farbstoff der Formel XII, worin $R^5$ Äthyl bedeutet, enthalten.

15. Flüssigkristallines Gemisch gemäß Anspruch 1, dadurch gekennzeichnet, daß es
4'-Pentyl-4-cyanobiphenyl,
trans-4-Propylcyclohexancarbonsäure-p-cyanophenylester,
trans-4-Pentylcyclohexancarbonsäure-p-cyanophenylester,
trans-4-Pentylcyclohexancarbonsäure-p-(methoxy)phenylester,
trans-p-[5-(4-Äthylcyclohexyl)-2-pyrimidinyl]benzonitril,
trans-p-[5-(4-Pentylcyclohexyl)-2-pyrimidinyl]benzonitril und
trans-p-[5-(4-Heptylcyclohexyl)-2-pyrimidinyl]benzonitril
enthält.

16. Flüssigkristallines Gemisch gemäß Anspruch 1, dadurch gekennzeichnet, daß es
p-Butylbenzoesäure-p'-cyanophenylester,
p-(5-Pentyl-2-pyrimidinyl)benzonitril,
p-(5-Heptyl-2-pyrimidinyl)benzonitril,
trans-4-Butylcyclohexancarbonsäure-p-cyanophenylester,
trans-4-Pentylcyclohexancarbonsäure-p-cyanophenylester,
trans-4-Butylcyclohexancarbonsäure-p-(äthoxy)phenylester,
trans-4-Pentylcyclohexancarbonsäure-p-(methoxy)phenylester und
trans-p-[5-(4-Äthylcyclohexyl)-2-pyrimidinyl]benzonitril
enthält.

17. Flüssigkristallines Gemisch gemäß Anspruch 1, dadurch gekennzeichnet, daß es
p-Butylbenzoesäure-p'-cyanophenylester,
p-(5-Pentyl-2-pyrimidinyl)benzonitril,
p-(5-Heptyl-2-pyrimidinyl)benzonitril,
trans-4-Propylcyclohexancarbonsäure-p-cyanophenylester,
trans-4-Pentylcyclohexancarbonsäure-p-cyanophenylester,
trans-4-Butylcyclohexancarbonsäure-p-(äthoxy)phenylester,
trans-4-Pentylcyclohexancarbonsäure-p-(methoxy)phenylester,
trans-p-[5-(4-Pentylcyclohexyl)-2-pyrimidinyl]benzonitril und
trans-p-[5-(4-Heptylcyclohexyl)-2-pyrimidinyl]benzonitril
enthält.

18. Flüssigkristallines Gemisch gemäß Anspruch 1, dadurch gekennzeichnet, daß es
p-Butylbenzoesäure-p'-cyanophenylester,
p-(5-Pentyl-2-pyrimidinyl)benzonitril,
p-(5-Heptyl-2-pyrimidinyl)benzonitril,
trans-4-Propylcyclohexancarbonsäure-p-cyanophenylester,
trans-4-Pentylcyclohexancarbonsäure-p-cyanophenylester,
trans-4-Butylcyclohexancarbonsäure-p-(äthoxy)phenylester,
trans-4-Pentylcyclohexancarbonsäure-p-(methoxy)phenylester und
trans-p-[5-(4-Äthylcyclohexyl)-2-pyrimidinyl]benzonitril
enthält.

19. Flüssigkristallines Gemisch gemäß Anspruch 1, dadurch gekennzeichnet, daß es
4'-Heptyl-4-cyanobiphenyl,
p-(5-Pentyl-2-pyrimidinyl)benzonitril,
p-(5-Heptyl-2-pyrimidinyl)benzonitril,
trans-4-Propylcyclohexancarbonsäure-p-cyanophenylester,
trans-4-Butylcyclohexancarbonsäure-p-cyanophenylester,
trans-4-Pentylcyclohexancarbonsäure-p-cyanophenylester,
trans-4-Pentylcyclohexancarbonsäure-p-(methoxy)phenylester,
trans-4-Butylcyclohexancarbonsäure-p-(äthoxy)phenylester und
trans-p-[5-(4-Äthylcyclohexyl)-2-pyrimidinyl]benzonitril
enthält.

20. Flüssigkristallines Gemisch gemäß Anspruch 1, dadurch gekennzeichnet, daß es
p-Butylbenzoesäure-p'-cyanophenylester,
p-(5-Pentyl-2-pyrimidinyl)benzonitril,
p-(5-Heptyl-2-pyrimidinyl)benzonitril,
trans-4-Propylcyclohexancarbonsäure-p-cyanophenylester,
trans-4-Butylcyclohexancarbonsäure-p-cyanophenylester,

trans-4-Pentylcyclohexancarbonsäure-p-cyanophenylester,
trans-4-Butylcyclohexancarbonsäure-p-(äthoxy)phenylester,
4″-Pentyl-4-cyano-p-terphenyl und
trans-p-[5-(4-Äthylcyclohexyl)-2-pyrimidinyl]benzonitril
enthält.

21. Flüssigkristallines Gemisch gemäß Anspruch 1, dadurch gekennzeichnet, daß es
4′-Pentyl-4-cyanobiphenyl,
trans-4-Propylcyclohexancarbonsäure-p-cyanophenylester,
trans-4-Pentylcyclohexancarbonsäure-p-cyanophenylester,
trans-4-Butylcyclohexancarbonsäure-p-(äthoxy)phenylester,
trans-4-Pentylcyclohexancarbonsäure-p-(methoxy)phenylester,
trans-p-[5-(4-Äthylcyclohexyl)-2-pyrimidinyl]benzonitril,
trans-p-[5-(4-Pentylcyclohexyl)-2-pyrimidinyl]benzonitril und
trans-p-[5-(4-Heptylcyclohexyl)-2-pyrimidinyl]benzonitril
enthält.

22. Flüssigkristallines Gemisch gemäß Anspruch 1, dadurch gekennzeichnet, daß es
4′-Pentyl-4-cyanobiphenyl,
p-Butylbenzoesäure-p′-cyanophenylester,
p-(5-Pentyl-2-pyrimidinyl)benzonitril,
trans-4-Propylcyclohexancarbonsäure-p-cyanophenylester,
trans-4-Pentylcyclohexancarbonsäure-p-cyanophenylester,
trans-4-Pentylcyclohexancarbonsäure-p-(methoxy)phenylester und
trans-p-[5-(4-Äthylcyclohexyl)-2-pyrimidinyl]benzonitril
enthält.

23. Flüssigkristallines Gemisch gemäß Anspruch 1, dadurch gekennzeichnet, daß es
4′-Heptyl-4-cyanobiphenyl,
p-Butylbenzoesäure-p′-cyanophenylester,
p-(5-Pentyl-2-pyrimidinyl)benzonitril,
p-(5-Heptyl-2-pyrimidinyl)benzonitril,
trans-4-Propylcyclohexancarbonsäure-p-cyanophenylester,
trans-4-Pentylcyclohexancarbonsäure-p-cyanophenylester,
trans-4-Butylcyclohexancarbonsäure-p-(äthoxy)phenylester und
trans-p-[5-(4-Äthylcyclohexyl)-2-pyrimidinyl]benzonitril
enthält.

24. Flüssigkristallines Gemisch gemäß Anspruch 1, dadurch gekennzeichnet, daß es
4′-Pentyl-4-cyanobiphenyl,
4′-Heptyl-4-cyanobiphenyl,
p-(5-Pentyl-2-pyrimidinyl)benzonitril,
p-(5-Heptyl-2-pyrimidinyl)benzonitril,
trans-4-Butylcyclohexancarbonsäure-p-(äthoxy)phenylester und
trans-p-[5-(4-Pentylcyclohexyl)-2-pyrimidinyl]benzonitril
enthält.

25. Flüssigkristallines Gemisch gemäß Anspruch 1, dadurch gekennzeichnet, daß es
4′-Pentyl-4-cyanobiphenyl,
4′-Heptyl-4-cyanobiphenyl,
p-(5-Pentyl-2-pyrimidinyl)benzonitril,
p-(5-Heptyl-2-pyrimidinyl)benzonitril und
trans-p-[5-(4-Äthylcyclohexyl)-2-pyrimidinyl]benzonitril
enthält.

26. Flüssigkristallines Gemisch gemäß Anspruch 1, dadurch gekennzeichnet, daß es
4′-Pentyl-4-cyanobiphenyl,
4′-Heptyl-4-cyanobiphenyl,
p-(5-Pentyl-2-pyrimidinyl)benzonitril,
p-(5-Heptyl-2-pyrimidinyl)benzonitril,
trans-4-Butylcyclohexancarbonsäure-p-(äthoxy)phenylester,
trans-4-Pentylcyclohexancarbonsäure-p-(methoxy)phenylester und
trans-p-[5-(4-Pentylcyclohexyl)-2-pyrimidinyl]benzonitril
enthält.

27. Flüssigkristallines Gemisch gemäß Anspruch 1, dadurch gekennzeichnet, daß es
4′-Pentyl-4-cyanobiphenyl,
4′-Octyloxy-4-cyanobiphenyl,
p-(5-Pentyl-2-pyrimidinyl)benzonitril,
p-(5-Heptyl-2-pyrimidinyl)benzonitril und
trans-p-[5-(4-Pentylcyclohexyl)-2-pyrimidinyl]benzonitril
enthält.

28. Flüssigkristallines Gemisch gemäß Anspruch 1, dadurch gekennzeichnet, daß es
4'-Pentyl-4-cyanobiphenyl,
p-Butylbenzoesäure-p'-cyanophenylester,
p-(5-Pentyl-2-pyrimidinyl)benzonitril,
p-(5-Heptyl-2-pyrimidinyl)benzonitril,
trans-4-Butylcyclohexancarbonsäure-p-cyanophenylester,
trans-4-Pentylcyclohexancarbonsäure-p-cyanophenylester,
trans-4-Butylcyclohexancarbonsäure-p-(äthoxy)phenylester,
trans-4-Pentylcyclohexancarbonsäure-p-(methoxy)phenylester und
trans-p-[5-(4-Äthylcyclohexyl)-2-pyrimidinyl]benzonitril
enthält.

29. Flüssigkristallines Gemisch gemäß Anspruch 1, dadurch gekennzeichnet, daß es
4'-Heptyl-4-cyanobiphenyl,
trans-4-Propylcyclohexancarbonsäure-p-cyanophenylester,
trans-4-Butylcyclohexancarbonsäure-p-(äthoxy)phenylester,
trans-4-Pentylcyclohexancarbonsäure-p-(methoxy)phenylester,
trans-4-Pentylcyclohexancarbonsäure-p-(propyloxy)phenylester und
trans-p-[5-(4-Pentylcyclohexyl)-2-pyrimidinyl]benzonitril
enthält.

30. Verfahren zur Herstellung von Verbindungen der allgemeinen Formel

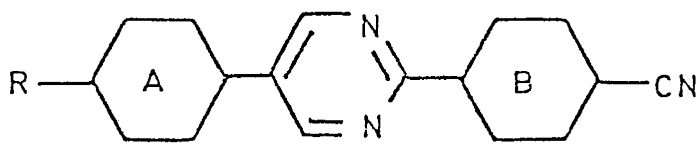

I

worin wenigstens einer der Ringe A und B einen trans-1,4-disubstituierten Cyclohexanring darstellt und der andere gegebenenfalls aromatisch ist und R eine geradkettige Alkyl- oder Alkoxygruppe mit 1 bis 10 Kohlenstoffatomen oder eine verzweigte Alkylgruppe

$C_2H_5-CH(CH_3)-(CH_2)_n-$,

worin n eine ganze Zahl von 1 bis 3 ist, bedeutet, dadurch gekennzeichnet, daß man

a) eine Verbindung der allgemeinen Formel

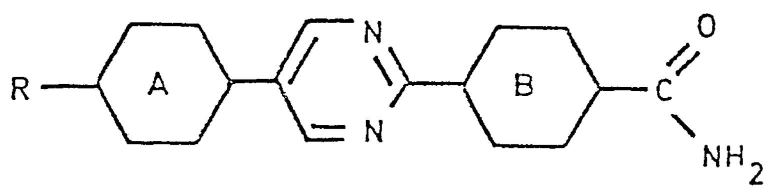

II

worin A, B und R die obige Bedeutung haben, dehydratisiert, oder

b) zur Herstellung einer Verbindung der Formel I, worin der Ring B aromatisch ist, eine Verbindung der allgemeinen Formel

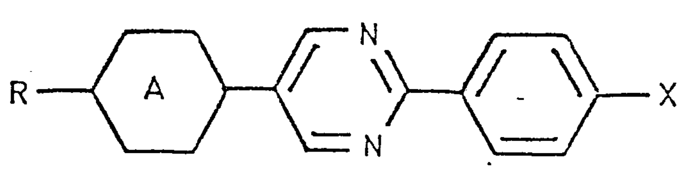

III

worin R und A die obige Bedeutung haben und X Fluor, Chlor oder Brom darstellt, mit Kupfer-(I)-cyanid oder mit Natrium- oder Kaliumcyanid umsetzt.

31. Elektro-optische Vorrichtung, enthaltend eine oder mehrere Verbindungen der in Anspruch 1 definierten Formel I in flüssigkristallinem Zustand.

**Claims for the Contracting States: BE, CH, DE, FR, IT, NL**

1. Compounds of the general formula

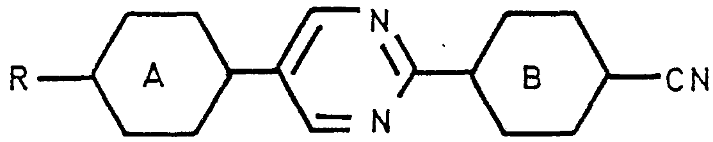

I

wherein at least one of rings A and B represents a trans-1,4-disubstituted cyclohexane ring and the other is optionally aromatic and R signifies a straight-chain alkyl or alkoxy group with 1 to 10 carbon atoms or a branched alkyl group

$C_2H_5-CH(CH_3)-(CH_2)_n-$,

wherein n is a whole number of 1 to 3.

2. Trans-p-[5-(4-alkyl- or 4-n-alkoxycyclohexyl)-2-pyrimidinyl]benzonitriles of the formula

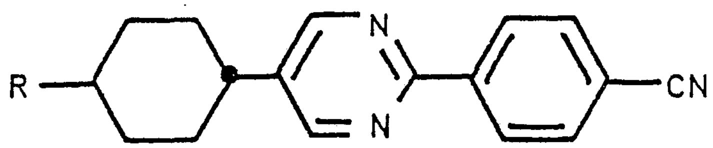

IA

wherein R represents straight-chain alkyl with 2 to 8 carbon atoms, straight-chain alkoxy with up to 6 carbon atoms or a branched alkyl group

$C_2H_5-CH(CH_3)-(CH_2)_n-$,

wherein n is a whole number of 1 to 3.

3. Trans-4-[5-(p-alkyl- or p-n-alkoxyphenyl)-2-pyrimidinyl]cyclohexanecarbonitriles of the formula

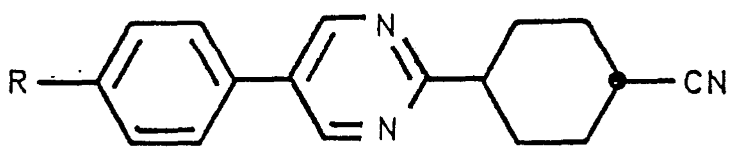

IB

wherein R represents straight-chain alkyl with 2 to 8 carbon atoms, straight-chain alkoxy with up to 6 carbon atoms or a branched alkyl group

$C_2H_5-CH(CH_3)-(CH_2)_n-$,

wherein n is a whole number of 1 to 3.

4. Trans-4-[5-(trans-4-alkyl- or trans-4-n-alkoxycyclohexyl)-2-pyrimidinyl]cyclohexanecarbonitriles of the formula

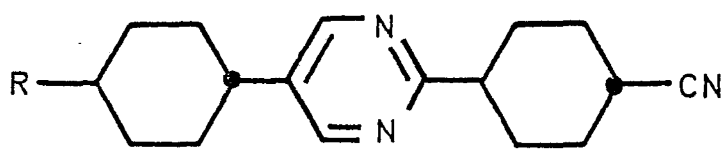

IC

wherein R represents straight-chain alkyl with 2 to 8 carbon atoms, straight-chain alkoxy with up to 6 carbon atoms or a branched alkyl group

$C_2H_5-CH(CH_3)-(CH_2)_n-$,

wherein n is a whole number of 1 to 3.

5. Trans-p-[5-(4-ethylcyclohexyl)-2-pyrimidinyl]benzonitrile.

6. Trans-p-[5-(4-pentylcyclohexyl)-2-pyrimidinyl]benzonitrile.

7. Trans-p-[5-(4-heptylcyclohexyl)-2-pyrimidinyl]benzonitrile.

8. Liquid crystalline mixture, characterized in that it contains one or more compounds of formula I defined in claim 1 and, if desired, other nematic and/or non-nematic compounds and/or one or more

dichroic colouring substances.

9. Liquid crystalline mixture according to claim 8, characterized in that it contains one or more compounds of formula I defined in claim 1 and one or more colouring substances of the general formulae

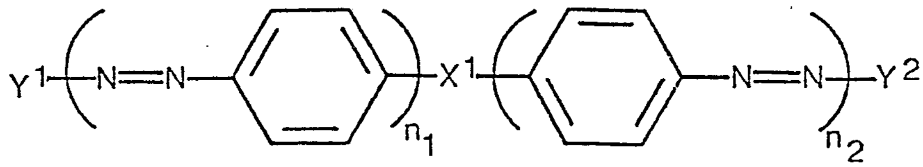

IV

wherein $n_1$ and $n_2$ signify whole numbers of 0 to 2 and $X^1$ signifies an azo or azoxy group, $Y^1$ and $Y^2$ can be the same or different and denote one of the substituents

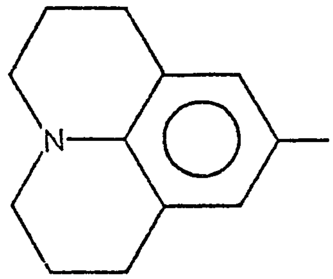

V or

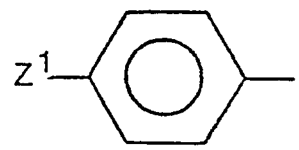

VI

and $Z^1$ represents hydrogen, cyano, nitro, phenyl, p-substituted phenyl, hydroxy, alkoxy, amino, dialkylamino or pyrrolidyl; or derivatives of formula IV in which one of the benzene rings additionally carries one or more of the groups halogen, methyl, halo-substituted methyl or methoxy;

or

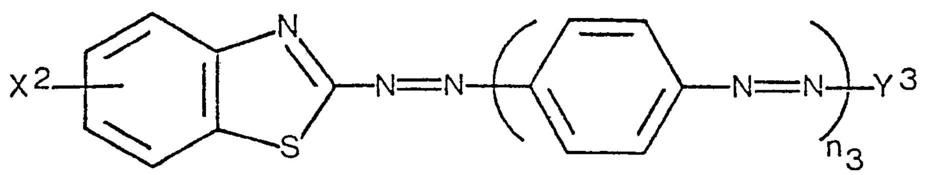

VII

wherein $n_3$ is 0 or 1, $Y^3$ denotes a substituent of formula V or VI, wherein $Z^1$ signifies dialkylamino or pyrrolidyl, and $X^2$ represents hydrogen, cyano, nitro, halogen, alkyl, alkoxy, an optionally substituted phenyl or benzothiazol-2-yl group, alkoxycarbonyl or alkylsulphonyl in the 5- or 6-position;

or

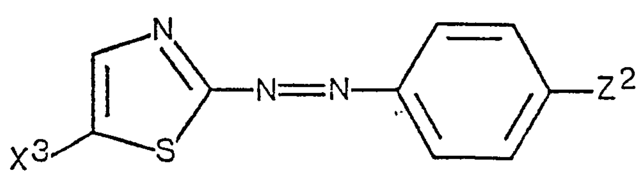

VIII

wherein $X^3$ signifies cyano, nitro or alkyl, and $Z^2$ represents hydroxy, alkoxy, aryl or an optionally alkyl- or aryl-substituted amino, pyrrolidyl or piperdyl group; or derivatives of formula VIII in which the benzene ring additionally carries one or more of the groups halogen, hydroxy, methyl or methoxy;

or

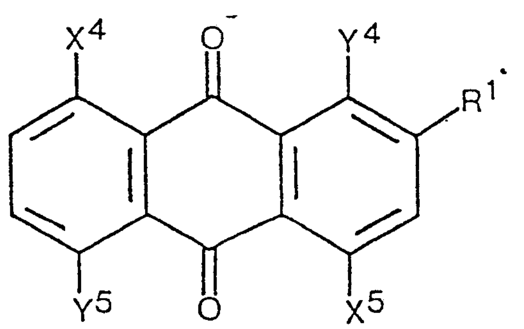

IX

wherein $X^4$ and $X^5$ signify hydroxy, $Y^4$ and $Y^5$ signify amino and $R^1$ signifies a substituent of formula VI or $X^4$, $Y^5$ and $R^1$ signify hydrogen, $X^5$ signifies hydroxy and $Y^4$ signifies the group $-NHR^2$ or $X^4$, $X^5$ and $R^1$ signify hydrogen and $Y^4$ and $Y^5$ signify the group $-NHR^2$, $R^2$ denotes a substituent of formula VI and $Z^1$ in the substituent of formula VI represents an alkyl, alkoxy or dialkylamino group;

or

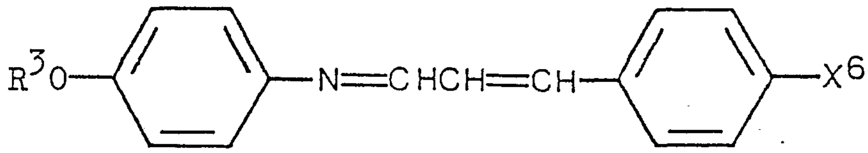

X

wherein $R^3$ signifies an alkyl group and $X^6$ signifies a cyano group or a nitro group;

or

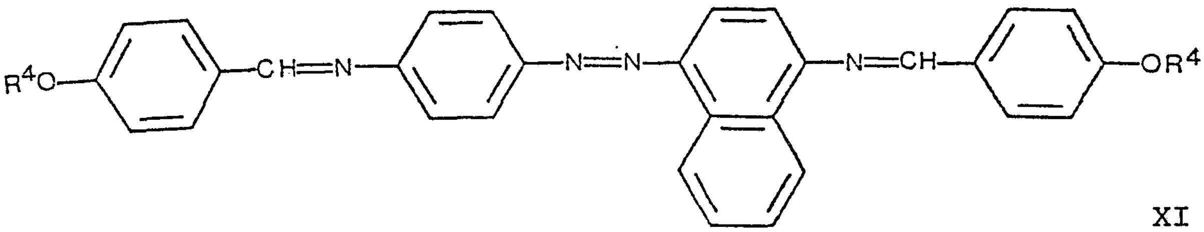

XI

wherein $R^4$ represents an alkyl group;

or

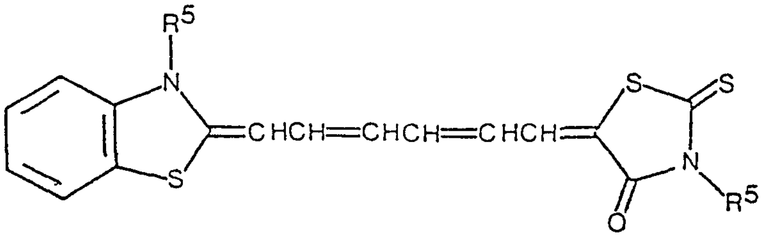

XII

wherein $R^5$ represents an alkyl group;

and, if desired, other nematic and/or non-nematic compounds.

10. Liquid crystalline mixture according to claim 9, characterized in that it contains at least one colouring substance of the formula

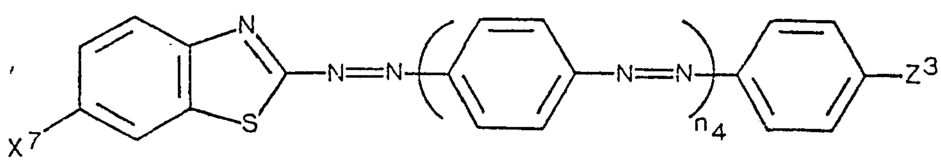

VIIa

wherein $n_4$ is 0 or 1, $Z^3$ signifies the dimethylamino group or the diethylamino group and $X^7$ represents hydrogen, methyl, ethoxy, n-butyloxy, nitro or n-butylsulphonyl.

11. Liquid crystalline mixture according to claim 9, characterized in that it contains at least one colouring substance of the formula

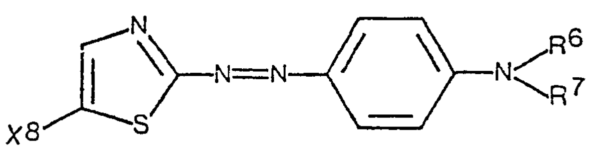

VIIIa

wherein $R^6$, $R^7$ and $X^8$ signify methyl or $R^6$ signifies methyl, $X^8$ signifies nitro and $R^7$ signifies methyl, phenyl or p-n-butylsulphonyl.

12. Liquid crystalline mixture according to claim 9, characterized in that it contains at least one colouring substance of the formula

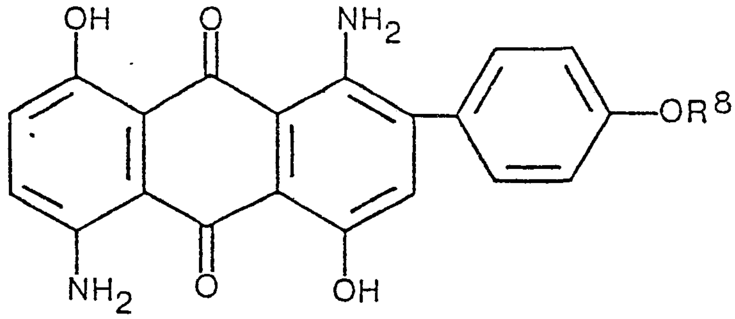

IXa

wherein $R^8$ signifies n-alkyl with 1 to 10 carbon atoms.

13. Liquid crystalline mixture according to claim 9, characterized in that it contains at least one colouring substance of the formula

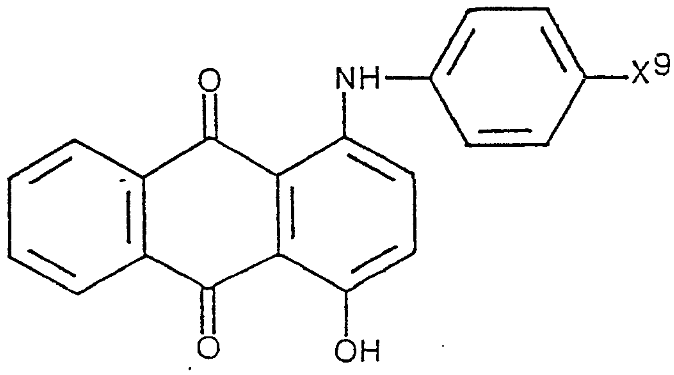

IXb

wherein $X^9$ signifies n-butyl, n-nonyloxy or dimethylamino.

14. Liquid crystalline mixture according to claim 9, characterized in that it contains at least one colouring substance of the formula

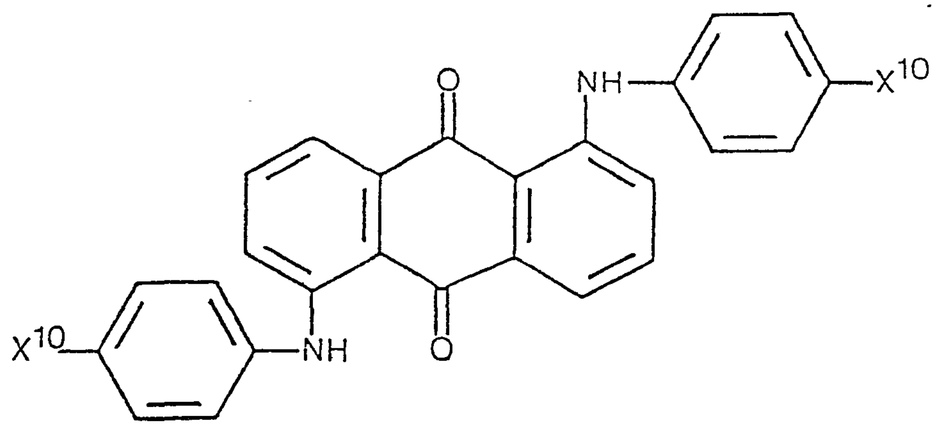

IXc

wherein $X^{10}$ signifies ethyl, n-butyl, isopropyl, pentyloxy or dimethylamino.

15. Liquid crystalline mixture according to claim 9, characterized in that it contains the colouring substance of the formula

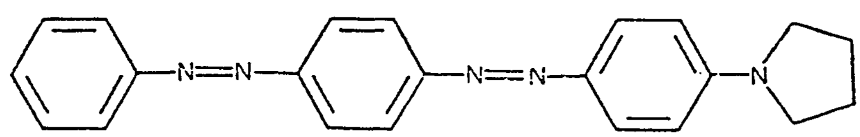

IVa

and/or the colouring substance of formula X wherein $R^3$ signifies n-butyl and $X^6$ signifies nitro,
and/or the colouring substance of formula XI wherein $R^4$ signifies ethyl,
and/or the colouring substance of formula XII wherein $R^5$ signifies ethyl.

16. Liquid crystalline mixture in accordance with claim 8, characterized in that it contains
4'-pentyl-4-cyanobiphenyl,
trans-4-propylcyclohexanecarboxylic acid p-cyanophenyl ester,
trans-4-pentylcyclohexanecarboxylic acid p-cyanophenyl ester,
trans-4-pentylcyclohexanecarboxylic acid p-(methoxy)phenyl ester,

trans-p-[5-(4-ethylcyclohexyl)-2-pyrimidinyl]benzonitrile,
trans-p-[5-(4-pentylcyclohexyl)-2-pyrimidinyl]benzonitrile and
trans-p-[5-(4-heptylcyclohexyl)-2-pyrimidinyl]benzonitrile.

17. Liquid crystalline mixture in accordance with claim 8, characterized in that it contains
p-butylbenzoic acid p'-cyanophenyl ester,
p-(5-pentyl-2-pyrimidinyl)benzonitrile,
p-(5-heptyl-2-pyrimidinyl)benzonitrile,
trans-4-butylcyclohexanecarboxylic acid p-cyanophenyl ester,
trans-4-pentylcyclohexanecarboxylic acid p-cyanophenyl ester,
trans-4-butylcyclohexanecarboxylic acid p-(ethoxy)phenyl ester,
trans-4-pentylcyclohexanecarboxylic acid p-(methoxy)phenyl ester and
trans-p-[5-(4-ethylcyclohexyl)-2-pyrimidinyl]benzonitrile.

18. Liquid crystalline mixture in accordance with claim 8, characterized in that it contains
p-butylbenzoic acid p'-cyanophenyl ester,
p-(5-pentyl-2-pyrimidinyl)benzonitrile,
p-(5-heptyl-2-pyrimidinyl)benzonitrile,
trans-4-propylcyclohexanecarboxylic acid p-cyanophenyl ester,
trans-4-pentylcyclohexanecarboxylic acid p-cyanophenyl ester,
trans-4-butylcyclohexanecarboxylic acid p-(ethoxy)phenyl ester,
trans-4-pentylcyclohexanecarboxylic acid p-(methoxy)phenyl ester,
trans-p-[5-(4-pentylcyclohexyl)-2-pyrimidinyl]benzonitrile and
trans-p-[5-(4-heptylcyclohexyl)-2-pyrimidinyl]benzonitrile.

19. Liquid crystalline mixture in accordance with claim 8, characterized in that it contains
p-butylbenzoic acid p'-cyanophenyl ester,
p-(5-pentyl-2-pyrimidinyl)benzonitrile,
p-(5-heptyl-2-pyrimidinyl)benzonitrile,
trans-4-propylcyclohexanecarboxylic acid p-cyanophenyl ester,
trans-4-pentylcyclohexanecarboxylic acid p-cyanophenyl ester,
trans-4-butylcyclohexanecarboxylic acid p-(ethoxy)phenyl ester,
trans-4-pentylcyclohexanecarboxylic acid p-(methoxy)phenyl ester and
trans-p-[5-(4-ethylcyclohexyl)-2-pyrimidinyl]benzonitrile.

20. Liquid crystalline mixture in accordance with claim 8, characterized in that it contains
4'-heptyl-4-cyanobiphenyl,
p-(5-pentyl-2-pyrimidinyl)benzonitrile,
p-(5-heptyl-2-pyrimidinyl)benzonitrile,
trans-4-propylcyclohexanecarboxylic acid p-cyanophenyl ester,
trans-4-butylcyclohexanecarboxylic acid p-cyanophenyl ester,
trans-4-pentylcyclohexanecarboxylic acid p-cyanophenyl ester,
trans-4-pentylcyclohexanecarboxylic acid p-(methoxy)phenyl ester,
trans-4-butylcyclohexanecarboxylic acid p-(ethoxy)phenyl ester and
trans-p-[5-(4-ethylcyclohexyl)-2-pyrimidinyl]benzonitrile.

21. Liquid crystalline mixture in accordance with claim 8, characterized in that it contains
p-butylbenzoic acid p'-cyanophenyl ester,
p-(5-pentyl-2-pyrimidinyl)benzonitrile,
p-(5-heptyl-2-pyrimidinyl)benzonitrile,
trans-4-propylcyclohexanecarboxylic acid p-cyanophenyl ester,
trans-4-butylcyclohexanecarboxylic acid p-cyanophenyl ester,
trans-4-pentylcyclohexanecarboxylic acid p-cyanophenyl ester,
trans-4-butylcyclohexanecarboxylic acid p-(ethoxy)phenyl ester,
4''-pentyl-4-cyano-p-terphenyl and
trans-p-[5-(4-ethylcyclohexyl)-2-pyrimidinyl]benzonitrile.

22. Liquid crystalline mixture in accordance with claim 8, characterized in that it contains
4'-pentyl-4-cyanobiphenyl,
trans-4-propylcyclohexanecarboxylic acid p-cyanophenyl ester,
trans-4-pentylcyclohexanecarboxylic acid p-cyanophenyl ester,
trans-4-butylcyclohexanecarboxylic acid p-(ethoxy)phenyl ester,
trans-4-pentylcyclohexanecarboxylic acid p-(methoxy)phenyl ester,
trans-p-[5-(4-ethylcyclohexyl)-2-pyrimidinyl]benzonitrile,
trans-p-[5-(4-pentylcyclohexyl)-2-pyrimidinyl]benzonitrile and
trans-p-[5-(4-heptylcyclohexyl)-2-pyrimidinyl]benzonitrile.

23. Liquid crystalline mixture in accordance with claim 8, characterized in that it contains
4'-pentyl-4-cyanobiphenyl,
p-butylbenzoic acid p'-cyanophenyl ester,
p-(5-pentyl-2-pyrimidinyl)benzonitrile,
trans-4-propylcyclohexanecarboxylic acid p-cyanophenyl ester,

trans-4-pentylcyclohexanecarboxylic acid p-cyanophenyl ester,
trans-4-pentylcyclohexanecarboxylic acid p-(methoxy)phenyl ester and
trans-p-[5-(4-ethylcyclohexyl)-2-pyrimidinyl]benzonitrile.

24. Liquid crystalline mixture in accordance with claim 8, characterized in that it contains
4′-heptyl-4-cyanobiphenyl,
p-butylbenzoic acid p′-cyanophenyl ester,
p-(5-pentyl-2-pyrimidinyl)benzonitrile,
p-(5-heptyl-2-pyrimidinyl)benzonitrile,
trans-4-propylcyclohexanecarboxylic acid p-cyanophenyl ester,
trans-4-pentylcyclohexanecarboxylic acid p-cyanophenyl ester,
trans-4-butylcyclohexanecarboxylic acid p-(ethoxy)phenyl ester and
trans-p-[5-(4-ethylcyclohexyl)-2-pyrimidinyl]benzonitrile.

25. Liquid crystalline mixture in accordance with claim 8, characterized in that it contains
4′-pentyl-4-cyanobiphenyl,
4′-heptyl-4-cyanobiphenyl,
p-(5-pentyl-2-pyrimidinyl)benzonitrile,
p-(5-heptyl-2-pyrimidinyl)benzonitrile,
trans-4-butylcyclohexanecarboxylic acid p-(ethoxy)phenyl ester and
trans-p-[5-(4-pentylcyclohexyl)-2-pyrimidinyl]benzonitrile.

26. Liquid crystalline mixture in accordance with claim 8, characterized in that it contains
4′-pentyl-4-cyanobiphenyl,
4′-heptyl-4-cyanobiphenyl,
p-(5-pentyl-2-pyrimidinyl)benzonitrile,
p-(5-heptyl-2-pyrimidinyl)benzonitrile and
trans-p-[5-(4-ethylcyclohexyl)-2-pyrimidinyl]benzonitrile.

27. Liquid crystalline mixture in accordance with claim 8, characterized in that it contains
4′-pentyl-4-cyanobiphenyl,
4′-heptyl-4-cyanobiphenyl,
p-(5-pentyl-2-pyrimidinyl)benzonitrile,
p-(5-heptyl-2-pyrimidinyl)benzonitrile,
trans-4-butylcyclohexanecarboxylic acid p-(ethoxy)phenyl ester,
trans-4-pentylcyclohexanecarboxylic acid p-(methoxy)phenyl ester and
trans-p-[5-(4-pentylcyclohexyl)-2-pyrimidinyl]benzonitrile.

28. Liquid crystalline mixture in accordance with claim 8, characterized in that it contains
4′-pentyl-4-cyanobiphenyl,
4′-octyloxy-4-cyanobiphenyl,
p-(5-pentyl-2-pyrimidinyl)benzonitrile,
p-(5-heptyl-2-pyrimidinyl)benzonitrile and
trans-p-[5-(4-pentylcyclohexyl)-2-pyrimidinyl]benzonitrile.

29. Liquid crystalline mixture in accordance with claim 8, characterized in that it contains
4′-pentyl-4-cyanobiphenyl,
p-butylbenzoic acid p′-cyanophenyl ester,
p-(5-pentyl-2-pyrimidinyl)benzonitrile,
p-(5-heptyl-2-pyrimidinyl)benzonitrile,
trans-4-butylcyclohexanecarboxylic acid p-cyanophenyl ester,
trans-4-pentylcyclohexanecarboxylic acid p-cyanophenyl ester,
trans-4-butylcyclohexanecarboxylic acid p-(ethoxy)phenyl ester,
trans-4-pentylcyclohexanecarboxylic acid p-(methoxy)phenyl ester and
trans-p-[5-(4-ethylcyclohexyl)-2-pyrimidinyl]benzonitrile.

30. Liquid crystalline mixture in accordance with claim 8, characterized in that it contains
4′-heptyl-4-cyanobiphenyl,
trans-4-propylcyclohexanecarboxylic acid p-cyanophenyl ester,
trans-4-butylcyclohexanecarboxylic acid p-(ethoxy)phenyl ester,
trans-4-pentylcyclohexanecarboxylic acid p-(methoxy)phenyl ester,
trans-4-pentylcyclohexanecarboxylic acid p-(propyloxy)phenyl ester and
trans-p-[5-(4-pentylcyclohexyl)-2-pyrimidinyl]benzonitrile.

31. Process for the manufacture of compounds of the general formula

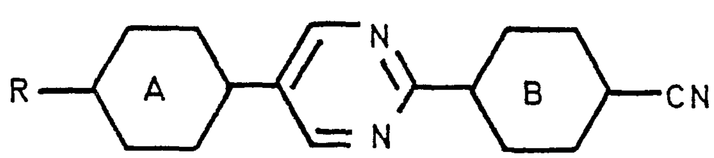 I

wherein at least one of the rings A and B represents a trans-1,4-disubstituted cyclohexane ring and the

other is optionally aromatic and R signifies a straight-chain alkyl or alkoxy group with 1 to 10 carbon atoms or a branched alkyl group

$C_2H_5-CH(CH_3)-(CH_2)_n-$,

wherein n is a whole number of 1 to 3, characterized by

a) dehydrating a compound of the general formula

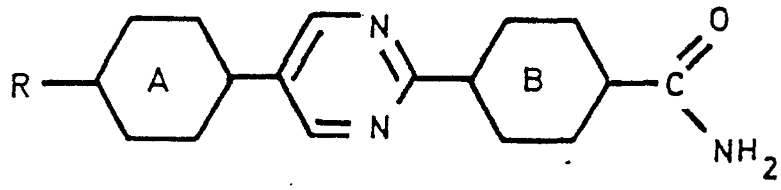

II

wherein A, B and R have the above significance,

or

b) for the manufacture of a compound of formula I wherein the ring B is aromatic, reacting a compound of the general formula

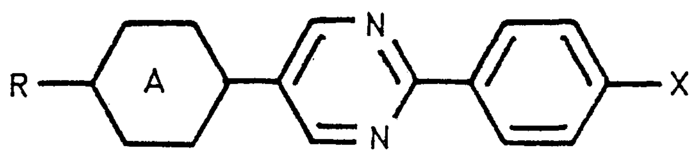

III

wherein R and A have the above significance and X represents fluorine, chlorine or bromine, with copper (I) cyanide or with sodium cyanide or potassium cyanide.

32. Process for the manufacture of liquid crystalline mixtures, characterized by mixing one or more compounds of formula I defined in claim 1 with other nematic and/or non-nematic compounds and/or one or more dichroic colouring substances.

33. Process for the manufacture of an electro-optical device, characterized by introducing one or more compounds of formula I defined in claim 1, if desired in admixture with other nematic and/or non-nematic compounds and/or one or more dichroic colouring substances, into a suitable cell.

34. Use of the compounds of formula I defined in claim 1 for electro-optical purposes.

35. Electro-optical device containing one or more compounds of formula I defined in claim 1 in liquid crystalline condition.

**Claims for the Contracting States: Austria**

1. Liquid crystalline mixture, characterized in that it contains one or more compounds of the general formula

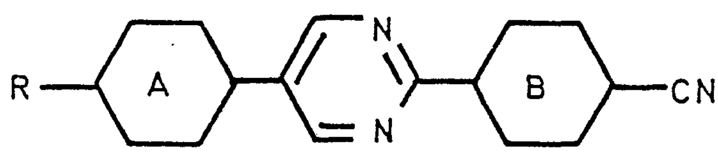

I

wherein at least one of the rings A and B represents a trans-1,4-disubstituted cyclohexane ring and the other is optionally aromatic and R signifies a straight-chain alkyl or alkoxy group with 1 to 10 carbon atoms or a branched alkyl group

$C_2H_5-CH(CH_3)-(CH_2)_n-$,

wherein n is a whole number of 1 to 3,
and, if desired, other nematic and/or non-nematic compounds and/or one or more dichroic colouring substances.

2. Liquid crystalline mixture in accordance with claim 1, characterized in that it contains one or more compounds of the general formula

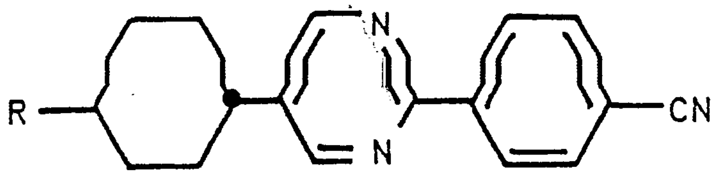

IA

wherein R represents straight-chain alkyl with 2 to 8 carbon atoms, straight-chain alkoxy with up to 6 carbon atoms or a branched alkyl group

$C_2H_5-CH(CH_3)-(CH_2)_n-$,

wherein n is a whole number of 1 to 3.

3. Liquid crystalline mixture in accordance with claim 1, characterized in that it contains one or more compounds of the general formula

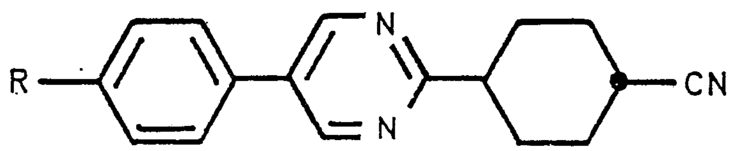

IB

wherein R represents straight-chain alkyl with 2 to 8 carbon atoms, straight-chain alkoxy with up to 6 carbon atoms or a branched alkyl group

$C_2H_5-CH(CH_3)-(CH_2)_n-$,

wherein n is a whole number of 1 to 3.

4. Liquid crystalline mixture in accordance with claim 1, characterized in that it contains one or more compounds of the general formula

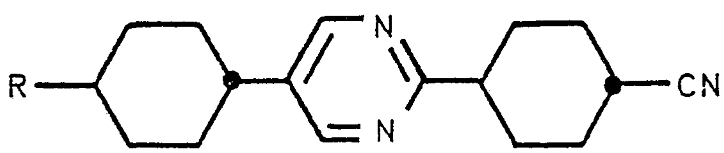

IC

wherein R represents straight-chain alkyl with 2 to 8 carbon atoms, straight-chain alkoxy with up to 6 carbon atoms or a branched alkyl group

$C_2H_5-CH(CH_3)-(CH_2)_n-$,

wherein n is a whole number of 1 to 3.

5. Liquid crystalline mixture in accordance with claim 1 or 2, characterized in that it contains trans-p-[5-(4-ethylcyclohexyl)-2-pyrimidinyl]benzonitrile.

6. Liquid crystalline mixture in accordance with claim 1 or 2, characterized in that it contains trans-p-[5-(4-pentylcyclohexyl)-2-pyrimidinyl]benzonitrile.

7. Liquid crystalline mixture in accordance with claim 1 or 2, characterized in that it contains trans-p-[5-(4-heptylcyclohexyl)-2-pyrimidinyl]benzonitrile.

8. Liquid crystalline mixture in accordance with any one of claims 1 to 7, characterized in that it contains one or more compounds of formula I and one or more dichroic colouring substances of the general formulae

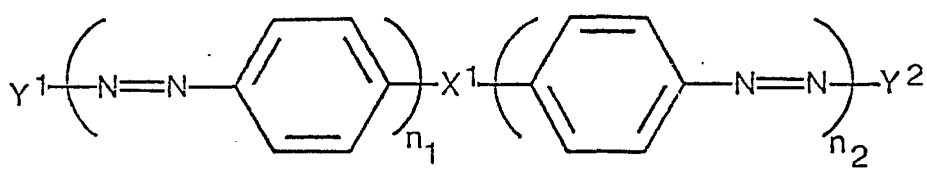

IV

wherein $n_1$ and $n_2$ signify whole numbers of 0 to 2 and $X^1$ signifies an azo or azoxy group, $Y^1$ and $Y^2$ can be the same or different and denote one of the substituents

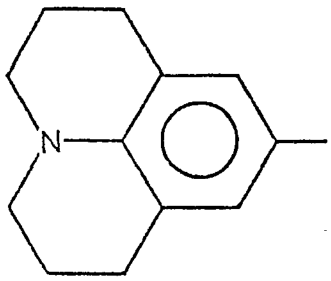 V. or 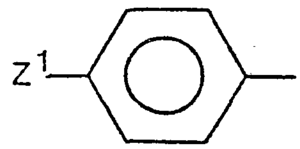 VI

and $Z^1$ represents hydrogen, cyano, nitro, phenyl, p-substituted phenyl, hydroxy, alkoxy, amino, dialkylamino or pyrrolidyl; or derivatives of formula IV in which one of the benzene rings additionally carries one or more of the groups halogen, methyl, halo-substituted methyl or methoxy;

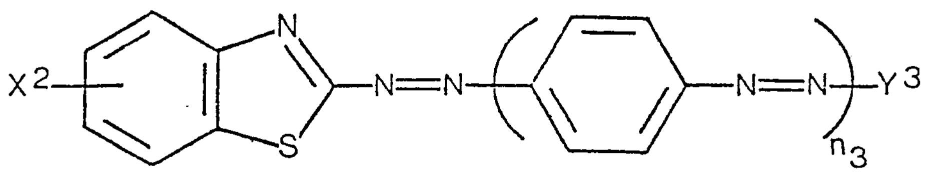 VII

wherein $n_3$ is 0 or 1, $Y^3$ denotes a substituent of formula V or VI, wherein $Z^1$ signifies dialkylamino or pyrrolidyl, and $X^2$ represents hydrogen, cyano, nitro, halogen, alkyl, alkoxy, an optionally substituted phenyl or benzothiazol-2-yl group, alkoxycarbonyl or alkylsulphonyl in the 5- or 6-position;

or

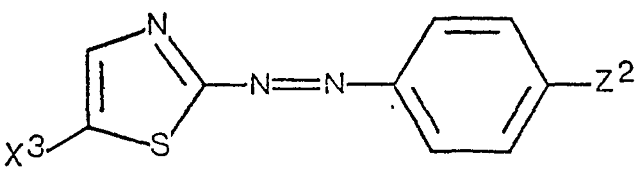 VIII

wherein $X^3$ signifies cyano, nitro or alkyl, and $Z^2$ represents hydroxy, alkoxy, aryl or an optionally alkyl- or aryl-substituted amino, pyrrolidyl or piperidyl group; or derivatives of formula VIII in which the benzene ring additionally carries one or more of the groups halogen, hydroxy, methyl or methoxy;

or

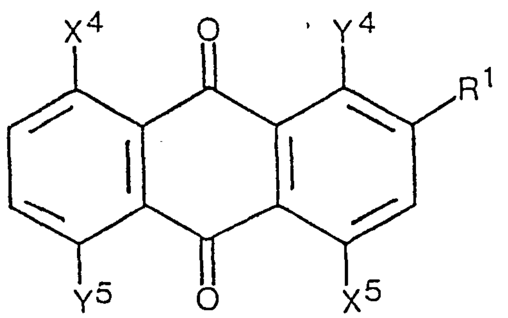 IX

wherein $X^4$ and $X^5$ signify hydroxy, $Y^4$ and $Y^5$ signify amino and $R^1$ signifies a substituent of formula VI or $X^4$, $Y^5$ and $R^1$ signify hydrogen, $X^5$ signifies hydroxy and $Y^4$ signifies the group $-NHR^2$ or $X^4$, $X^5$ and $R^1$ signify hydrogen and $Y^4$ and $Y^5$ signify the group $-NHR^2$, $R^2$ denotes a substituent of formula VI and $Z^1$ in the substituent of formula VI represents an alkyl, alkoxy or dialkylamino group;

or

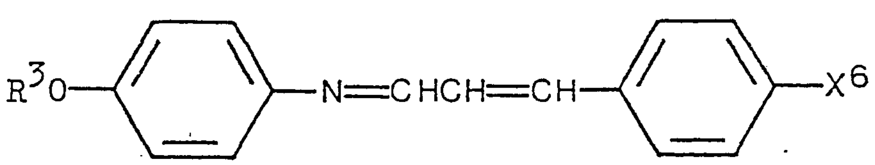 X

wherein $R^3$ signifies an alkyl group and $X^6$ signifies a cyano group or a nitro group;

or

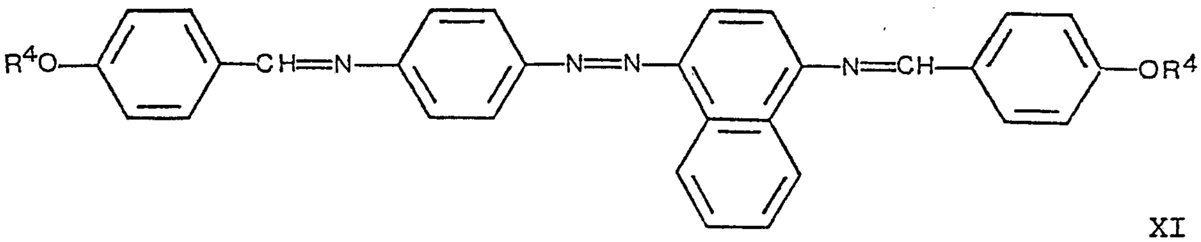

XI

wherein $R^4$ represents an alkyl group;

or

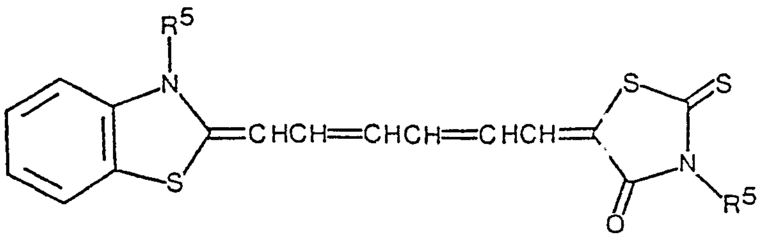

XII

wherein $R^5$ represents an alkyl group;

and, if desired, other nematic and/or non-nematic compounds.

9. Liquid crystalline mixture according to claim 8, characterized in that it contains at least one colouring substance of the formula

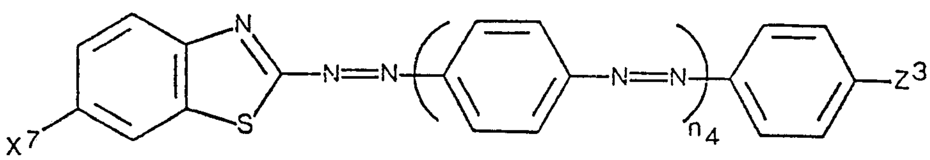

VIIa

wherein $n_4$ is 0 or 1, $Z^3$ signifies the dimethylamino group or the diethylamino group and $X^7$ represents hydrogen, methyl, ethoxy, n-butyloxy, nitro or n-butylsulphonyl.

10. Liquid crystalline mixture according to claim 8, characterized in that it contains at least one colouring substance of the formula

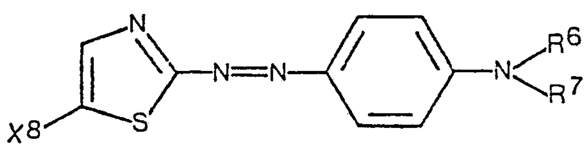

VIIIa

wherein $R^6$, $R^7$ and $X^8$ signify methyl or $R^6$ signifies methyl, $X^8$ signifies nitro and $R^7$ signifies methyl, phenyl or p-n-butylsulphonyl.

11. Liquid crystalline mixture according to claim 8, characterized in that it contains at least one colouring substance of the formula

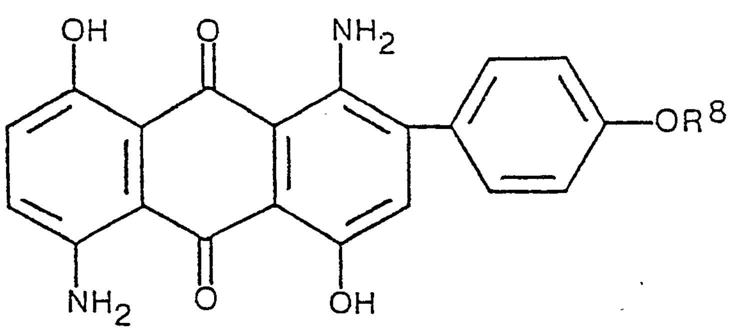

IXa

wherein $R^8$ signifies n-alkyl with 1 to 10 carbon atoms.

12. Liquid crystalline mixture according to claim 8, characterized in that it contains at least one colouring substance of the formula

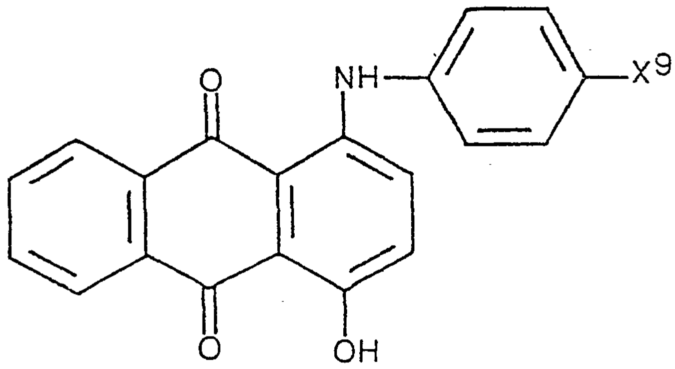

IXb

wherein $X^9$ signifies n-butyl, n-nonyloxy or dimethylamino.

13. Liquid crystalline mixture according to claim 8, characterized in that it contains at least one colouring substance of the formula

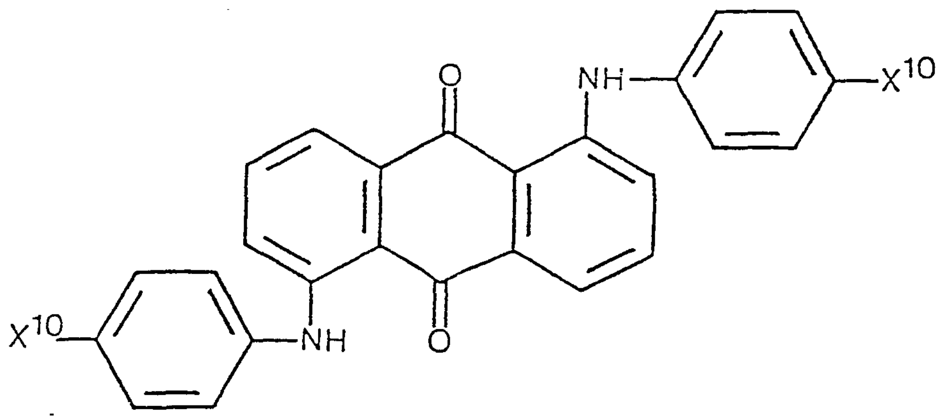

IXc

wherein $X^{10}$ signifies ethyl, n-butyl, isopropyl, pentyloxy or dimethylamino.

14. Liquid crystalline mixture according to claim 8, characterized in that it contains the colouring substance of the formula

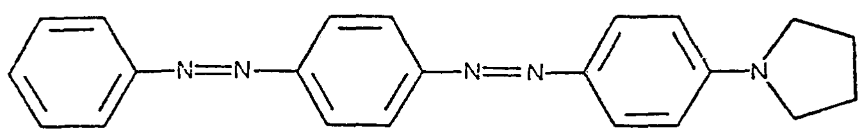

IVa

and/or the colouring substance of formula X wherein $R^3$ signifies n-butyl and $X^6$ signifies nitro,
and/or the colouring substance of formula XI wherein $R^4$ signifies ethyl,
and/or the colouring substance of formula XII wherein $R^5$ signifies ethyl.

15. Liquid crystalline mixture in accordance with claim 1, characterized in that it contains
4′-pentyl-4-cyanobiphenyl,
trans-4-propylcyclohexanecarboxylic acid p-cyanophenyl ester,
trans-4-pentylcyclohexanecarboxylic acid p-cyanophenyl ester,
trans-4-pentylcyclohexanecarboxylic acid p-(methoxy)phenyl ester,
trans-p-[5-(4-ethylcyclohexyl)-2-pyrimidinyl]benzonitrile,
trans-p-[5-(4-pentylcyclohexyl)-2-pyrimidinyl]benzonitrile and
trans-p-[5-(4-heptylcyclohexyl)-2-pyrimidinyl]benzonitrile.

16. Liquid crystalline mixture in accordance with claim 1, characterized in that it contains
p-butylbenzoic acid p′-cyanophenyl ester,
p-(5-pentyl-2-pyrimidinyl)benzonitrile,
p-(5-heptyl-2-pyrimidinyl)benzonitrile,
trans-4-butylcyclohexanecarboxylic acid p-cyanophenyl ester,
trans-4-pentylcyclohexanecarboxylic acid p-cyanophenyl ester,
trans-4-butylcyclohexanecarboxylic acid p-(ethoxy)phenyl ester,
trans-4-pentylcyclohexanecarboxylic acid p-(methoxy)phenyl ester and
trans-p-[5-(4-ethylcyclohexyl)-2-pyrimidinyl]benzonitrile.

17. Liquid crystalline mixture in accordance with claim 1, characterized in that it contains
p-butylbenzoic acid p'-cyanophenyl ester,
p-(5-pentyl-2-pyrimidinyl)benzonitrile,
p-(5-heptyl-2-pyrimidinyl)benzonitrile,
trans-4-propylcyclohexanecarboxylic acid p-cyanophenyl ester,
trans-4-pentylcyclohexanecarboxylic acid p-cyanophenyl ester,
trans-4-butylcyclohexanecarboxylic acid p-(ethoxy)phenyl ester,
trans-4-pentylcyclohexanecarboxylic acid p-(methoxy)phenyl ester,
trans-p-[5-(4-pentylcyclohexyl)-2-pyrimidinyl]benzonitrile and
trans-p-[5-(4-heptylcyclohexyl)-2-pyrimidinyl]benzonitrile.

18. Liquid crystalline mixture in accordance with claim 1, characterized in that it contains
p-butylbenzoic acid p'-cyanophenyl ester,
p-(5-pentyl-2-pyrimidinyl)benzonitrile,
p-(5-heptyl-2-pyrimidinyl)benzonitrile,
trans-4-propylcyclohexanecarboxylic acid p-cyanophenyl ester,
trans-4-pentylcyclohexanecarboxylic acid p-cyanophenyl ester,
trans-4-butylcyclohexanecarboxylic acid p-(ethoxy)phenyl ester,
trans-4-pentylcyclohexanecarboxylic acid p-(methoxy)phenyl ester and
trans-p-[5-(4-ethylcyclohexyl)-2-pyrimidinyl]benzonitrile.

19. Liquid crystalline mixture in accordance with claim 1, characterized in that it contains
4'-heptyl-4-cyanobiphenyl,
p-(5-pentyl-2-pyrimidinyl)benzonitrile,
p-(5-heptyl-2-pyrimidinyl)benzonitrile,
trans-4-propylcyclohexanecarboxylic acid p-cyanophenyl ester,
trans-4-butylcyclohexanecarboxylic acid p-cyanophenyl ester,
trans-4-pentylcyclohexanecarboxylic acid p-cyanophenyl ester,
trans-4-pentylcyclohexanecarboxylic acid p-(methoxy)phenyl ester,
trans-4-butylcyclohexanecarboxylic acid p-(ethoxy)phenyl ester and
trans-p-[5-(4-ethylcyclohexyl)-2-pyrimidinyl]benzonitrile.

20. Liquid crystalline mixture in accordance with claim 1, characterized in that it contains
p-butylbenzoic acid p'-cyanophenyl ester,
p-(5-pentyl-2-pyrimidinyl)benzonitrile,
p-(5-heptyl-2-pyrimidinyl)benzonitrile,
trans-4-propylcyclohexanecarboxylic acid p-cyanophenyl ester,
trans-4-butylcyclohexanecarboxylic acid p-cyanophenyl ester,
trans-4-pentylcyclohexanecarboxylic acid p-cyanophenyl ester,
trans-4-butylcyclohexanecarboxylic acid p-(ethoxy)phenyl ester,
4''-pentyl-4-cyano-p-terphenyl and
trans-p-[5-(4-ethylcyclohexyl)-2-pyrimidinyl]benzonitrile.

21. Liquid crystalline mixture in accordance with claim 1, characterized in that it contains
4'-pentyl-4-cyanobiphenyl,
trans-4-propylcyclohexanecarboxylic acid p-cyanophenyl ester,
trans-4-pentylcyclohexanecarboxylic acid p-cyanophenyl ester,
trans-4-butylcyclohexanecarboxylic acid p-(ethoxy)phenyl ester,
trans-4-pentylcyclohexanecarboxylic acid p-(methoxy)phenyl ester,
trans-p-[5-(4-ethylcyclohexyl)-2-pyrimidinyl]benzonitrile,
trans-p-[5-(4-pentylcyclohexyl)-2-pyrimidinyl]benzonitrile and
trans-p-[5-(4-heptylcyclohexyl)-2-pyrimidinyl]benzonitrile.

22. Liquid crystalline mixture in accordance with claim 1, characterized in that it contains
4'-pentyl-4-cyanobiphenyl,
p-butylbenzoic acid p'-cyanophenyl ester,
p-(5-pentyl-2-pyrimidinyl)benzonitrile,
trans-4-propylcyclohexanecarboxylic acid p-cyanophenyl ester,
trans-4-pentylcyclohexanecarboxylic acid p-cyanophenyl ester,
trans-4-pentylcyclohexanecarboxylic acid p-(methoxy)phenyl ester and
trans-p-[5-(4-ethylcyclohexyl)-2-pyrimidinyl]benzonitrile.

23. Liquid crystalline mixture in accordance with claim 1, characterized in that it contains
4'-heptyl-4-cyanobiphenyl,
p-butylbenzoic acid p'-cyanophenyl ester,
p-(5-pentyl-2-pyrimidinyl)benzonitrile,
p-(5-heptyl-2-pyrimidinyl)benzonitrile,
trans-4-propylcyclohexanecarboxylic acid p-cyanophenyl ester,
trans-4-pentylcyclohexanecarboxylic acid p-cyanophenyl ester,
trans-4-butylcyclohexanecarboxylic acid p-(ethoxy)phenyl ester and
trans-p-[5-(4-ethylcyclohexyl)-2-pyrimidinyl]benzonitrile.

24. Liquid crystalline mixture in accordance with claim 1, characterized in that it contains
4′-pentyl-4-cyanobiphenyl,
4′-heptyl-4-cyanobiphenyl,
p-(5-pentyl-2-pyrimidinyl)benzonitrile,
p-(5-heptyl-2-pyrimidinyl)benzonitrile,
trans-4-butylcyclohexanecarboxylic acid p-(ethoxy)phenyl ester and
trans-p-[5-(4-pentylcyclohexyl)-2-pyrimidinyl]benzonitrile.

25. Liquid crystalline mixture in accordance with claim 1, characterized in that it contains
4′-pentyl-4-cyanobiphenyl,
4′-heptyl-4-cyanobiphenyl,
p-(5-pentyl-2-pyrimidinyl)benzonitrile,
p-(5-heptyl-2-pyrimidinyl)benzonitrile and
trans-p-[5-(4-ethylcyclohexyl)-2-pyrimidinyl]benzonitrile.

26. Liquid crystalline mixture in accordance with claim 1, characterized in that it contains
4′-pentyl-4-cyanobiphenyl,
4′-heptyl-4-cyanobiphenyl,
p-(5-pentyl-2-pyrimidinyl)benzonitrile,
p-(5-heptyl-2-pyrimidinyl)benzonitrile,
trans-4-butylcyclohexanecarboxylic acid p-(ethoxy)phenyl ester,
trans-4-pentylcyclohexanecarboxylic acid p-(methoxy)phenyl ester and
trans-p-[5-(4-pentylcyclohexyl)-2-pyrimidinyl]benzonitrile.

27. Liquid crystalline mixture in accordance with claim 1, characterized in that it contains
4′-pentyl-4-cyanobiphenyl,
4′-octyloxy-4-cyanobiphenyl,
p-(5-pentyl-2-pyrimidinyl)benzonitrile,
p-(5-heptyl-2-pyrimidinyl)benzonitrile and
trans-p-[5-(4-pentylcyclohexyl)-2-pyrimidinyl]benzonitrile.

28. Liquid crystalline mixture in accordance with claim 1, characterized in that it contains
4′-pentyl-4-cyanobiphenyl,
p-butylbenzoic acid p′-cyanophenyl ester,
p-(5-pentyl-2-pyrimidinyl)benzonitrile,
p-(5-heptyl-2-pyrimidinyl)benzonitrile,
trans-4-butylcyclohexanecarboxylic acid p-cyanophenyl ester,
trans-4-pentylcyclohexanecarboxylic acid p-cyanophenyl ester,
trans-4-butylcyclohexanecarboxylic acid p-(ethoxy)phenyl ester,
trans-4-pentylcyclohexanecarboxylic acid p-(methoxy)phenyl ester and
trans-p-[5-(4-ethylcyclohexyl)-2-pyrimidinyl]benzonitrile.

29. Liquid crystalline mixture in accordance with claim 1, characterized in that it contains
4′-heptyl-4-cyanobiphenyl,
trans-4-propylcyclohexanecarboxylic acid p-cyanophenyl ester,
trans-4-butylcyclohexanecarboxylic acid p-(ethoxy)phenyl ester,
trans-4-pentylcyclohexanecarboxylic acid p-(methoxy)phenyl ester,
trans-4-pentylcyclohexanecarboxylic acid p-(propyloxy)phenyl ester and
trans-p-[5-(4-pentylcyclohexyl)-2-pyrimidinyl]benzonitrile.

30. Process for the manufacture of compounds of the general formula

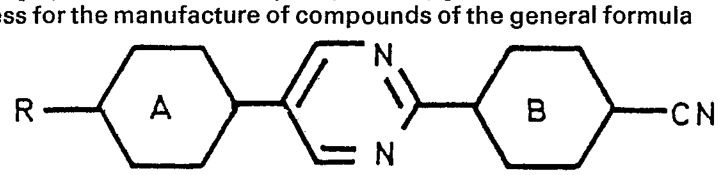

I

wherein at least one of the rings A and B represents a trans-1,4-disubstituted cyclohexane ring and the other is optionally aromatic and R signifies a straight-chain alkyl or alkoxy group with 1 to 10 carbon atoms or a branched alkyl group

$C_2H_5-CH(CH_3)-(CH_2)_n-$,

wherein n is a whole number of 1 to 3, characterized by

a) dehydrating a compound of the general formula

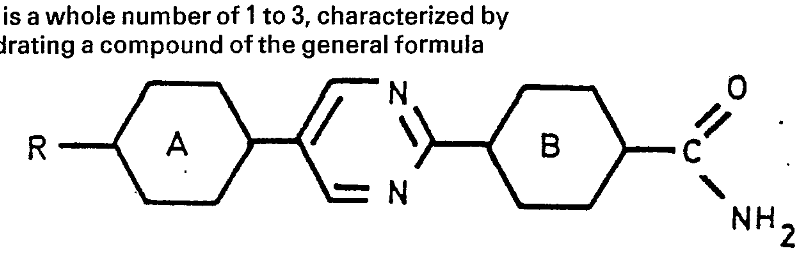

II

wherein A, B and R have the above significance, or

b) for the manufacture of a compound of formula I wherein the ring B is aromatic, reacting a compound of the general formula

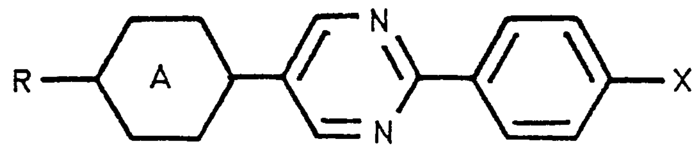

III

wherein R and A have the above significance and X represents fluorine, chlorine or bromine, with copper (I) cyanide or with sodium cyanide or potassium cyanide.

31. Electro-optical device containing one or more compounds of formula I defined in claim 1 in liquid crystalline condition.

**Revendications pour l'Etats contractants: BE, CH, DE, FR, IT, NL**

1. Composés de formule générale

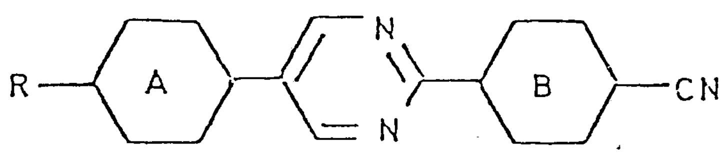

I

où au moins l'un des noyaux A et B représente un noyau cyclohexane trans-1,4-disubstitué et l'autre est éventuellement aromatique et R représente un groupe alcoyle ou alcoxy à chaîne droite en $C_1$ à $C_{10}$ ou un groupe alcoyle ramifié

$C_2H_5-CH(CH_3)-(CH_2)_n-$,

où n est un nombre entier allant de 1 à 3.

2. trans-p-[5-(4-Alcoyl- ou 4-n-alcoxycyclohexyl)-2-pyrimidinyl]benzonitriles de formule

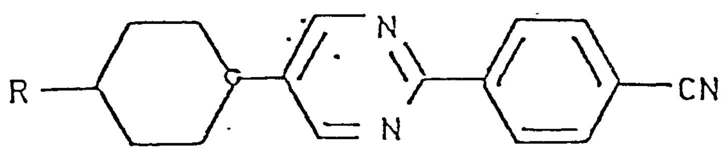

I A

où R est un alcoyle à chaîne droite en $C_2$ à $C_8$, un alcoxy à chaîne droite ayant jusqu'à 6 atomes de carbone ou un groupe alcoyle ramifié

$C_2H_5-CH(CH_3)-(CH_2)_n-$,

où n est un nombre entier allant de 1 à 3.

3. trans-4-[5-(p-Alcoyl- ou p-n-alcoxyphényl)-2-pyrimidinyl]cyclohexancarbonitriles de formule

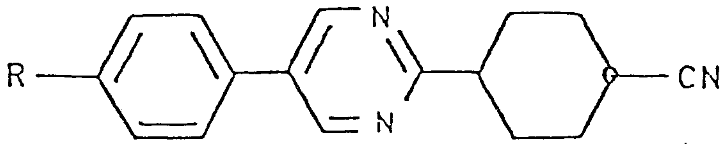

I B

où R est un alcoyle à chaîne droite en $C_2$ à $C_8$, un alcoxy à chaîne droite ayant jusqu'à 6 atomes de carbone ou un groupe alcoyle ramifié

$C_2H_5-CH(CH_3)-(CH_2)_n-$,

où n est un nombre entier allant de 1 à 3.

4. trans-4-[5-(trans-Alcoyl- ou trans-4-n-alcoxycyclohexyl)-2-pyrimidinyl]cyclohexancarbonitriles de formule

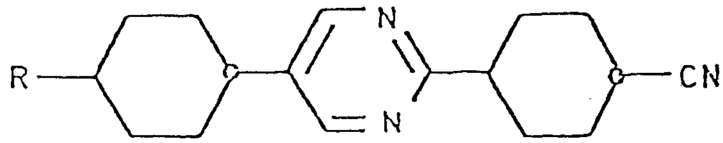

I C

où R est un alcoyle à chaîne droite en $C_2$ à $C_8$, un alcoxy à chaîne droite ayant jusqu'à 6 atomes de carbone ou un groupe alcoyle ramifié

$C_2H_5-CH(CH_3)-(CH_2)_n-$

où n est un nombre entier allant de 1 à 3.

5. trans-p-[5-(4-éthylcyclohexyl)-2-pyrimidinyl]benzonitrile.
6. trans-p-[5-(4-Pentylcyclohexyl)-2-pyrimidinyl]benzonitrile.
7. trans-p-[5-(4-heptylcyclohexyl)-2-pyrimidinyl]benzonitrile.
8. Mélanges de cristaux liquides caractérisés en ce qu'ils contiennent un ou plusieurs composés de formule I définie dans la revendication 1 et éventuellement d'autres composés nématiques et/ou non nématiques et/ou un ou plusieurs colorants dichroïques.
9. Mélanges de cristaux liquides selon la revendication 8, caractérisés en ce qu'ils contiennent un ou plusieurs composés de formule I définie dans la revendication 1 et un ou plusieurs colorants de formule générale

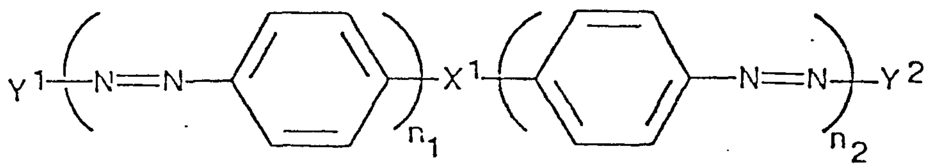

IV

où $n_1$ et $n_2$ représentent des nombres entiers allant de 0 à 2 et $X^1$ est un groupe azo ou azoxy, $Y^1$ et $Y^2$ peuvent être semblables ou différents et représentent l'un des substituants

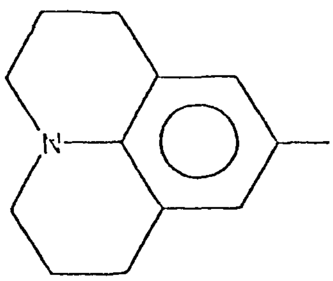

V ou

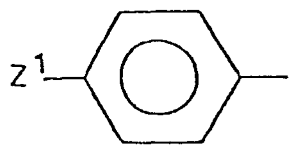

VI

et $Z^1$ représente un hydrogène, un cyano, un nitro, un phényle, un phényle p-substitué, un hydroxy, un alcoxy, un amino, un dialcoylamino ou un pyrrolidyle; ou des dérivés de formule IV où l'un des noyaux benzéniques posède en outre un ou plusieurs des groupes halogène, méthyle, méthyle halogénosubstitué ou méthoxy;

ou

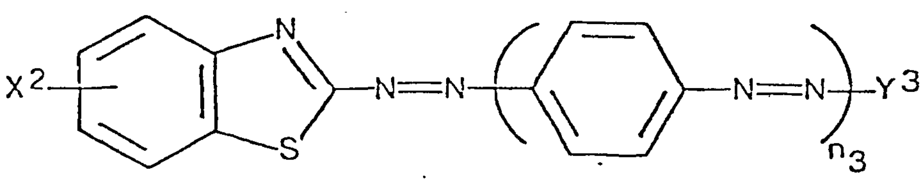

VII

où $n_3$ vaut 0 ou 1, $Y^3$ représente un substituant de formule V ou VI, où $Z^1$ représente un dialcoylamino ou un pyrrolidyle, et $X^2$ représente un hydrogène, un cyano, un nitro, un halogène, un alcoyle, un alcoxy, un groupe phényle ou benzothiazol-2-yle éventuellement substitué, un alcoxycarbonyle ou un alcoylsulfonyle en position 5 ou 6;

ou

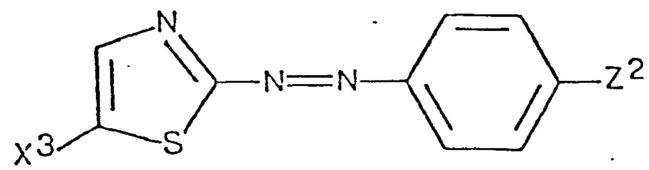

VIII

où $X^3$ représente un cyano, un nitro ou un alcoyle, et $Z^2$ représente un hydroxy, un alcoxy, un aryle ou un groupe amino, pyrrolidyle ou pipéridyle éventuellement substitué par un alcoyle ou un aryle; ou des dérivés de formule VIII dans lesquels le noyau benzénique possède en outre un ou plusieurs des groupes halogène, hydroxy, méthyle ou méthoxy;

ou

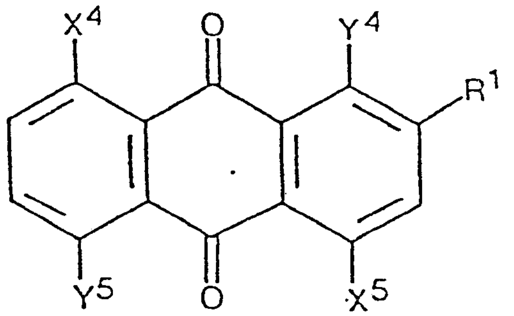

IX

où $X^4$ et $X^5$ représentent un hydroxy, $Y^4$ et $Y^5$ un amino et $R^1$ un substituant de formule VI ou bien où $X^4$, $Y^5$ et $R^1$ représentent un hydrogène, $X^5$ un hydroxy et $Y^4$ le groupe $-NHR^2$ ou bien où $X^4$, $X^5$ et $R^1$ représentent un hydrogène et $Y^4$ et $Y^5$ le groupe $-NHR^2$, $R^2$ représente un substituant de formule VI et $Z^1$ dans les substituants de formule VI représente un groupe alcoyle, alcoxy ou dialcoylamino;

ou

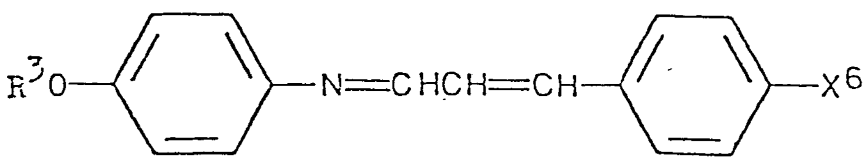

X

où $R^3$ représente un groupe alcoyle et $X^6$ un groupe cyano ou un groupe nitro;

ou

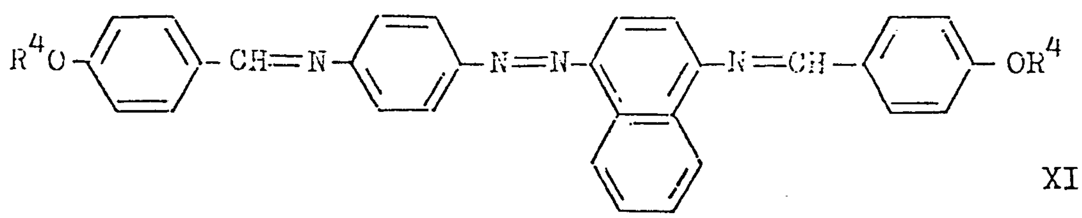

XI

où $R^4$ représente un groupe alcoyle ou

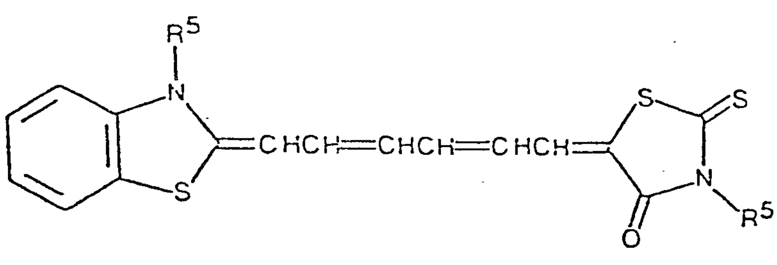

XII

où $R^5$ représente un groupe alcoyle;

et, éventuellement, d'autres composés nématiques et/ou non nématiques.

10. Mélanges de cristaux liquides selon la revendication 9, caractérisés en ce qu'ils contiennent au moins un colorant de formule

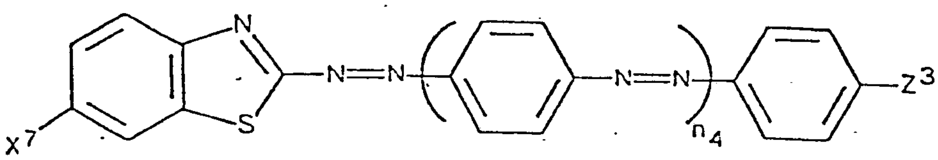

VIIa

où $n_4$ vaut 0 ou 1, $Z^3$ représente le groupe diméthylamino ou diéthylamino et $X^7$ représente un hydrogène, un méthyle, un éthoxy, un n-butyloxy, un nitro ou un n-butylsulfonyle.

11. Mélanges de cristaux liquides selon la revendication 9, caractérisés en ce qu'ils contiennent au moins un colorant de formule

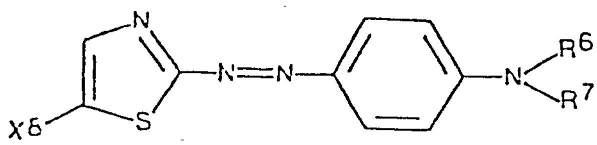

VIIIa

où $R^6$, $R^7$ et $X^8$ représentent un méthyle ou bien où $R^6$ représente un méthyle, $X^8$ un nitro et $R^7$ un méthyle, un phényle ou un p-n-butylsulfonyle.

12. Mélanges de cristaux liquides selon la revendication 9, caractérisés en ce qu'ils contiennent au moins un colorant de formule

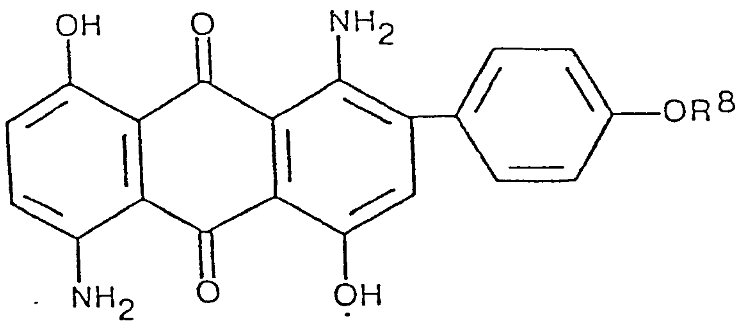

IXa

où $R^8$ représente un n-alcoyle en $C_1$ à $C_{10}$.

13. Mélanges de cristaux liquides selon la revendication 9, caractérisés en ce qu'ils contiennent au moins un colorant de formule

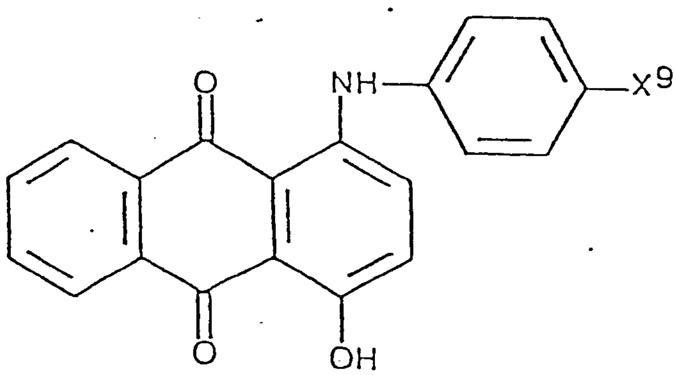

IXb

où $X^9$ représente un n-butyle, un n-nonyloxy ou un diméthylamino.

14. Mélanges de cristaux liquides selon la revendication 9, caractérisés en ce qu'ils contiennent au moins un colorant de formule

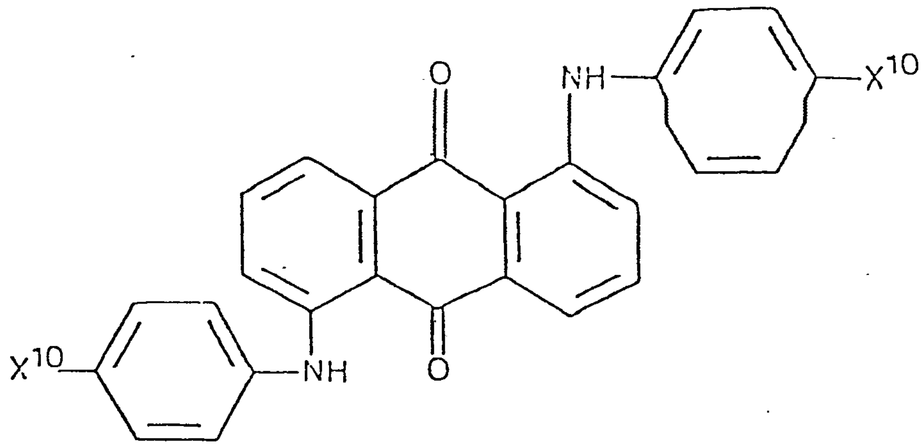

IXc

où $X^{10}$ représente un éthyle, un n-butyle, un isopropyle, un pentyloxy ou un diméthylamino.

15. Mélanges de cristaux liquides selon la revendication 9, caractérisés en ce qu'ils contiennent le colorant de formule

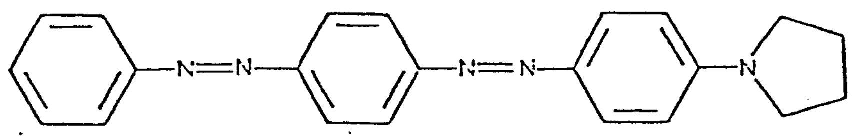

IVa

et/ou le colorant de formule X, où $R^3$ représente un n-butyle et $X^6$ un nitro,
et/ou le colorant de formule XI, où $R^4$ représente un éthyle,
et/ou le colorant de formule XII, où $R^5$ représente un éthyle.

16. Mélange de cristaux liquides selon la revendication 8, caractérisé en ce qu'il contient le
4′-pentyl-4-cyanobiphényle, le
p-cyanophénylester de l'acide trans-4-propylcyclohexanecarboxylique, le
p-cyanophénylester de l'acide trans-4-pentylcyclohexanecarboxylique, le
p-(méthoxy)phénylester de l'acide trans-4-pentylcyclohexanecarboxylique, le
trans-p-[5-(4-éthylcyclohexyl)-2-pyrimidinyl]benzonitrile, le
trans-p-[5-(4-pentylcyclohexyl)-2-pyrimidinyl]benzonitrile et le
trans-p-[5-(4-heptylcyclohexyl)-2-pyrimidinyl]benzonitrile.

17. Mélange de cristaux liquides selon la revendication 8, caractérisé en ce qu'il contient le
p′-cyanophénylester de l'acide p-butylbenzoïque, le
p-(5-pentyl-2-pyrimidinyl)benzonitrile, le
p-(5-heptyl-2-pyrimidinyl)benzonitrile, le
p-cyanophénylester de l'acide trans-4-butylcyclohexanecarboxylique, le
p-cyanophénylester de l'acide trans-4-pentyl-cyclohexanecarboxylique, le
p-(éthoxy)phénylester de l'acide trans-4-butylcyclohexanecarboxylique, le
p-(méthoxy)phénylester de l'acide trans-4-pentylcyclohexanecarboxylique et le
trans-p-[5-(4-éthylcyclohexyl)-2-pyrimidinyl]benzonitrile.

18. Mélange de cristaux liquides selon la revendication 8, caractérisé en ce qu'il contient le p′-cyanophénylester de l'acide p-butylbenzoïque, le
p-(5-pentyl-2-pyrimidinyl)benzonitrile, le
p-(5-heptyl-2-pyrimidinyl)benzonitrile, le
p-cyanophénylester de l'acide trans-4-propyl-cyclohexanecarboxylique, le
p-cyanophénylester de
l'acide trans-4-pentyl-cyclohexanecarboxylique, le
p-(éthoxy)phénylester de l'acide trans-4-butylcyclohexanecarboxylique, le
p-(méthoxy)-phénylester de l'acide trans-4-pentylcyclohexanecarboxylique, le
trans-p-[5-(4-pentylcyclohexyl)-2-pyrimidinyl]benzonitrile et le
trans-p-[5-(4-heptylcyclohexyl)-2-pyrimidinyl]benzonitrile.

19. Mélange de cristaux liquides selon la revendication 8, caractérisé en ce qu'il contient le
p′-cyanophénylester de l'acide p-butylbenzoïque, le
p-(5-pentyl-2-pyrimidinyl)benzonitrile, le
p-(5-heptyl-2-pyrimidinyl)benzonitrile, le
p-cyanophénylester de l'acide trans-4-propyl-cyclohexanecarboxylique, le
p-cyanophénylester de l'acide trans-4-pentylcyclohexanecarboxylique, le
p-(éthoxy)-phénylester de l'acide trans-4-butyl-cyclohexanecarboxylique, le
p-(méthoxy)phénylester de l'acide trans-4-pentylcyclohexanecarboxylique et le
trans-p-[5-(4-éthylcyclohexyl)-2-pyrimidinyl]benzonitrile.

20. Mélange de cristaux liquides selon la revendication 8, caractérisé en ce qu'il contient le
4'-heptyl-4-cyanobiphényle, le
p-(5-pentyl-2-pyrimidinyl)benzonitrile, le
p-(5-heptyl-2-pyrimidinyl)benzonitrile, le
p-cyanophénylester de l'acide trans-4-propylcyclohexanecarboxylique, le
p-cyanophénylester de l'acide trans-4-butylcyclohexanecarboxylique, le
p-cyanophénylester de l'acide trans-4-pentylcyclohexanecarboxylique, le
p-(méthoxy)phénylester de l'acide trans-4-pentylcyclohexanecarboxylique, le
p-(éthoxy)phénylester de l'acide trans-4-butylcyclo-hexanecarboxylique et le
trans-p-[5-(4-éthylcyclohexyl)-2-pyrimidinyl]benzonitrile.

21. Mélange de cristaux liquides selon la revendication 8, caractérisé en ce qu'il contient le
p'-cyanophénylester de l'acide p-butylbenzoïque, le
p-(5-pentyl-2-pyrimidinyl)benzonitrile, le
p-(5-heptyl-2-pyrimidinyl)benzonitrile, le
p-cyanophénylester de l'acide trans-4-propyl-cyclohexanecarboxylique, le
p-cyanophénylester de l'acide trans-4-butyl-cyclohexanecarboxylique, le
p-cyanophénylester de l'acide trans-4-pentylcyclohexanecarboxylique, le
p-(éthoxy)phénylester de l'acide trans-4-butylcyclohexanecarboxylique, le
4''-pentyl-4-cyano-p-terphényle et le
trans-p-[5-(4-éthylcyclohexyl)-2-pyrimidinyl]benzonitrile.

22. Mélange de cristaux liquides selon la revendication 8, caractérisé en ce qu'il contient le
4'-pentyl-4-cyanobiphényle, le
p-cyanophénylester de l'acide trans-4-propylcyclohexanecarboxylique, le
p-cyanophénylester de l'acide trans-4-pentylcyclohexanecarboxylique, le
p-(éthoxy)phénylester de l'acide trans-4-butylcyclohexanecarboxylique, le
p-(méthoxy)phénylester de l'acide trans-4-pentylcyclohexanecarboxylique, le
trans-p-[5-(4-éthylcyclohexyl)-2-pyrimidinyl]benzonitrile, le
trans-p-[5-(4-pentylcyclohexyl)-2-pyrimidinyl]benzonitrile et le
trans-p-[5-(4-heptylcyclohexyl)-2-pyrimidinyl]benzonitrile.

23. Mélange de cristaux liquides selon la revendication 8, caractérisé en ce qu'il contient le
4'-pentyl-4-cyanobiphényle, le
p'-cyanophénylester de l'acide p-butylbenzoïque, le
p-(5-pentyl-2-pyrimidinyl)benzonitrile, le
p-cyanophénylester de l'acide trans-4-propylcyclohexanecarboxylique, le
p-cyanophénylester de l'acide trans-4-pentylcyclohexanecarboxylique, le
p-(méthoxy)phénylester de l'acide trans-4-pentylcyclohexanecarboxylique et le
trans-p-[5-(4-éthylcyclohexyl)-2-pyrimidinyl]benzonitrile.

24. Mélange de cristaux liquides selon la revendication 8, caractérisé en ce qu'il contient le
4'-heptyl-4-cyanobiphényle, le
p'-cyanophénylester de l'acide p-butylbenzoïque, le
p-(5-pentyl-2-pyrimidinyl)benzonitrile, le
p-(5-heptyl-2-pyrimidinyl)benzonitrile, le
p-cyanophénylester de l'acide trans-4-propylcyclohexanecarboxylique, le
p-cyanophénylester trans-4-pentylcyclohexanecarboxylique, le
p-(éthoxy)phénylester de l'acide trans-4-butylcyclohexanecarboxylique et le
trans-p-[5-(4-éthylcyclohexyl)-2-pyrimidinyl]benzonitrile.

25. Mélange de cristaux liquides selon la revendication 8, caractérisé en ce qu'il contient le
4'-pentyl-4-cyanobiphényle, le
4'-heptyl-4-cyanobiphényle, le
p-(5-pentyl-2-pyrimidinyl)benzonitrile, le
p-(5-heptyl-2-pyrimidinyl)benzonitrile, le
p-(éthoxy)phénylester de l'acide trans-4-butylcyclohexane-carboxylique et le
trans-p-[5-(4-pentylcyclohexyl)-2-pyrimidinyl]benzonitrile.

26. Mélange de cristaux liquides selon la revendication 8, caractérisé en ce qu'il contient le
4'-pentyl-4-cyanobiphényle, le
4'-heptyl-4-cyanobiphényle, le
p-(5-pentyl-2-pyrimidinyl)benzonitrile, le
p-(5-heptyl-2-pyrimidinyl)benzonitrile et le
trans-p-[5-(4-éthylcyclohexyl)-2-pyrimidinyl]benzonitrile.

27. Mélange de cristaux liquides selon la revendication 8, caractérisé en ce qu'il contient le
4'-pentyl-4-cyanobiphényle, le
4'-heptyl-4-cyanobiphényle, le
p-(5-pentyl-2-pyrimidinyl)benzonitrile, le
p-(5-heptyl-2-pyrimidinyl)benzonitrile, le
p-(éthoxy)phénylester de l'acide trans-4-butylcyclohexanecarboxylique, le

p-(méthoxy)phénylester de l'acide trans-4-pentylcyclohexanecarboxylique, et le trans-p-[5-(4-pentylcyclohexyl)-2-pyrimidinyl]benzonitrile.

28. Mélange de cristaux liquides selon la revendication 8, caractérisé en ce qu'il contient le 4'-pentyl-4-cyanobiphényle, le 4'-octyloxy-4-cyanobiphényle, le p-(5-pentyl-2-pyrimidinyl)benzonitrile, le p-(5-heptyl-2-pyrimidinyl)benzonitrile et le trans-p-[5-(4-pentylcyclohexyl)-2-pyrimidinyl]benzonitrile.

29. Mélange de cristaux liquides selon la revendication 8, caractérisé en ce qu'il contient le 4'-pentyl-4-cyanobiphényle, le p'-cyanophénylester de l'acide p-butylbenzoïque, le p-(5-pentyl-2-pyrimidinyl)benzonitrile, le p-(5-heptyl-2-pyrimidinyl)benzonitrile, le p-cyanophénylester de l'acide trans-4-butylcyclohexanecarboxylique, le p-cyanophénylester de l'acide trans-4-pentyl-cyclohexanecarboxylique, le p-(éthoxy)phénylester de l'acide trans-4-butyl-cyclohexanecarboxylique, le p-(méthoxy)-phénylester de l'acide trans-4-pentylcyclohexanecarboxylique et le trans-p-[5-(4-éthylcyclohexyl)-2-pyrimidinyl]benzonitrile.

30. Mélange de cristaux liquides selon la revendication 8, caractérisé en ce qu'il contient le 4'-heptyl-4-cyanobiphényle, le p-cyanophénylester de l'acide trans-4-propylcyclohexanecarboxylique, le p-(éthoxy)phénylester de l'acide trans-4-butylcyclohexanecarboxylique, le p-(méthoxy)phénylester de l'acide trans-4-pentylcyclohexanecarboxylique, le p-(propyloxy)phénylester de l'acide trans-4-pentylcyclohexanecarboxylique et le trans-p-[5-(4-pentylcyclohexyl)-2-pyrimidinyl]benzonitrile.

31. Procédé de préparation de composés de formule générale

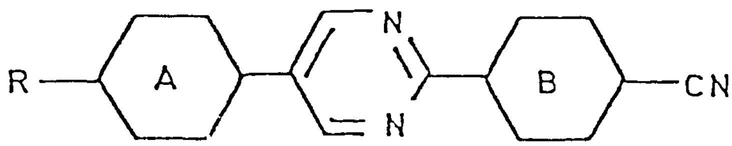

I

où au moins l'un des noyaux A et B représente un noyau cyclohexane trans-1,4-disubstitué et l'autre est éventuellement aromatique et R représente un groupe alcoyle ou alcoxy à chaîne droite en $C_1$ à $C_{10}$ ou un groupe alcoyle ramifié

$C_2H_5-CH(CH_3)-(CH_2)_n-$,

où n est un nombre entier allant de 1 à 3, caractérisé en ce que

a) On déshydrate un composé de formule générale

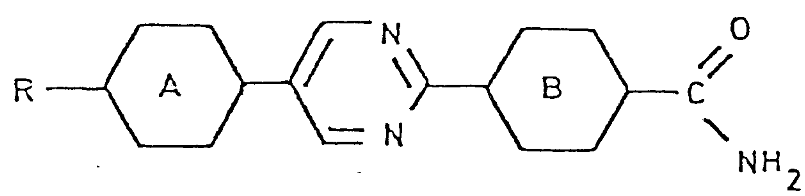

II

où A, B et R ont la signification donnée ci-dessus,

ou

b) Pour préparer un composé de formule I, où le noyau B est aromatique, on fait réagir un composé de formule générale

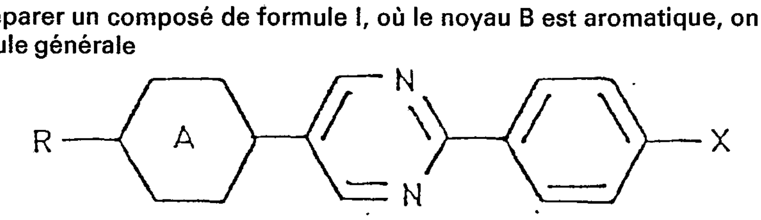

III

où R et A ont la signification donnée ci-sessus et X représente un fluor, un chlore ou un brome, avec du cyanure de cuivre (I) ou avec du cyanure de sodium ou de potassium.

32. Procédé de préparation de mélanges de cristaux liquides, caractérisé en ce qu'on mélange un ou plusieurs composés de la formule I définie dans la revendication 1 avec d'autres composés nématiques et/ou non nématiques et/ou un ou plusieurs colorants dichroïques.

33. Procédé de fabrication d'un dispositif électro-optique, caractérisé en ce qu'on met dans une

cellule appropriée un ou plusieurs composés de formule I définie dans la revendication 1, éventuellement mélangé(s) avec d'autres composés nématiques et/ou non nématiques et/ou un ou plusieurs colorants dichroïques.

34. Application des composés de formule I définie dans la revendication 1 à des buts électro-optiques.

35. Dispositif électro-optique contenant un ou plusieurs composés de formule I définis dans la revendication 1 à l'état de cristaux liquides.

**Revendications pour les Etat Autriche**

1. Mélanges de cristaux liquides, caractérisés en ce qu'ils contiennent un ou plusieurs composés de formule générale

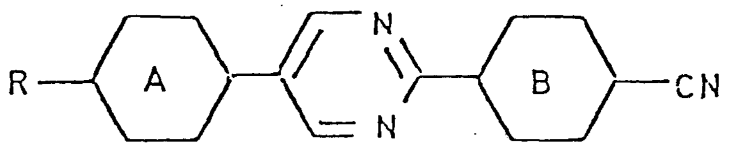

I

où au moins l'un des noyaux A et B représente un noyau cyclohexane trans-1,4-disubstitué et l'autre est éventuellement aromatique et R représente un groupe alcoyle ou alcoxy à chaîne droite en $C_1$ à $C_{10}$ ou un groupe alcoyle ramifié

$C_2H_5-CH(CH_3)-(CH_2)_n-$,

où n est un nombre entier allant de 1 à 3,
et, éventuellement, d'autres composés nématiques et/ou non nématiques et/ou un ou plusieurs colorants dichroïques.

2. Mélanges de cristaux liquides selon la revendication 1, caractérisés en ce qu'ils contiennent un ou plusieurs composés de formule générale

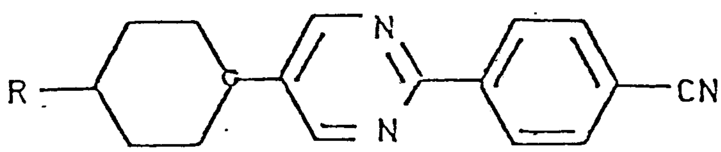

I A

où R est un alcoyle à chaîne droite en $C_2$ à $C_8$, un alcoxy à chaîne droite ayant jusqu'à 6 atomes de carbone ou un groupe alcoyle ramifié

$C_2H_5-CH(CH_3)-(CH_2)_n-$,

où n est un nombre entier allant de 1 à 3.

3. Mélanges de cristaux liquides selon la revendication 1, caractérisés en ce qu'ils contiennent un ou plusieurs composés de formule générale

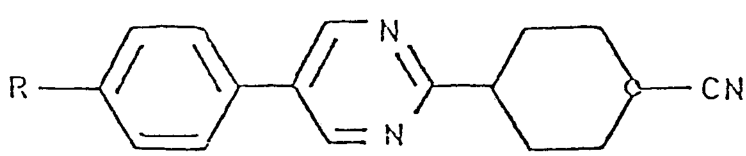

I B

où R est un alcoyle à chaîne droite en $C_2$ à $C_8$, un alcoxy à chaîne droite ayant jusqu'à 6 atomes de carbone ou un groupe alcoyle ramifié

$C_2H_5-CH(CH_3)-(CH_2)_n-$,

où n est un nombre entier allant de 1 à 3.

4. Mélanges de cristaux liquides selon la revendication 1, caractérisés en ce qu'ils contiennent un ou plusieurs composés de formule générale

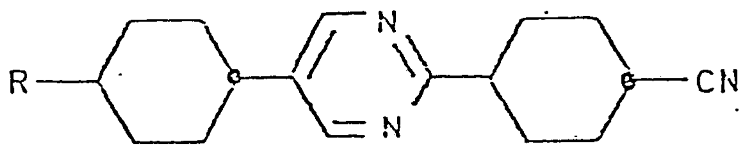

I C

où R est un alcoyle à chaîne droite en $C_2$ à $C_8$, un alcoxy à chaîne droite ayant jusqu'à 6 atomes de carbone ou un groupe alcoyle ramifié

$C_2H_5-CH(CH_3)-(CH_2)_n-$,

où n est un nombre entier allant de 1 à 3.

5. Mélanges de cristaux liquides selon l'une des revendications 1 ou 2, caractérisés en ce qu'ils contiennent le trans-p-[5-(4-éthylcyclohexyl)-2-pyrimidinyl)benzonitrile.

6. Mélanges de cristaux liquides selon l'une des revendications 1 ou 2, caractérisés en ce qu'ils contiennent le trans-p-[5-(4-pentylcyclohexyl)-2-pyrimidinyl)benzonitrile.

7. Mélanges de cristaux liquides selon l'une des revendications 1 ou 2, caractérisés en ce qu'ils contiennent le trans-p-[5-(4-heptylcyclohexyl)-2-pyrimidinyl]benzonitrile.

8. Mélanges de cristaux liquides selon l'une des revendications 1 à 7, caractérisés en ce qu'ils contiennent un ou plusieurs composés de formule I et un ou plusieurs colorants dichroïques de formule générale

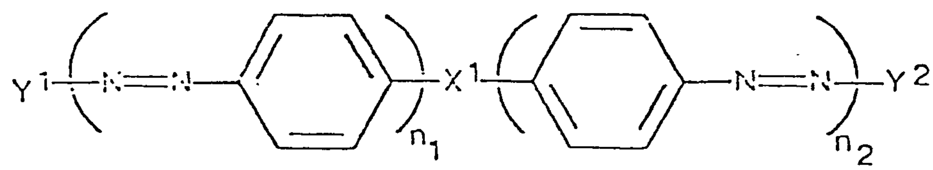

IV

où $n_1$ et $n_2$ représentent des nombres entiers allant de 0 à 2 et $X^1$ est un groupe azo ou azoxy, $Y^1$ et $Y^2$ peuvent être semblables ou différents et représentent l'un des substituants

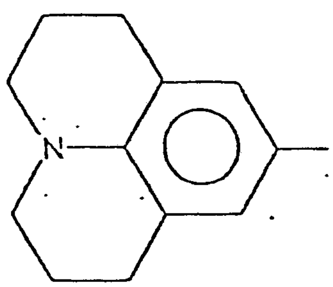

V ou

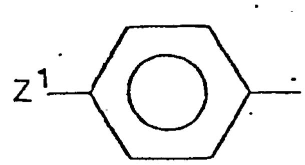

VI

et $Z^1$ représente un hydrogène, un cyano, un nitro, un phényle, un phényle p-substitué, un hydroxy, un alcoxy, un amino, un dialcoylamino ou un pyrrolidyle; ou des dérivés de formule IV où l'un des noyaux benzéniques possède en outre un ou plusieurs des groupes halogène, méthyle, méthyle halogénosubstitué ou méthoxy;

ou

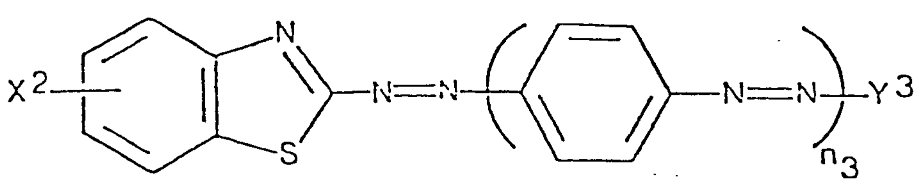

VII

où $n_3$ vaut 0 ou 1, $Y^3$ représente un substituant de formule V ou VI, où $Z^1$ représente un dialcoylamino ou un pyrrolidyle, et $X^2$ représente un hydrogène, un cyano, un nitro, un halogène, un alcoyle, un alcoxy, un groupe phényle ou benzothiazol-2-yle éventuellement substitué, un alcoxycarbonyle ou un alcoylsulfonyle en position 5 ou 6;

ou

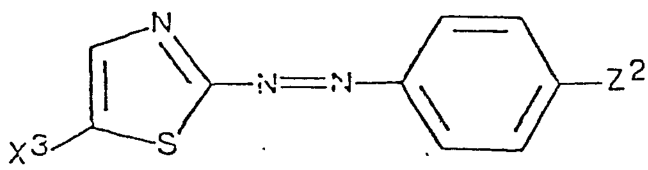

VIII

où $X^3$ représente un cyano, un nitro ou un alcoyle, et $Z^2$ représente un hydroxy, un alcoxy, un aryle ou un groupe amino, pyrrolidyle ou pipéridyle éventuellement substitué par un alcoyle ou un aryle; ou des dérivés de formule VIII dans lesquels le noyau benzénique possède en outre un ou plusieurs des groupes halogène, hydroxy, méthyle ou méthoxy;

ou

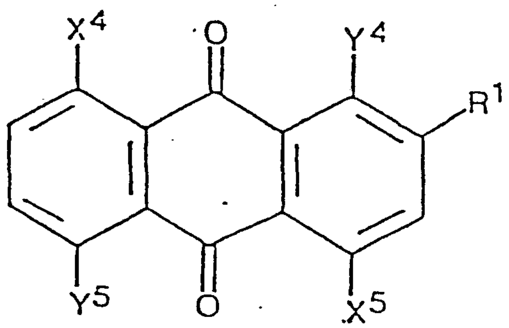

IX

où $X^4$ et $X^5$ représentent un hydroxy, $Y^4$ et $Y^5$ un amino et $R^1$ un substituant de formule VI ou bien où $X^4$, $Y^5$ et $R^1$ représentent un hydrogène, $X^5$ un hydroxy et $Y^4$ le groupe $-NHR^2$ ou bien où $X^4$, $X^5$ et $R^1$ représentent un hydrogène et $Y^4$ et $Y^5$ le groupe $-NHR^2$, $R^2$ représente un substituant de formule VI et $Z^1$ dans les substituants de formule VI représente un groupe alcoyle, alcoxy ou dialcoylamino;

ou

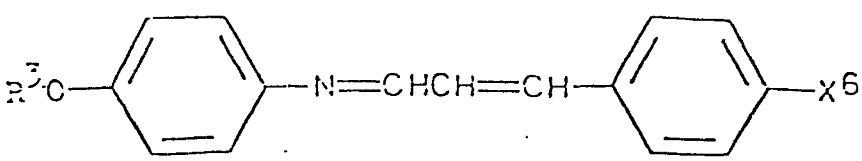

X

où $R^3$ représente un groupe alcoyle et $X^6$ un groupe cyano ou un groupe nitro;

ou

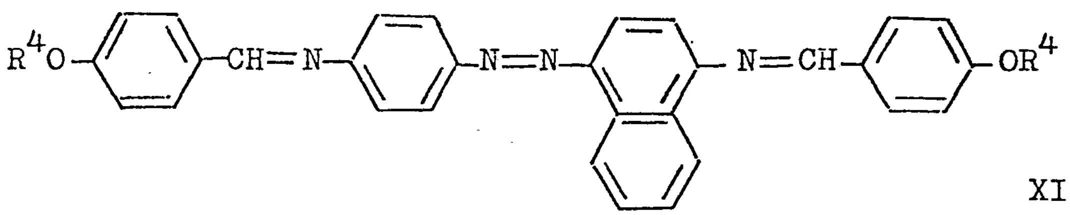

XI

où $R^4$ représente un groupe alcoyle ou

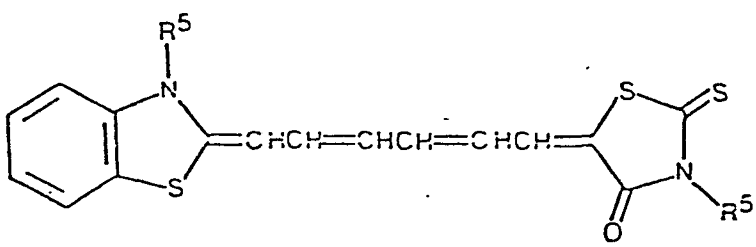

XII

où $R^5$ représente un groupe alcoyle;

et, éventuallement, d'autres composés nématiques et/ou non nématiques.

9. Mélanges de cristaux liquides selon la revendication 8, caractérisés en ce qu'ils contiennent au moins un colorant de formule

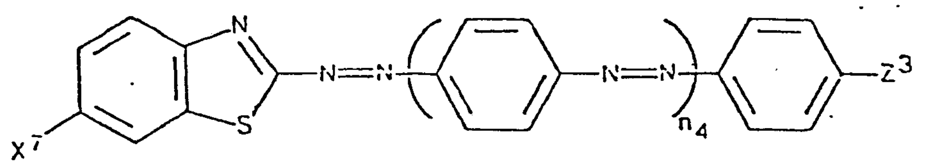

VIIa

où $n_4$ vaut 0 ou 1, $Z^3$ représente le groupe diméthylamino ou diéthylamino et $X^7$ représente un hydrogène, un méthyle, un éthoxy, un n-butyloxy, un nitro ou un n-butylsulfonyle.

10. Mélanges de cristaux liquides selon la revendication 8, caractérisés en ce qu'ils contiennent au moins un colorant de formule

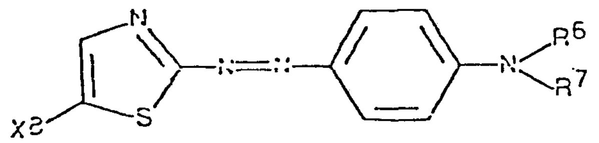

VIIIa

où $R^6$, $R^7$ et $X^8$ représentent un méthyle ou bien où $R^6$ représente un méthyle, $X^8$ un nitro et $R^7$ un méthyle, un phényle ou un p-n-butylsulfonyle.

11. Mélanges de cristaux liquides selon la revendication 8, caractérisés en ce qu'ils contiennent au moins un colorant de formule

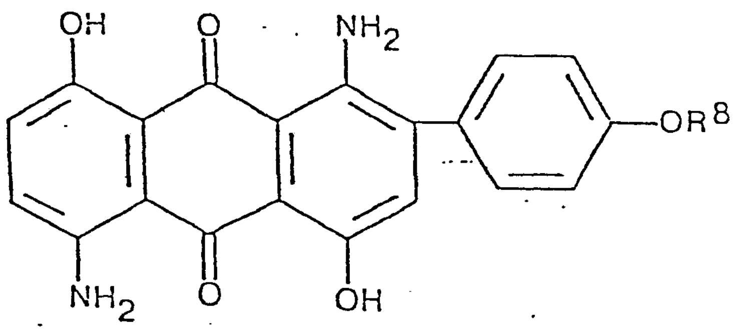

IXa

où $R^8$ représente un n-alcoyle en $C_1$ à $C_{10}$.

12. Mélanges de cristaux liquides selon la revendication 8, caractérisés en ce qu'ils contiennent au moins un colorant de formule

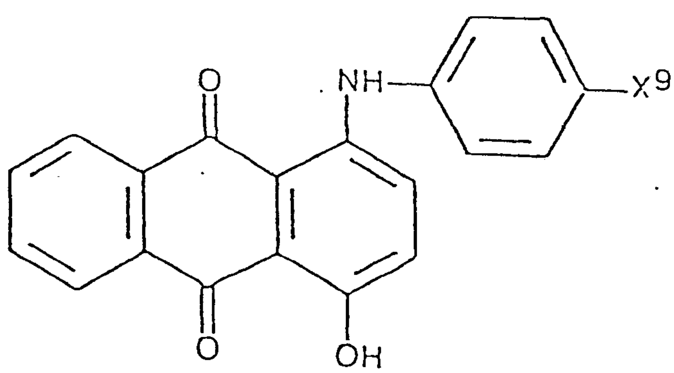

IXb

où $X^9$ représente un n-butyle, un n-nonyloxy ou un diméthylamino.

13. Mélanges de cristaux liquides selon la revendication 8, caractérisés en ce qu'ils contiennent au moins un colorant de formule

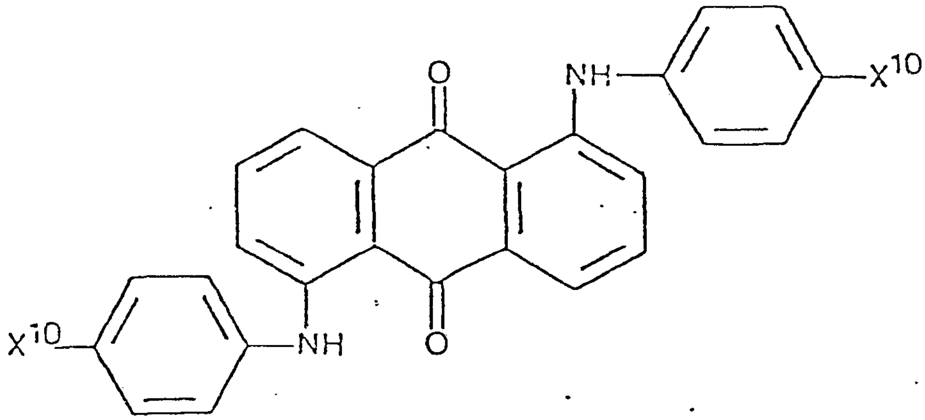

IVc

où $X^{10}$ représente un éthyle, un n-butyle, un isopropyle, un pentyloxy ou un diméthylamino.

14. Mélanges de cristaux liquides selon la revendication 8, caractérisés en ce qu'ils contiennent le colorant de formule

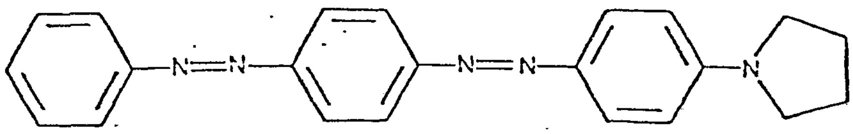

IVa

et/ou le colorant de formule X, où $R^3$ représente un n-butyle et $X^6$ un nitro,
et/ou le colorant de formule XI, où $R^4$ représente un éthyle,
et/ou le colorant de formule XII, où $R^5$ représente un éthyle.

15. Mélange de cristaux liquides selon la revendication 1, caractérisé en ce qu'il contient le
4'-pentyl-4-cyanobiphényle, le
p-cyanophénylester de l'acide trans-4-propylcyclohexanecarboxylique, le
p-cyanophénylester de l'acide trans-4-pentylcyclohexanecarboxylique, le
p-(méthoxy)phénylester de l'acide trans-4-pentylcyclohexanecarboxylique, le
trans-p-[5-(4-éthylcyclohexyl)-2-pyrimidinyl]benzonitrile, le
trans-p-[5-(4-pentylcyclohexyl)-2-pyrimidinyl]benzonitrile et le
trans-p-[5-(4-heptylcyclohexyl)-2-pyrimidinyl]benzonitrile.

16. Mélange de cristaux liquides selon la revendication 1, caractérisé en ce qu'il contient le
p'-cyanophénylester de l'acide p-butylbenzoïque, le
p-(5-pentyl-2-pyrimidinyl)benzonitrile, le
p-(5-heptyl-2-pyrimidinyl)benzonitrile, le
p-cyanophénylester de l'acide trans-4-butylcyclohexanecarboxylique, le
p-cyanophénylester de l'acide trans-4-pentyl-cyclohexanecarboxylique, le
p-(éthoxy)phénylester de l'acide trans-4-butylcyclohexanecarboxylique, le
p-(méthoxy)phénylester de l'acide trans-4-pentylcyclohexanecarboxylique et le
trans-p-[5-(4-éthylcyclohexyl)-2-pyrimidinyl]benzonitrile.

17. Mélange de cristaux liquides selon la revendication 1, caractérisé en ce qu'il contient le
p'-cyanophénylester de l'acide p-butylbenzoïque, le
p-(5-pentyl-2-pyrimidinyl)benzonitrile, le
p-(5-heptyl-2-pyrimidinyl)benzonitrile, le
p-cyanophénylester de l'acide trans-4-propyl-cyclohexanecarboxylique, le
p-cyanophénylester de l'acide trans-4-pentyl-cyclohexanecarboxylique, le
p-(éthoxy)phénylester de l'acide trans-4-butylcyclohexanecarboxylique, le
p-(méthoxy)phénylester de l'acide trans-4-pentylcyclohexanecarboxylique, le
trans-p-[5-(4-pentylcyclohexyl)-2-pyrimidinyl]benzonitrile et le
trans-p-[5-(4-heptylcyclohexyl)-2-pyrimidinyl]benzonitrile.

18. Mélange de cristaux liquides selon la revendication 1, caractérisé en ce qu'il contient le
p'-cyanophénylester de l'acide p-butylbenzoïque, le
p-(5-pentyl-2-pyrimidinyl)benzonitrile, le
p-(5-heptyl-2-pyrimidinyl)benzonitrile, le
p-cyanophénylester de l'acide trans-4-propyl-cyclohexanecarboxylique, le
p-cyanophénylester de l'acide trans-4-pentylcyclohexanecarboxylique, le
p-(éthoxy)phénylester de l'acide trans-4-butyl-cyclohexanecarboxylique, le
p-(méthoxy)phénylester de l'acide trans-4-pentylcyclohexanecarboxylique et le
trans-p-[5-(4-éthylcyclohexyl)-2-pyrimidinyl]benzonitrile.

19. Mélange de cristaux liquides selon la revendication 1, caractérisé en ce qu'il contient le
4'-heptyl-4-cyanobiphényle, le
p-(5-pentyl-2-pyrimidinyl)benzonitrile, le
p-(5-heptyl-2-pyrimidinyl)benzonitrile, le
p-cyanophénylester de l'acide trans-4-propylcyclohexanecarboxylique, le
p-cyanophénylester de l'acide trans-4-butylcyclohexanecarboxylique, le
p-cyanophénylester de l'acide trans-4-pentylcyclohexanecarboxylique, le
p-(méthoxy)phénylester de l'acide trans-4-pentylcyclohexanecarboxylique, le
p-(éthoxy)phénylester de l'acide trans-4-butylcyclohexanecarboxylique et le
trans-p-[5-(4-éthylcyclohexyl)-2-pyrimidinyl]benzonitrile.

20. Mélange de cristaux liquides selon la revendication 1, caractérisé en ce qu'il contient le
p'-cyanophénylester de l'acide p-butylbenzoïque, le
p-(5-pentyl-2-pyrimidinyl)benzonitrile, le
p-(5-heptyl-2-pyrimidinyl)benzonitrile, le
p-cyanophénylester de l'acide trans-4-propyl-cyclohexanecarboxylique, le
p-cyanophénylester de l'acide trans-4-butyl-cyclohexanecarboxylique, le
p-cyanophénylester de l'acide trans-4-pentylcyclohexanecarboxylique, le
p-(éthoxy)phénylester de l'acide trans-4-butylcyclohexanecarboxylique, le
4''-pentyl-4-cyano-p-terphényle et le
trans-p-[5-(4-éthylcyclohexyl)-2-pyrimidinyl]benzonitrile.

21. Mélange de cristaux liquides selon la revendication 1, caractérisé en ce qu'il contient le
4'-pentyl-4-cyanobiphényle, le
p-cyanophénylester de l'acide trans-4-propylcyclohexanecarboxylique, le
p-cyanophénylester de l'acide trans-4-pentylcyclohexanecarboxylique, le
p-(éthoxy)phénylester de l'acide trans-4-butylcyclohexanecarboxylique, le
p-(méthoxy)phénylester de l'acide trans-4-pentylcyclohexanecarboxylique, le
trans-p-[5-(4-éthylcyclohexyl)-2-pyrimidinyl]benzonitrile, le
trans-p-[5-(4-pentylcyclohexyl)-2-pyrimidinyl]benzonitrile et le
trans-p-[5-(4-heptylcyclohexyl)-2-pyrimidinyl]benzonitrile.

22. Mélange de cristaux liquides selon la revendication 1, caractérisé en ce qu'il contient le
4'-pentyl-4-cyanobiphényle, le
p'-cyanophénylester de l'acide p-butylbenzoïque, le
p-(5-pentyl-2-pyrimidinyl)benzonitrile, le
p-cyanophénylester de l'acide trans-4-propylcyclohexanecarboxylique, le
p-cyanophénylester de l'acide trans-4-pentylcyclohexanecarboxylique, le
p-(méthoxy)phénylester de l'acide trans-4-pentylcyclohexanecarboxylique et le
trans-p-[5-(4-éthylcyclohexyl)-2-pyrimidinyl]benzonitrile.

23. Mélange de cristaux liquides selon la revendication 1, caractérisé en ce qu'il contient le
4'-heptyl-4-cyanobiphényle, le
p'-cyanophénylester de l'acide p-butylbenzoïque, le
p-(5-pentyl-2-pyrimidinyl)benzonitrile, le
p-(5-heptyl-2-pyrimidinyl)benzonitrile, le
p-cyanophénylester de l'acide trans-4-propylcyclohexanecarboxylique, le
p-cyanophénylester trans-4-pentylcyclohexanecarboxylique, le
p-(éthoxy)phénylester de l'acide trans-4-butylcyclohexanecarboxylique et le
trans-p-[5-(4-éthylcyclohexyl)-2-pyrimidinyl]benzonitrile.

24. Mélange de cristaux liquides selon la revendication 1, caractérisé en ce qu'il contient le
4'-pentyl-4-cyanobiphényle, le
4'-heptyl-4-cyanobiphényle, le
p-(5-pentyl-2-pyrimidinyl)benzonitrile, le
p-(5-heptyl-2-pyrimidinyl)benzonitrile, le
p-(éthoxy)phénylester de l'acide trans-4-butylcyclohexanecarboxylique et le
trans-p-[5-(4-pentylcyclohexyl)-2-pyrimidinyl]benzonitrile.

25. Mélange de cristaux liquides selon la revendication 1, caractérisé en ce qu'il contient le
4'-pentyl-4-cyanobiphényle, le
4'-heptyl-4-cyanobiphényle, le
p-(5-pentyl-2-pyrimidinyl)benzonitrile, le
p-(5-heptyl-2-pyrimidinyl)benzonitrile et le
trans-p-[5-(4-éthylcyclohexyl)-2-pyrimidinyl]benzonitrile.

26. Mélange de cristaux liquides selon la revendication 1, caractérisé en ce qu'il contient le
4'-pentyl-4-cyanobiphényle, le
4'-heptyl-4-cyanobiphényle, le
p-(5-pentyl-2-pyrimidinyl)benzonitrile, le
p-(5-heptyl-2-pyrimidinyl)benzonitrile, le
p-(éthoxy)phénylester de l'acide trans-4-butylcyclohexanecarboxylique, le

p-(méthoxy)phénylester de l'acide trans-4-pentylcyclohexanecarboxylique, et le
trans-p-[5-(4-pentylcyclohexyl)-2-pyrimidinyl]benzonitrile.

27. Mélange de cristaux liquides selon la revendication 1, caractérisé en ce qu'il contient le
4'-pentyl-4-cyanobiphényle, le
4'-octyloxy-4-cyanobiphényle, le
p-(5-pentyl-2-pyrimidinyl)benzonitrile, le
p-(5-heptyl-2-pyrimidinyl)benzonitrile et le
trans-p-[5-(4-pentylcyclohexyl)-2-pyrimidinyl]benzonitrile.

28. Mélange de cristaux liquides selon la revendication 1, caractérisé en ce qu'il contient le
4'-pentyl-4-pcanobiphényle, le
p'-cyanophénylester de l'acide p-butylbenzoïque, le
p-(5-pentyl-2-pyrimidinyl)benzonitrile, le
p-(5-heptyl-2-pyrimidinyl)benzonitrile, le
p-cyanophénylester de l'acide trans-4-butylcyclohexanecarboxylique, le
p-cyanophénylester de l'acide trans-4-pentyl-cyclohexanecarboxylique, le
p-(éthoxy)phénylester de l'acide trans-4-butyl-cyclohexanecarboxylique, le
p-(méthoxy)-phénylester de l'acide trans-4-pentylcyclohexanecarboxylique et le
trans-p-[5-(4-éthylcyclohexyl)-2-pyrimidinyl]benzonitrile.

29. Mélange de cristaux liquides selon la revendication 1, caractérisé en ce qu'il contient le
4'-heptyl-4-cyanobiphényle, le
p-cyanophénylester de l'acide trans-4-propylcyclohexanecarboxylique, le
p-(éthoxy)phénylester de l'acide trans-4-butylcyclohexanecarboxylique, le
p-(méthoxy)phénylester de l'acide trans-4-pentylcyclohexanecarboxylique, le
p-(propyloxy)phénylester de l'acide trans-4-pentylcyclohexanecarboxylique et le
trans-p-[5-(4-pentylcyclohexyl)-2-pyrimidinyl]benzonitrile.

30. Procédé de préparation de composés de formule générale

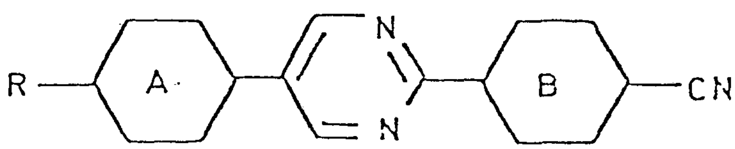

I

où au moins l'un des noyaux A et B représente un noyau cyclohexane trans-1,4-disubstitué et l'autre est éventuellement aromatique et R représente un groupe alcoyle ou alcoxy à chaîne droite en $C_1$ à $C_{10}$ ou un groupe alcoyle ramifié

$C_2H_5-CH(CH_3)-(CH_2)_n-$,

où n est un nombre entier allant de 1 à 3, caractérisé en ce que

a) on déshydrate un composé de formule générale

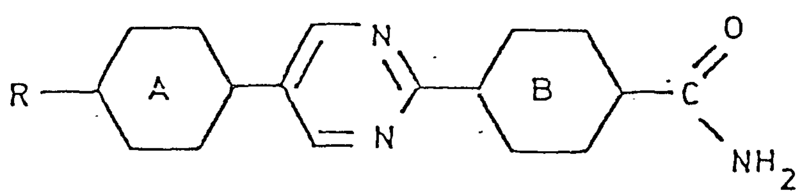

II

où A, B et R ont la signification donnée ci-dessus, ou

b) Pour préparer un composé de formule I, où le noyau B est aromatique, on fait réagir un composé de formule générale

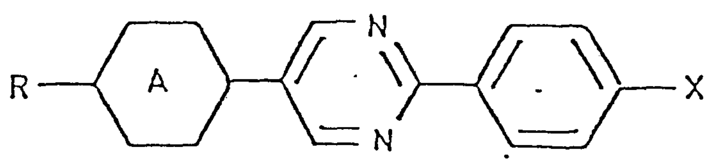

III

où R et A ont la signification donnée ci-dessus et X représente un fluor, un chlore ou un brome, avec du cyanure de cuivre (I) ou avec du cyanure de sodium ou de potassium.

31. Dispositif électro-optique contenant un ou plusieurs composés de formule I définis dans la revendication 1 à l'état de cristaux liquides.